(19) Europäisches Patentamt
European Patent Office
Office européen des brevets

(11) EP 1 304 101 A1

(12) EUROPEAN PATENT APPLICATION

(43) Date of publication:
**23.04.2003 Bulletin 2003/17**

(51) Int Cl.[7]: **A61K 7/48**, A61K 31/44, A61P 17/00

(21) Application number: **01204295.8**

(22) Date of filing: **12.11.2001**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **19.10.2001 IN CA06052001**
**01.11.2001 IN CA06202001**

(71) Applicant: **Torrent Pharmaceuticals Ltd**
**Ahmedabad 380 009, Gujarat State (IN)**

(72) Inventor: **Sankaranarayanan, Alangudi**
**Ahmedabad - 380 054, Gujarat State (IN)**

(74) Representative: **Schmitz, Jean-Marie et al**
**Dennemeyer & Associates S.A.,**
**P.O. Box 1502**
**1015 Luxembourg (LU)**

(54) **Composition and method for use of pyridinium derivatives in cosmetic and therapeutic applications**

(57) The invention discloses a new class of compounds particularly pyridinium derivatives, which have been found to exhibit triple function of a free radical scavenger (antioxidant), AGE breaker and AGE inhibitor, and cosmetic composition comprising these compounds contained in a cosmetically acceptable carrier. The invention also discloses a method of cosmetic application by applying such composition. The invention further discloses a pharmaceutical composition, comprising said compounds useful in scavenging free radicals from the body cells of a mammal, a method of scavenging free radicals from the body cells of a mammal and a method of treating of diseases caused by accumulation of free radicals in the body cells of a mammal by administering a composition made with the compounds of the invention. The invention in addition, also discloses composition and method for inhibiting AGE in a mammal by use of the compounds of the same group.

EP 1 304 101 A1

## Description

### Field of the Invention

The present invention relates to a new class of compounds particularly pyridinium derivatives which have been found to exhibit triple function of a free radical scavenger, AGE breaker and AGE inhibitor and cosmetic composition comprising them capable of arresting and reversing the process of skin aging resulting from an increased accumulation of advanced glycation end-products (AGEs) on the skin proteins and photo damage through free radical actions. The invention also relates to cosmetic application on skin by applying cosmetic composition comprising of said compounds. The invention further relates to composition and method for scavenging free-radicals from the body cells. The invention in addition relates to composition and method for inhibiting AGE by using compounds of the invention.

### Literature References

The background of the invention has been described with reference to the following publications as indicated by their reference serial numbers within parenthesis:

1. Boni R, Burg G: Aging skin: physiological bases, preventive measures and therapeutic modalities . Schweiz Med Wochenschr (2000) Sep 9; 130(36): 1272-8.
2. Odetti P, Aragno I, et al. : Role of advance glycation end products in aging collagen. A scanning force microscope study. Gerontology (1998); 44 (4): 187-91.
3. Pugliese PT: The skin's antioxidant systems Dermatol.Nurs (1998) Dec: 10 (6): 401-16; quiz 417-18.
4. Calabrese V, Scapagnini G et al.: Oxidative stress and antooxidants at skin bio surface: a novel antioxidant form lemon oil capable of inhibiting oxidative damage to the skin. Drugs Exp Clin Res(1999); 25(6): 281-7
5. Berneburg M, Plettenberg H, Krutmann J : Photoaging of human skinPhotodermatol. Photoimmunol. Photomed (2000) Dec: 16(6): 238-44
6. Masaki H, Okano Y, Sakurai H : Generation of active oxygen species from advanced glycation end products (AGE) under ultraviolet light A (UVA) irradiation: Biochem. Biophys.Res.commun. (997) Jun 18: 235.
7. Hitoshi Masaki, Yuri Okano, Hiromu Sakurai : Generation of active oxygen species from advanced glycation end products (AGEs) during ultraviolet light A (UVA) irradiation and a possible mechanism for cell damaging.: Biochemica et Biophysica Acta 1428 (1999) 45-56.

### BACKGROUND OF THE INVENTION

Health, resilience and youthful appearance of the skin depends, among other things, on several key classes of biological molecules. The key skin molecules are collagen and elastin. Collagen is a protein, forming the structural grid that holds other skin structures. It gives the skin its strength and durability. As any other protein, collagen is composed of amino acids. However it is unusually rich in a few specific amino acids; proline, hydroxy proline, lysine and glycine. Elastin is also a protein, more stretchable than collagen and helps to maintain skin resilience and elasticity. It contains two special amino acids: desmosine and isodesmosine. When both elastin and collagen are at scarce and damaged, the skin looses its shape after being stretched or folded leading to wrinkles and facial sag that happens during the process of aging.

Most modern theories of aging have centered around the notion that age-related deterioration is primarily due to structural and functional modifications of cellular constituents. The currently popular hypothesis are the Free Radical, Glycation or Maillard theories of aging. The first hypothesis proposes that age-related effects are due to free radical reactions that damage cellular constituents. "Free radical" refers to an unstable molecule that has an unpaired or odd electron in an outer orbit, which indiscriminately react with other molecules causing lipid, DNA and protein damage. The latter hypothesis propose that the primary cause of aging is cellular damage resulting from the modification of macromolecules induced by non-enzymatic glycation and Maillard reactions to form advanced glycosylation end-products (AGEs). Non-enzymatic glycation is the chemical attachment of sugars to protein that eventually causes protein cross linking, which is irreversible. Although these hypothesis were formulated independently, it suggests that free radicals, glycation, and Maillard reactions may in fact represent partially interactive elements of a single, more complex biochemical pathway, and that age-related deterioration is produced by the sum of the damages induced by all three hypotheses, and by their interactions.

Skin, a highly differentiated and complexly structured organ, is particularly vulnerable to free radical damage on exposure to UV radiation resulting in an increased accumulation of AGEs on the skin as well as an increased production of singlet oxygen and super oxide radicals which damage the important skin molecules such as collagen and elastin.

Under such situations an anti-oxidative condition through free radical scavenging would certainly enable the skin to maintain its normal resilience and integrity against damage.

Hence, the present invention is directed towards a cosmetic application with an active molecule capable of reversing the AGE cross links and creating an anti-oxidative environment in tissues through its AGE breaking and free radical quenching actions, thereby significantly slowing down the aging manifestations.

The skin is the largest organ in the body, comprising about 15% of the body weight. In terms of chemical composition, the skin is about 70% water, 25% protein and 2% lipids. The remainder includes trace minerals, nucleic acids, glycosoaminoglycans, proteoglycans and numerous other chemicals.

The skin consists of 3 main layers : Epidermis, dermis, subcutaneous tissue. The epidermis is the first barrier between us and the outside world. This layer consists of 3 types of cells; keretinocytes, melanocytes and langerhans cells. The dermis is the middle layer of the skin, the thickest of the skin layers and comprises a tight, sturdy mesh of collagen (type-I and III) and elastin fibers which are the critically important skin proteins. The dermis also consists of fibroblasts, capillaries, lymph nodes, sebaceous glands, sweat glands and hair follicles. The subcutaneous tissue is the innermost layer of the skin comprising mainly of adipocytes, acts as a shock absorber and heat insulator, protecting underlying tissues from cold and mechanical trauma.

Aging is a biological phenomenon which is symbolized by wrinkles and sagging skin. As a person ages, skin cells divide more slowly, and the inner skin, or dermis, starts to thin. Fat cells beneath the dermis begin to atrophy, and the underlying network of elastin and collagen fibers, which provides scaffolding for the surface layers, loosens and unravels. Skin loses its elasticity; when pressed, it no longer springs back to its initial position but instead sags and forms furrows. The skin's ability to retain moisture diminishes; the sweat-and oil-secreting glands atrophy, depriving the skin of their protective water-lipid emulsions. As a consequence, the skin becomes dry and scaly. In addition, the ability of the skin to repair itself diminishes with age, so wounds are slower to heal. Frown lines (those between the eyebrows) and crow's feet (lines that radiate from the corners of the eyes) appear to develop because of permanent small muscle contractions. Habitual facial expressions also form characteristic lines, and gravity exacerbates the situation, contributing to the formation of jowls and drooping eyelids. Since the skin represents the most visible organ of the aging, there is increasing interest in the physiology and reversal of wrinkles, elastoses and senile xerosis. Cutaneous aging is a complex phenomenon consisting of genetically determined intrinsic and extrinsic aging factors(1).

Mainly, there are two biologically independent aging processes that occur simultaneously, which account for the major changes seen in skin over time.

1. Extrinsic aging or Photoaging/External Factors and
2. Innate or Intrinsic aging/Internal Factors

Extrinsic aging or Photoaging, which results when skin is exposed to the elements like Ultraviolet (UV) radiation, Chemical Pollutants, Allergens, Mechanical damage, etc. Extrinsic aging is primarily caused by ultraviolet radiation of the sun.

Intrinsic aging affects skin by slow, irreversible degeneration of tissue. The factors causing intrinsic aging are genetic, nervous (stresses), immune, hormone disorders and others. Intrinsic aging can be observed over the entire surface of the body, including skin protected from ultraviolet radiation of sun. The phenomenon of glycation as discussed above plays a serious part in intrinsic aging. Proteins from dermis, elastin and collagen react with sugars in the body, especially glucose to result in the binding together of collagen fibers and the synthesis of free radicals. This modifies the structure of the skin causing it to loose its suppleness and become more rigid. Thus, the most noticeable changes on facial skin result from a combination of intrinsic and extrinsic aging processes.

Basically two factors free radicals and AGE formation are the prominent accelerators of skin wrinkles. The Maillard theory of Skin aging dates back to 1912 when Maillard found that reducing sugars such as glucose and ribose react with proteins to form brown pigments. The Maillard reaction is a series of complex reactions that cause the cross-linking Of protein via the interaction of reducing sugars with amino groups of proteins to form stable Amadori products, which subsequently cross-link to form Advanced Glycation End products (AGE). Another property of critical biological significance is the observation that the Amadori products continue to cross-link and polymerize even in the absence of free glucose. Protein crosslink is important since it is responsible for deep wrinkling in the dermis. The formation of AGE crosslinks is also a natural part of the aging and all the processes where protein aging is a serious detriment. During the aging process reducing sugar chemically attaches to the skin's support proteins like elastin and collagen, causing them to become gradually rigid and slowing their renewal. This nonspecific and non-enzymatic attachment of the sugar to collagen and elastin lead to the formation of AGE which continues to cross-link and polymerize even in the absence of free glucose. The studies on the role of AGEs in aging collagen using scanning force microscope reveal that in the presence of an increased concentration of AGEs, significant structural alterations have been observed in the collagen fibrils of old rats (2). As a result of this aging process, collagen loses its elasticity and the skin develops wrinkles.

The covalent binding of glucose to the amino group of protein alone is not sufficient to account for structural changes observed in collagen. Oxygen radicals formed during glucose oxidation, and glycated protein oxidation may be involved directly in the formation of AGEs and collagen cross-linking. In vitro studies demonstrate that the presence of oxygen is indispensable for the advanced glycation and cross-linking of collagen. Antioxidative condition and free radical scavengers have been proven to inhibit or slow down the formation of AGEs and the cross-linking of collagen. It is also known that free radical scavengers are essential in protecting the epidermis from damage by free radicals generated both by environmental and endogenous factors (3).

Skin, which has a highly differentiated and certainly complex organizational structure, is particularly vulnerable to free radical damage because of its contact with oxygen and other environmental stimuli (4). Studies have proved that UV radiation increases the formation of AGEs on collagen, elastin and other skin proteins. It forms a vicious cycle by increasing the accumulation of AGEs on the skin as well as increased production of singlet oxygen and super oxide radicals which damage the skin protein.

With recent years, substantial progress has been made in unraveling the underlying mechanisms of photoaging. Induction of matrix metalloproteinases as a consequence of activator protein (AP)- 1 and Nuclear factor (NF) -kB activation as well as mutations of mitochondrial DNA have been identified recently(5). In the early stage of glycation the condensation of reducing sugars such as glucose with amino groups of proteins generates UVA photo generated singlet oxygen free radicals. It is reported that AGE is an important factor for promoting photoaging in the skin via generation of active oxygen species involving $O_2^-$, $H_2O_2$ and -OH (6). On the basis of invitro fibroblast studies a possible mechanism is proposed in which AGEs under UVA irradiation generate active oxygen species involving $O_2^-$ , $H_2O_2$ and OH while the OH species place a harmful role in promoting cell damage (7). These radicals disrupt the natural balance of the skin by stimulating the skin cells to synthesize metalloproteinases. The metalloproteinase enzymes degrade collagen without synthesizing anti- metalloprotenases that keeps a check on the skin protein degradation, which is a normal biological response. The unbalanced production of metalloproteinase over anti -metalloprotenases induced by singlet oxygen free radicals leads to break down of collagen and elastin of the skin. This is followed by imperfect wound repair of damaged collagenous matrix and accumulation of elastotic material, as a consequence the skin sags and wrinkles.

Due to the exposure of AGEs to UV A radiations, the generation of super oxide anion gets enhanced. This is accomplished through cellular electron transfer chain in which UV A-AGEs energy enhances the passing of electrons onto ground state oxygen. This leads to enhanced formation of super oxide anion during Adenosine Triphosphate (ATP) synthesis. An enzyme super oxide dismutase converts the super oxide anion into hydrogen peroxide and oxygen. Finally, the catalytic action of iron and copper transforms hydrogen peroxide into toxic hydroxyl radical causes the degradation of skin collagen and elastin which is followed by imperfect wound healing and solar scar develop that photoage the skin.

The shelves in the cosmetics market are full of products treating extrinsic aging, but there is still a vacuum for a product, which targets intrinsic aging by inhibiting AGE in skin support proteins.

The ability to inhibit the formation of Advanced Glycation End products (in skin support proteins, like collagen) along with AGE breaker activity and Free Radical Scavenging activity, carries with it significant implications in treatment of Skin aging and wrinkles etc. Thus, using these molecules it is possible to prevent the signs of skin aging and wrinkle formation etc., and using them for cosmetic applications.

Experience shows that skin aging and wrinkle formation occur in-spite of good skin care. Hence, there is a need for development of an agent to prevent or treat aging of skin caused by formation of AGE. The compounds of the present invention are non-peptide, capable of modifying the AGE cross-links, formation in Collagen and Elastin. The compounds of the instant invention can be formulated along with other agents into a Cosmetic preparation.

To prevent or delay skin wrinkles, it is important to inhibit formation of AGE, to reverse the already formed AGE as well as to lower the oxidative stress by means of an antioxidant or free radical scavenger. Essentially a molecule that inhibits AGE, breaks AGE, and slows down the formation of AGE and prevent collagen degradation, would be an ideal candidate for cosmeceuticals. The molecules of the instant invention exhibit the properties of being an AGE inhibitor and a potent AGE breaker as well as free radical scavenger which make them most suitable for cosmetic applications.

Free radicals are atoms or molecules that have one or more unpaired electrons in their atomic structures and are highly reactive. Free radicals- reactive oxygen species (ROS)-are produced continuously in mammalian systems as a consequence of normal metabolic processes. Exogenous sources of ROS include exercise, pollution (especially cigarette smoke and car exhaust), alcohol, sunlight, and drugs (like anesthetics). Although free radicals have an important role in normal physiologic mechanisms, the excessive production of ROS results in oxidative stress- the terms usually applied to the out come of oxidative damage to biologically important molecules, such as protein, lipids, and nucleic acids. Proteins have long been known to be susceptible to oxidation by ROS. Aromatic amino acids like cystine, and disulfide bonds are particularly vulnerable. All biological materials contain a variety of polyunsaturated fatty acids, which are predominantly located in membrane lipids. They are highly susceptible to damage by ROS.

The group of compounds known as antioxidants (also referred to as "free radical scavengers") is the major defense against oxidative stress. These compounds function to protect membrane and cytosolic components against damage from ROS. Primary antioxidants, which prevent the formation of new radical species, include enzyme systems such as superoxide dismutase (SOD) and glutathione peroxidase (GSH Px). Secondary antioxidants trap radical species, thus preventing chain reactions, and include nutrients such as vitamin E, vitamin C, taurine and β-carotene. The final line of antioxidant defense is provided by the repair systems such as the enzyme methionine sulfoxide reductase that regenerates methionine residues within oxidized proteins and restores function.
Endogenous oxidative damage to cellular components, primarily proteins, lipids, and DNA is thought to contribute to the pathogenesis of numerous chronic diseases. The association between compromised antioxidant status, indices of oxidative damage, and clinical conditions like diabetes mellitus, asthma, chronic renal failure, hepatitis, colitis, atopic dermatitis, arthritis and various degenerative disorders is now well documented. There is considerable circumstantial evidence linking diminished antioxidant status including enzymes and nonezymatic scavengers, to increased oxidative damage and disease severity.
The present invention relates to molecules with ability to break the protein cross linking. In addition, they have shown to have potent anti-oxidant activity and thus useful in several disease conditions where oxidative stress plays vital role in the pathogenesis besides their cosmetic applications as discussed above.
The co-pending European Patent application Nos. 99973986.5, 99974071.5 and 01201057.5 by the same applicant demonstrates the AGE-breaking activity of the pyridinium compounds of the invention. It has been now found that these compounds are also good free radical scavengers and thus showing anti-oxidant activity. The triple AGE-breaking AGE-inhibiting and free radical scavenging activity of the compounds make them most ideal for cosmetic applications as discussed earlier. Further the free radical scavenging activity of the compounds now discovered, make them useful in treatment of various diseases caused by accumulation of free radicals in the body cells. In addition, these compounds are also useful in inhibiting onset of AGE in a mamml.

## SUMMARY OF THE INVENTION

The first object of the invention is to provide a new class of compounds having a) free radical scavenging, b) AGE-breaker and c) AGE inhibiting activity in the same molecule. The second object of the invention is to provide a cosmetic composition comprising these compounds as active ingredients.
The third object of the invention is the process for making the cosmetic composition.
The fourth object of the invention is to provide a method for cosmetic application by applying the cosmetic composition of the invention.
The fifth object of the invention is to provide a pharmaceutical composition useful for scavenging free-radicals from the body cells.
Yet another object of the invention is to provide a method for scavenging free radicals from the body cells of a mammal .
A further object of the invention is to provide a method of treatment of diseases caused by accumulation of free radicals in the body cells of a mammal.
Yet another object of the invention is to provide a method for inhibiting AGE and also a composition for inhibiting AGE in a mammal.
Accordingly the present invention provides for a cosmetic composition comprising an effective amount of a compound with free radical scavenging, AGE-breaking and AGE inhibiting activity of the formula (I).

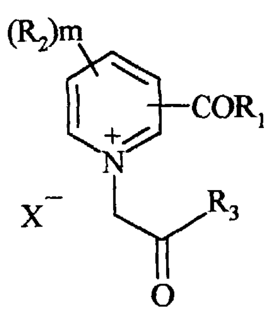

(I)

or its cosmetically acceptable salts contained in a cosmetically acceptable carrier
wherein
$R_1$ is selected from $-R_4-R_5$, -N ($R_7$) N ($R_7$) $R_9$ and Y-$R_{11}$
$R_4$ is selected from the group consisting of -N ($R_7$) $R_6$O-, -N ($R_7$) $R_6$N ($R_7$)-, O$R_6$O, and -O$R_6$N ($R_7$)-, where $R_6$ is alkyl;
$R_5$ is selected from the group consisting of alkyl, aryl including heteroaryl, -CO$R_7$, $SO_2R_7$, -C (S) NH$R_7$, -C (NH) NH$R_7$, -CO$R_{10}$,

$-C(O)NHR_7$ and 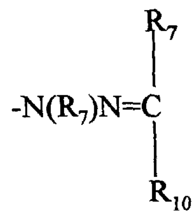

where $R_7$ is selected from the group consisting of H, alkyl and aryl including heteroaryl provided $R_7$ may be the same or different for $R_1$ and $R_3$ in the same compound;
$R_2$ is selected from the group consisting of F, Cl, Br, I, O$R_7$, $NO_2$, alkyl, aryl including heteroaryl, formyl, acyl, C (O) N$R_7R_{10}$, C (O) O$R_7$, N$R_7R_{10}$, N=C ($R_7$)($R_{10}$), S$R_7$, $SO_2NH_2$, $SO_2$ alkyl and $SO_2$aryl, and m is 0, 1 or 2;
$R_3$ is selected from the group consisting of $R_7$, O$R_7$, N ($R_7$) ($R_{10}$), N=C ($R_7$) ($R_{10}$), N ($R_7$) N ($R_7$) ($R_{10}$), N ($R_7$) N=C ($R_7$) ($R_{10}$) and CH ($R_7$) C (O) R8
Where $R_8$ is selected from the group consisting of $R_7$, O$R_7$ and N$R_7R_{10}$; $R_9$ is selected from the group consisting of hydrogen, alkyl, aryl including heteroaryl, C (O) $R_{10}$,-$SO_2R_{10}$, -C (S) NH$R_{10}$, -C (NH) NH ($R_{10}$) and -C (O) NH$R_{10}$
$R_{10}$ is selected for the group consisting of H, alkyl or aryl including heteroaryl and in each case may be the same or different from substituent $R_7$, provided $R_{10}$ may be the same or different for $R_1$ and $R_3$ in the same compound;
Y is selected from oxygen, NH, N$R_{12}$ and null
$R_{11}$ and $R_{12}$ are independently selected from hydrogen, alkyl and aryl.
X is selected from group consisting of a halide ion, acetate ion, perchlorate ion, sulfonate ion, oxalate ion, citrate ion, tosylate ion, maleate ion, mesylate ion, carbonate ion, sulfite ion, phosphoric hydrogen ion, phosphonate ion, phosphate ion, $BF_4^-$and $PF_6^-$ with proviso that,

1. when alkyl groups are present on the same carbon or nitrogen they may be linked together to form a cyclic structure and
2. the nitrogen of heteroaryl ring of $R_{10}$, when present, may be quarternized
3. When $R_3$ is O$R_7$, $R_1$ is $NHNH_2$ then $R_7$ is not alkyl.
4. When $R_3$ is O$R_7$, $R_1$ is N($R_7$)N($R_7$)$R_9$ and $R_9$ is C(O)$R_{10}$ where $R_{10}$ is alkyl, then $R_7$ is not hydrogen. As used herein, "alkyl" refers to an optionally substituted nydrocarbon group joined by carbon-carbon bond(s) and having 1-8 carbon atoms joined together or
heteroalicyclic group with one or two heteroatoms independently selected from oxygen, nitrogen and sulfur. The alkyl hydrocarbon group may be linear, branched or cyclic, saturated or unsaturated. The substituents are selected from F, Cl, Br, N S O aryl and I. Preferably, no more than three substituents are present.
As used herein "aryl" refers to an optionally substituted aromatic group with atleast one ring having a conjugated pi-electron system, containing upto two conjugated or fused ring systems. Aryl includes carbocyclic aryl, heterocyclic aryl and biaryl groups, all of which may be optionally substituted. The substituents are selected from F, Cl, Br, I, N, S, O and straight chain or branched $C_1$-$C_6$ hydrocarbon.

The invention also provides for a method of cosmetic treatment by applying the composition as above.

The invention further provides a pharmaceutical composition useful for scavenging free radicals from the body cells of a mammal comprising the compound as defined above or its pharmaceutically acceptable salts in admixture with a pharmaceutically acceptable carrier, diluent excipient or solvate.

The invention further provides a method of scavenging free radicals from the body cells of a mammal by administering the pharmaceutical composition as mentioned above or a method of treatment of diseases caused by

accumulation of free radicals by administering the said composition.

The invention in addition provides for use of the compound of formula (I) as defined above for the manufacture of a medicament for treating diseases caused by accumulation of free radicals in the body cells.

The invention further provides a method for inhibiting AGE and a composition for inhibiting AGE by use of the compounds of Formula (I) as defined above.

## BRIEF DESCRISPTION OF THE DRAWINGS

The Figs. 1 and 2 illustrate the AGE inhibiting activity of the compounds of the invention.

Fig. 1: Shows the results of SDS-PAGE (Sodium Dodecyl Sulphate-Polyacrylamide Gel Electrophoresis) of test compounds No. 5 and 33 and control.

Fig. 2 Shows the degree of formation of the dimer (peak 1) an trimer (peak 2) of lysozyme relative to that in the control, plotted in terms of optical density (OD) of each band on SDS-PAGE.

## DETAILED DESCRIPTION OF THE INVENTION

The AGE breaker / inhibitor cum free radical scavenger compounds useful for the composition of the invention of general formula I having m as 0 or 1 and - $COR_1$ at position 3 are listed in Table 1A and such compounds of general formula I having m as 0 and - $COR_1$ at position 4 are listed in Table 1B. The following compounds suggested are by way of example alone of the representative compounds of the general formula I as defined above and in no way restrict the invention:

N, N'-bis [3-carbonyl-1- (2-phenyl-2-oxoethyl)-pyridinium] hydrazine dibromide (compound 1);
N, N'-bis [3-carbonyl-1- (2-ethoxy -2- oxoethyl) pyridinium] hydrazine dibromide (compound 2);
N, N'-bis [3-carbonyl-1- (2-(2', 4'-dichlorophenyl)-2-oxoethyl) pyridinium] hydrazine dibromide (compound 3);
1- (2- ethoxy -2- oxoethyl) -3- (2- (2- pyridyl) hydrazinocarbonyl) pyridinium bromide (compound 4);
1- (2- thien -2'- yl -2- oxoethyl) -3- (methanesulfonylhydrazinocarbonyl) pyridinium bromide (compound 5);
N, N'-bis [3-carbonyl-1- (2- thien -2'- yl -2- oxoethyl) pyridinium] hydrazine dibromide (compound 6);
1- (2- ethoxy -2- oxoethyl) -3- (2- (benzoyloxy) ethylaminocarbonyl) pyridinium bromide (compound 7);
1- (2- (2',4'- dichlorophenyl) -2- oxoethyl) -3- (2-(benzoyloxy) ethylamino-carbonyl) pyridinium bromide (compound 8);
1- (2- thien -2'- yl -2- oxoethyl) -3- (2- (2- pyridyl) hydrazinocarbonyl) pyridinium bromide (compound 9);
1- (2- phenyl -2- oxoethyl) -3- (2- (2- pyridyl) hydrazinocarbonyl) pyridinium bromide (compound 10);
1-(2-phenyl-2-oxoethyl)-3-(hydrazinocarbonyl)pyridinium bromide (compound 11);
1-(2- phenyl -2- oxoethyl) -3- (methanesulfonyl hydrazinocarbonyl) pyridinium bromide (compound 12);
1- (2- ethoxy -2- oxoethyl) -3- (methanesulfonyl hydrazinocarbonyl) pyridinium bromide (compound 13);
1-(2-phenyl-2-oxoethyl) -3- (phenylsulfonylhydrazino carbonyl) pyridinium bromide (compound 14);
1-(2-phenyl-2-oxoethyl) -2-chloro-3- (phenylsulfonylhydrazino carbonyl) pyridinium bromide (compound 15);
1-(2- phenyl -2- oxoethyl) -3- (2- (acetoxy) ethyloxy carbonyl) pyridinium bromide (compound 16);
1-(2-ethoxy -2- oxoethyl) -3- (2- (benzoyloxy) ethyloxy carbonyl) pyridinium bromide (compound 17);
1-(2- thien -2'- yl -2- oxoethyl)-4-(2-(benzoyloxy)ethylaminocarbonyl) pyridinium bromide (compound 18);
1-(2-ethoxy -2- oxoethyl) -4-(phenylsulfonyl hydrazino carbonyl) pyridinium bromide (compound 19);
1-(2-phenylamino-2-oxoethyl)-4-(phenylsulfonyl hydrazino carbonyl) pyridinium chloride (compound 20);
1-(2-ethoxy -2- oxoethyl) -3-(phenylsulfonyl hydrazino carbonyl) pyridinium bromide (compound 21);
1-(2-(2', 4'-dichlorophenyl)-2-oxoethyl)-3-(2(methoxy) ethyloxycarbonyl) pyridinium bromide (Compound 22);
1-(2-phenylamino-2-oxoethyl)-3-((benzoyloxy) ethylaminocarbonyl) pyridinium chloride (compound 23);
1-(2-thien-2'-yl- 2-oxoethyl)-3-(phenylaminocarbonyl hydrazinocarbonyl) pyridinium bromide (compound 24);
1-(2-phenyl-2-oxoethyl)-3-(2-(acetoxy) ethylaminocarbonyl) pyridinium bromide (compound 25);
1-(2-phenylamino-2-oxoethyl)-3-(phenyl sulfonyl hydrazino carbonyl) pyridinium chloride (compound 26);
1-(2-phenylamino-2-oxoethyl)-3-((4-methylphenyl) sulfonyl hydrazino carbonyl) pyridinium chloride (compound 27);
1-(2-phenyl-2-oxoethyl)-3-(2-(benzoyloxy) ethyloxy carbonyl) pyridinium bromide (compound 28);
1-(2-thien-2'-yl-2-oxoethyl)-3-(phenylcarbonyl hydrazino carbonyl) pyridinium bromide (compound 29);
1-(2-ethoxy-2-oxoethyl)-3-((phenylmethyl) sulfonyl hydrazino carbonyl) pyridinium bromide (compound 30);
1-(2-phenyl-2-oxoethyl)-3-((phenylmethyl) sulfonyl hydrazino carbonyl) pyridinium bromide (compound 31);
N, N' - bis [3-carbonyl-1- (2-furan-2'-yl-2-oxoethyl) pyridinium] hydrazine dibromide. (Compound No: 32);

N, N'-bis [3-carbonyl-1- (2-thien-2'-yl-2-oxoethyl) pyridinium] hydrazine dichloride. (Compound No: 33);
1-(2-thien-2'-yl-2-oxoethyl)-3-((2-(1-oxo-3-cyclohexyl)-ethyl)-hydrazino carbonyl)-pyridinium bromide. (Compound No: 34);
1-(2-phenylamino-2-oxo ethyl)-3-({2-(1-oxo-3-cyclohexyl)-ethyl} -hydrazinocarbonyl}-pyridinium bromide. (Compound No: 35);
1-(2-thien-2'-yl-2-oxoethyl)-3-[2-(benzoyloxy) ethylamino carbonyl]-pyridinium bromide (Compound No: 36);
1-(4-ethoxy-2, 4-dioxobutyl)-3-(2-(benzoyloxy) ethylamino carbonyl)-pyridinium chloride. (Compound No: 37);
1-(2', 4'-dichlorophenyl-2-oxoethyl)-3-(2-methoxyethyl aminocarbonyl)-pyridinium bromide. (Compound No: 38);
N, N'-bis- [3-carbonyl-1- (2-cyclopropylamino-2-oxoethyl) pyridinium] hydrazine dichloride. (Compound No: 39);
1-(2-cyclopropylamino-2-oxoethyl)-3-(2-methoxyethylaminocarbonyl)-pyridinium chloride. (Compound No: 40);
N-N'-bis [3-carbonyl-1- (2-isopropylamino-2-oxoethyl) pyridinium] hydrazine dichloride. (Compound No: 41);
1-(2-thien-2'yl-2-oxoethyl)-3-(2-(2-chloro-3-pyridoyl)hydrazinocarbonyl) -pyridinium chloride. (Compound No: 42);
1-(2-isopropylamino-2-oxoethyl)-3-(2-methylsulfonylhydrazinocarbonyl) -pyridinium chloride. (Compound No: 43);
1-(2-(1-pyrrolidinyl)-2-oxoethyl)-3-(methanesulfonyl hydrazino carbonyl) pyridinium chloride. (Compound No: 44);
1-(2-thien-2'-yl-2-oxoethyl)-3-(methanesulfonyl hydrazino carbonyl) pyridinium chloride. (Compound No: 45);
N, N'-bis [3-carbonyl-1- (2-hydroxy-2-oxoethyl) pyridinium] hydrazine dichloride (Compound No: 46);
1-(2-thien-2'-yl-2-oxoethyl)-3-((2-methoxy ethyl) amino carbonyl)-5-bromo pyridinium chloride (Compound No: 47);
1-(2-thien-2'-yl-2-oxoethyl)-3-[1-oxo-1- (2-methoxy carbonyl) pyridyl] hydrazino pyridinium chloride. (Compound No: 48);
1-[1-(2-thien-2'-yl-2-oxoethyl)-6-methyl-3-carbonyl pyridinium]-2-[1-(2-Thien-2' -yl-2-oxoethyl )-3-carbonyl pyridinium ] hydrazine dichloride(compound no: 49).
1-(2-thien-2'-yl-2-oxoethyl)-3-(isopropylsulfonyl hydrazino carbonyl) pyridinium bromide (compound no: 50).
1-(2-(4-benzyl piperidin-1-yl)-2-oxoethyl)-3-(methanesulfonyl hydrazino carbonyl) pyridinium chloride (compound no: 51).
1-(2-(2-ethoxy carbonyl pyrrolidin-1-yl)-2-oxoethyl)-3-(methanesulfonyl hydrazino carbonyl) pyridinium chloride (compound no: 52).
1-(2-thien-2'-yl-2-oxoethyl)-3-(methanesulfonyl hydrazino carbonyl )-5-bromo pyridinium bromide. (compound no: 53).
1-(2-thien-2'-yl-2-oxoethyl)-3-(ethoxycarbonyl hydrazino carbonyl) pyridinium bromide. (compound no: 54).
1-(2-(5-chlorothien-2-yl)-2-oxoethyl)-3-(methanesulfonyl hydrazino carbonyl) pyridinium bromide (compound no: 55).
N, N'-bis [3-carbonyl-1- (2-(4-nitrothien-2-yl)-2-oxoethyl) pyridinium] hydrazine dichloride. (compound no: 56).
1-(2-thien-2'-yl-2-oxoethyl)-3-(methanesulfonyl hydrazino carbonyl) -6-methyl pyridinium bromide. (compound no: 57).
N, N'-bis [3-carbonyl-1- (2-(5-methylthien-2-yl)-2-oxoethyl) pyridinium] hydrazine dichloride. (compound no: 58).
N, N'-bis [3-carbonyl-1- (2-(2-ethoxycarbonyl pyrrolidin-1-yl)-2-oxoethyl) pyridinium] hydrazine dichloride. (compound no: 59).
1-[1-(2-thien-2'-yl-2-oxoethyl)-5-aminocarbonyl-3-carbonyl pyridinium]-2-[1-(2-Thien-2'-yl-2-oxoethyl)-3-carbonyl pyridinium] hydrazine dichloride (compound no: 60).
1-(2-(4-carboethoxy-thiazolidin-3-yl)-2-oxoethyl) -3-(methanesulfonyl hydrazino carbonyl) pyridinium chloride (compound no: 61).
N, N'-bis [3-carbonyl-1-(2-(5-chlorothien-2-yl)-2-oxoethyl) pyridinium] hydrazine dichloride. (compound no: 62).
1-(2-(5-methylthien-2-yl)-2-oxoethyl)-3-(methanesulfonyl hydrazino carbonyl) pyridinium chloride (compound no: 63).
1-(2-(4-nitrothien-2-yl)-2-oxoethyl)-3-(methanesulfonyl hydrazino carbonyl) pyridinium bromide. (compound no: 64).
1-(2-phenylamino-2-oxoethyl)-3-(phenyl hydrazino carbonyl) pyridinium chloride (compound no: 65).
1-(2-phenylamino-2-oxoethyl)-4 -[2-(benzoyloxy) ethylamino carbonyl] pyridinium chloride (compound no: 66).
1-2-(5-nitro-thien-2-yl)-2-oxoethyl)-3-(methanesulfonyl hydrazino carbonyl) pyridinium chloride (compound no: 67).
1-(2-thien-2'-yl-2-oxoethyl)-3-(trifluoromethanesulfonyl hydrazino carbonyl) - pyridinium bromide. (compound no. 68).
1-(2-thien-2'-yl-2-oxoethyl)-3-(phenyl hydrazino carbonyl) pyridinium bromide (compound no.69).
1-(2-thien-2'-yl-2-oxoethyl)-3-(p-methoxy phenyl sulfonyl hydrazino carbonyl) pyridinium bromide (compound no. 70).
1-(2-ethoxy-2-oxoethyl)-3-(phenyl aminocarbonyl hydrazino carbonyl) pyridinium bromide (compound no. 71).
1-(2-ethoxy-2-oxoethyl)-3-(p-toluene sulfonyl hydrazino carbonyl) pyridinium bromide (compound no. 72).
1-(2-phenyl-2-oxoethyl)-3-(phenylamino carbonyl hydrazino carbonyl) pyridinium bromide (compound no. 73).

1-(2-phenylamino-2-oxoethyl)-3-(benzyl sulfonyl hydrazino carbonyl) pyridiniumchloride. (compound no. 74).
1-(2-phenyl-2-oxoethyl)-4-(methanesulfonyl hydrazino carbonyl) pyridinium bromide (compound no. 75).
1-(2-phenyl-2-oxoethyl)-3-(phenyl hydrazino carbonyl) pyridinium bromide. (compound no. 76).
1-(2-ethoxy-2-oxoethyl)-4-[2-(benzoyloxy) ethyl amino carbonyl ] pyridinium bromide (compound no. 77).
1-(2-ethoxy-2-oxoethyl)-3-(phenyl hydrazino carbonyl) pyridinium bromide. (compound no. 78).
1-(2-phenyl-2-oxoethyl)-3-(p-methoxyphenyl sulfonyl hydrazino carbonyl) pyridinium bromide. (compound no. 79).
1-(2-phenyl-2-oxoethyl)- 4-[2-(benzoyloxy) ethyl amino carbonyl ] pyridinium bromide. (compound no. 80).
1-(2-ethoxy-2-oxoethyl)- 4-(p-methanesulfonyl hydrazino carbonyl) pyridinium bromide. (compound no. 81).
3-Carbonylamino-1- (2-(2, 4-dichlorophenyl)-2-oxoethyl)-pyridinium bromide (compound no. 82).
3- (Tetrahydrobenzothiazol-2-yl) aminocarbonyl -1-(2-(2, 4-dichlorophenyl)-2-oxoethyl)-pyridinium bromide (compound 83).
1-(2-Phenyl-2-oxoethyl)-3-((2-hydroxyethyl) aminocarbonyl) pyridinium bromide (compound 84).
3-Carbonylamino-1- (2-thien-2'-yl-2-oxoethyl)-pyridinium bromide (compound 85).
1-(2-Phenyl -2-oxoethyl)-3-((p-sulfonamidophenylene) aminocarbonyl) pyridinium bromide (compound 86).
1-(2-Ethoxy-2-oxoethyl)-3-((2-hydroxyethyl) aminocarbonyl) pyridinium bromide. (compound 87).
1-(2-Phenyl-2-oxoethyl)-3-(isopropyloxycarbonyl) pyridinium bromide (compound 88).
1-(2-Oxopropyl)-3-((2-hydroxyethyl) aminocarbonyl) pyridinium chloride (compound 89).
1-(2-Thien-2'-yl-2-oxoethyl)-3-((2-hydroxyethyl) aminocarbonyl) pyridinium bromide (compound 90).
1-(2-(2,4 - Dichlorophenyl-2-oxoethyl) -3- (isopropyloxycarbonyl) pyridinium bromide (compound 91).
1- (2-Phenyl - 2- oxoethyl) - 3- ((4-methylthiazol - 2 -yl) aminocarbonyl) pyridinium bromide (compound 92).
1-(2- Phenylamino -2- oxoethyl ) -3- (n-butyloxycarbonyl) pyridinium chloride (compound 93).
1-(2-Phenylamino - 2- oxoethyl) - 3 - (n-butylaminocarbonyl) pyridinium chloride (compound 94).
1- (2- Phenylamino - 2- oxoethyl)- 3- ((2-hydroxyethyl) aminocarbonyl) - pyridinium chloride (compound 95).
1- (2- (2,4 - Dichlorophenyl ) - 2-oxoethyl) - 3- (n - butoxycarbonyl) pyridinium bromide (compound 96).
1-(2 - (2, 4 - Dichlorophenyl) - 2-oxoethyl) - 3- (n-butylamino-carbonyl) pyridinium bromide (compound 97).
1-(2-Phenyl-2-oxoethyl)-3-(1-phenyl-1-oxomethyl) pyridinium bromide (compound 98).
1- (2 Phenyl - 2-oxoethyl) - 3- (methoxycarbonyl) pyridinium bromide (compound 99).

Table 1A -

| Representative Pyridinium derivatives (having m as 0 or 1 and $-COR_1$ at position 3) | | | | | |
|---|---|---|---|---|---|
| Compd. No. | $R_1$ | m | $-R_2$ | $-R_3$ | -X |
| 1 | Structure (a) | 0 | - | -Ph | Br |
| 2 | Structure (b) | 0 | - | OEt | Br |
| 3 | Structure (c) | 0 | - | 2,4-dichlorophenyl | Br |
| 4 | NHNH-(2-pyridyl) | 0 | - | OEt | Br |
| 5 | $NHNHSO_2CH_3$ | 0 | - | 2-thienyl | Br |
| 6 | Structure (d) | 0 | - | 2-thienyl | Br |
| 7 | $NHCH_2CH_2OCOPh$ | 0 | - | OEt | Br |
| 8 | $NHCH_2CH_2OCOPh$ | 0 | - | 2,4-dichlorophenyl | Br |
| 9 | NHNH-(2-pyridyl) | 0 | - | 2-thienyl | Br |
| 10 | NHNH-(2-pyridyl) | 0 | - | -Ph | Br |
| 11 | $NHNH_2$ | 0 | - | -Ph | Br |
| 12 | $NHNHSO_2CH_3$ | 0 | - | -Ph | Br |
| 13 | $NHNHSO_2CH_3$ | 0 | - | OEt | Br |
| 14 | NHNH-$SO_2$phenyl | 0 | - | -Ph | Br |
| 15 | NHNH-$SO_2$phenyl | 1 | 2-Cl | -Ph | Br |
| 16 | $OCH_2CH_2OCOCH_3$ | 0 | - | -Ph | Br |
| 17 | $OCH_2CH_2OCOPh$ | 0 | - | OEt | Br |

Table 1A - (continued)

| Representative Pyridinium derivatives (having m as 0 or 1 and $-COR_1$ at position 3) | | | | | |
|---|---|---|---|---|---|
| Compd. No. | $R_1$ | m | $-R_2$ | $-R_3$ | -X |
| 21 | $-NHNH-SO_2Ph$ | 0 | - | OEt | Br |
| 22 | $-OCH_2CH_2OCH_3$ | 0 | - | 2,4-dichlorophenyl | Br |
| 23 | $-NHCH_2CH_2OCOPh$ | 0 | - | -NHPh | Cl |
| 24 | -NHNHCONHPh | 0 | - | 2-thienyl | Br |
| 25 | $NHCH_2CH_2OCOCH_3$ | 0 | - | -Ph | Br |
| 26 | $NHNHSO_2Ph$ | 0 | - | -NHPh | Cl |
| 27 | $NHNHSO_2Ph(4\text{-}CH_3)$ | 0 | - | -NHPh | Cl |
| 28 | $OCH_2CH_2OCOPh$ | 0 | - | -Ph | Br |
| 29 | -NHNHCOPh | 0 | - | 2-thienyl | Br |
| 30 | $NHNHSO_2CH_2Ph$ | 0 | - | OEt | Br |
| 31 | $NHNHSO_2CH_2Ph$ | 0 | - | -Ph | Br |
| 32 | Structure (e) | 0 | - | 2-furyl | Br |
| 33 | Structure-(f) | 0 | - | 2-thienyl | Cl |
| 34 | $NHNHCOCH_2CH_2$-cyclohexyl | 0 | - | 2-thienyl | Br |
| 35 | $NHNHCOCH_2CH_2$-cyclohexyl | 0 | - | -NHPh | Cl |
| 36 | $NHCH_2CH_2OCO$-phenyl | 0 | - | 2-thienyl | Br |
| 37 | $NHCH_2CH_2OCO$-phenyl | 0 | - | $CH_2CO_2$-ethyl | Cl |
| 38 | $-NHCH_2CH_2OCH_3$ | 0 | - | -2,4-dichlorophenyl | Br |
| 39 | Structure-(g) | 0 | - | NH-cyclopropyl | Cl |
| 40 | $-NHCH_2CH_2OCH_3$ | 0 | - | NH-cyclopropyl | Cl |
| 41 | Structure-(h) | 0 | - | NH-isopropyl | Cl |
| 42 | Structure-(i) | 0 | - | 2-thienyl | Cl |
| 43 | $NHNHSO_2CH_3$ | 0 | - | NH-isopropyl | Cl |
| 44 | $NHNHSO_2CH_3$ | 0 | - | 1-pyrrolidinyl | Cl |
| 45 | $NHNHSO_2CH_3$ | 0 | - | 2-thienyl | Cl |
| 46 | Structure-(j) | 0 | - | -OH | Cl |
| 47 | $NHCH_2CH_2OCH_3$ | 0 | - | 2-thienyl | Cl |
| 48 | Structure-(k) | 0 | - | 2-thienyl | Cl |
| 49 | Structure - (l) | 0 | - | 2-thienyl | Cl |
| 50 | $-NHNHSO_2$isopropyl | 0 | - | 2-thienyl | Br |
| 51 | $-NHNHSO_2CH_3$ | 0 | - | Structure (m) | Cl |
| 52 | $-NHNHSO_2CH_3$ | 0 | - | Structure (n) | Cl |
| 53 | $-NHNHSO_2CH_3$ | 1 | 5-Bromo | 2-thienyl | Br |
| 54 | $-NHHCO_2C_2H_5$ | 0 | - | 2-thienyl | Br |
| 55 | $-NHNHSO_2CH_3$ | 0 | - | 5-chloro-2-thienyl | Br |
| 56 | Structure (o) | 0 | - | 4-nitro-2-thienyl | Cl |
| 57 | $-NHNHSO_2CH_3$ | 1 | 6-methyl | 2-thienyl | Br |

Table 1A - (continued)

| Representative Pyridinium derivatives (having m as 0 or 1 and $-COR_1$ at position 3) | | | | | |
|---|---|---|---|---|---|
| Compd. No. | $R_1$ | m | $-R_2$ | $-R_3$ | -X |
| 58 | Structure (p) | 0 | - | 5-methyl-2-thienyl | Cl |
| 59 | Structure (q) | 0 | - | Structure (n) | Cl |
| 60 | Structure (r) | 0 | - | 2-thienyl | Cl |
| 61 | $-NHNHSO_2CH_3$ | 0 | - | Structure (s) | Cl |
| 62 | Structure (t) | 0 | - | 5-chloro-2-thienyl | Cl |
| 63 | $-NHNHSO_2CH_3$ | 0 | - | 5-methyl-2-thienyl | Cl |
| 64 | - $NHNHSO_2CH_3$ | 0 | - | 4-nitro-2-thienyl | Br |
| 65 | -NHNHPh | 0 | - | -NHPh | Cl |
| 67 | $-NHNHSO_2CH_3$ | 0 | - | 5-nitro-2-thienyl | Cl |
| 68 | $-NHNHSO_2CF_3$ | 0 | - | 2-thienyl | Br |
| 69 | -NHNHPh | 0 | - | 2-thienyl | Br |
| 70 | $-NHNHSO_2$-4-methoxy-Phenyl | 0 | - | 2-thienyl | Br |
| 71 | -NHNHCONHPh | 0 | - | -OEt | Br |
| 72 | $-NHNHSO_2$-4-methyl-Phenyl | 0 | - | -OEt | Br |
| 73 | -NHNHCONHPh | 0 | - | -Ph | Br |
| 74 | $-NHNHSO_2CH_2Ph$ | 0 | - | -NHPh | Cl |
| 76 | -NHNHPh | 0 | - | -Ph | Br |
| 78 | -NHNHPh | 0 | - | -Oet | Br |
| 79 | $-NHNHSO_2$-4-methoxy-Phenyl | 0 | - | -Ph | Br |
| 82 | $NH_2$ | 0 | - | 2,4-dichorophenyl | Br |
| 83 | Tetrahydrobenzo-thiazol-2-yl-amino | 0 | - | 2,4-dichorophenyl | Br |
| 84 | $NHCH_2CH_2OH$ | 0 | - | -Ph | Br |
| 85 | $NH_2$ | 0 | - | 2-thienyl | Br |
| 86 | (p-sulfonamido-phenylene)amino | 0 | - | -Ph | Br |
| 87 | $NHCH_2CH_2OH$ | 0 | - | OEt | Br |
| 88 | $OCH(CH_3)_2$ | 0 | - | -Ph | Br |
| 89 | $NHCH_2CH_2OH$ | 0 | - | $CH_3$ | Cl |
| 90 | $NHCH_2CH_2OH$ | 0 | - | 2-thienyl | Br |
| 91 | $OCH(CH_3)_2$ | 0 | - | 2,4-dichorophenyl | Br |
| 92 | (4-methylthiazol-2-yl)amino | 0 | - | -Ph | Br |
| 93 | $OCH_2CH_2CH_2CH_3$ | 0 | - | -NHPh | Cl |
| 94 | $NHCH_2CH_2CH_2CH_3$ | 0 | - | -NHPh | Cl |
| 95 | $NHCH_2CH_2OH$ | 0 | - | -NHPh | Cl |
| 96 | $OCH_2CH_2CH_2CH_3$ | 0 | - | 2,4-dichorophenyl | Br |
| 97 | $NHCH_2CH_2CH_2CH_3$ | 0 | - | 2,4-dichorophenyl | Br |
| 98 | -Ph | 0 | - | -Ph | Br |
| 99 | $OCH_3$ | 0 | - | -Ph | Br |

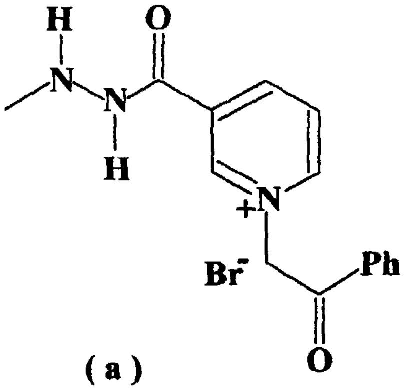

(a)

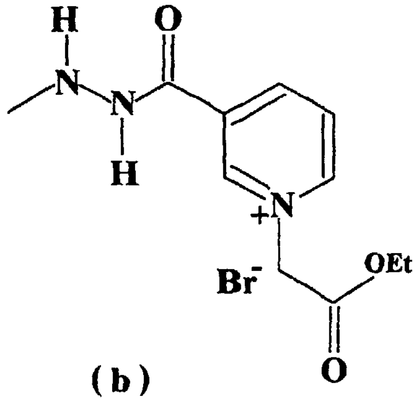

(b)

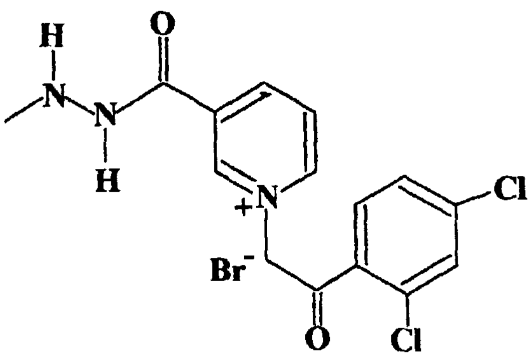

(c)

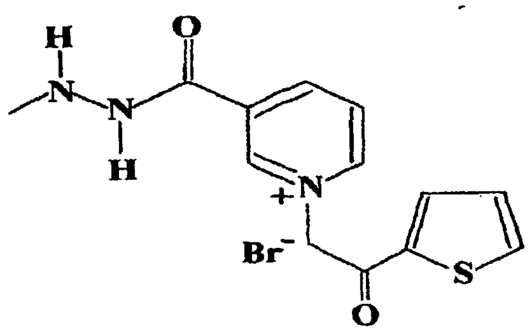

(d)

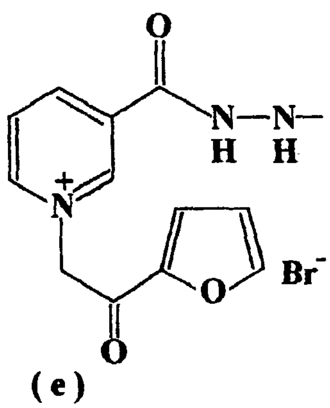

(e)

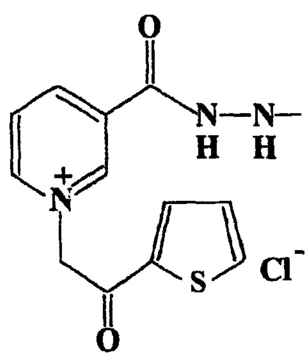

(f)

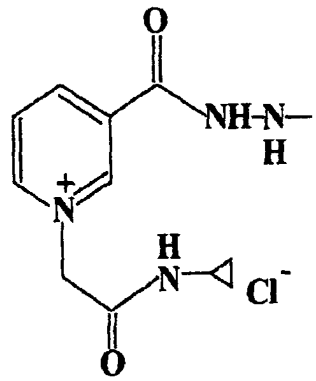

(g)

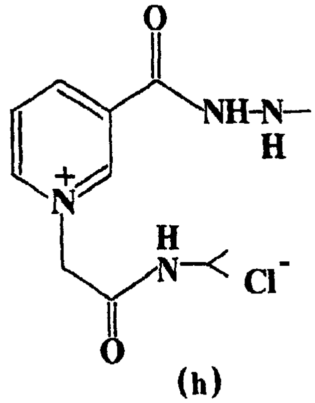

(h)

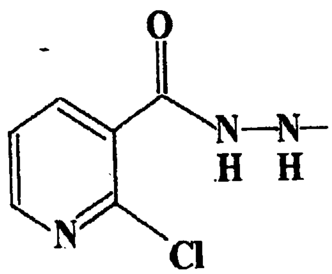

( i )

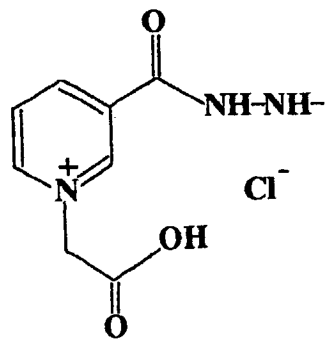

( j )

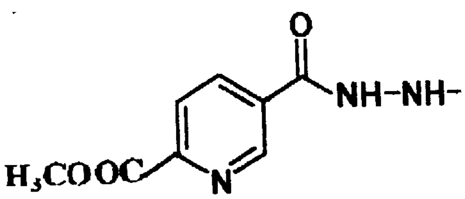

Structure ( k )

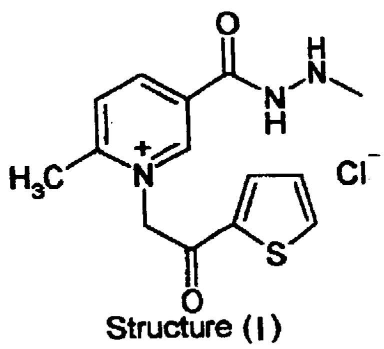

Structure ( l )

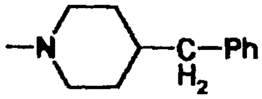

Structure (m)

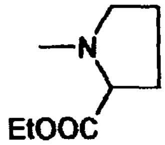

Structure (n)

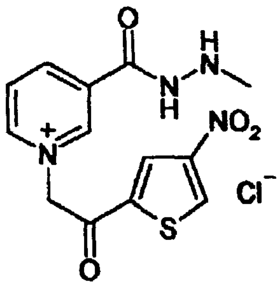

Structure (o)

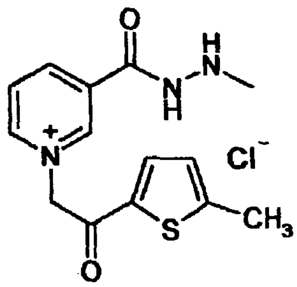

Structure (p)

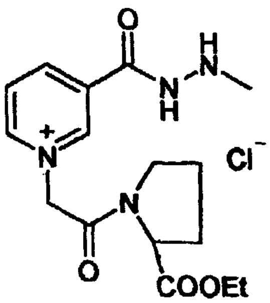

Structure (q)

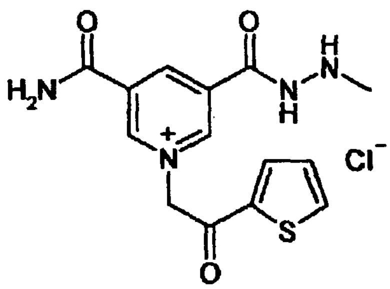

Structure ( r )

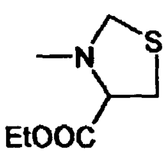

Structure (S)

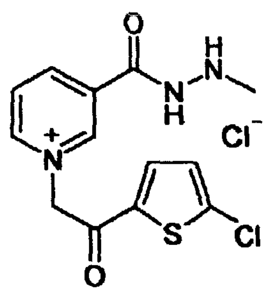

Structure ( t )

Table 1B-

| Representative Pyridinium derivatives (having m as 0 and $-COR_1$ at position 4) | | | | |
|---|---|---|---|---|
| **Compound No.** | **$-R_1$** | **$-R_2$** | **$-R_3$** | **-X** |
| 18 | $NHCH_2CH_2OCOPh$ | - | 2-thienyl | Br |
| 19 | $NHNHSO_2Ph$ | - | OEt | Br |
| 20 | $NHNHSO_2Ph$ | - | -NHPh | Cl |
| 66 | $-NHCH_2CH_2OCOPh$ | - | -NHPh | Cl |
| 75 | $-NHNHSO_2CH_3$ | - | -Ph | Br |
| 77 | $-NHCH_2CH_2OCOPh$ | - | -OEt | Br |
| 80 | $-NHCH_2CH_2OCOPh$ | - | -Ph | Br |
| 81 | $-NHNHSO_2CH_3$ | - | -OEt | Br |

The Compounds of general formula (I) can be prepared by known process. For example compound 1, may be prepared by adding a solution of phenacyl bromide in isopropanol to N,N'-bis-(nicotinyl)hydrazine dissolved in methanol, refluxing for six hours, cooling, filtering the precipitated solid, washing the solid with hot ethyl acetate and finally purifying the solid with 20 ml of methanol: ethyl acetate (3 : 1) to yield the desired compound.

Similarly, the other compounds of general formula I, can be prepared from properly substituted pyridine derivatives followed by quarternization with appropriate reagent by refluxing in alcoholic solvents like, methanol, ethanol, propanol, etc and high boiling solvents like toluene or xylene etc, for 6 - 48 hrs. to give the desired compounds.

The examples of substituted pyridine derivatives which can be used for preparation of specific compounds of the invention are given below:

1. N,N' -bis(nicotinyl)hydrazine
2. 3-[(2-pyridyl)hydrazinocarbonyl]pyridine
3. 3-[2-methanesulfonyl)hydrazinocarbonyl]pyridine
4. 3-[(2-benzoyloxy)ethylaminocarbonyl]pyridine
5. 3-[(2-phenylsulfonyl)hydrazinocarbonyl]pyridine
6. 3-[(2-acetoxy)ethyloxycarbonyl]pyridine
7. 3-[(2-benzoyloxy)ethyloxycarbonyl]pyridine
8. 3-[(2-methoxy)ethyloxycarbonyl]pyridine
9. 3-[(2-phenylaminocarbonyl)hydrazinocarbonyl]pyridine
10. 3-[(2-acetoxy)ethylaminocarbonyl]pyridine
11. 3-[(2-(4-methylphenyl sulfonylhydrazinocarbonyl))]pyridine
12. 3-[(2-benzoyl)- hydrazino carbonyl]pyridine
13. 3-[(2-phenylmethane sulfonyl) hydrazino carbonyl]pyridine
14. 3-[(2-(3- cyclohexylpropanoyl) hydrazino carbonyl]pyridine
15. 3-[(2-methoxy)ethylaminocarbonyl]pyridine
16. 3-[1-oxo-1-(2-methoxycarbonyl)pyridyl]hydrazino pyridine

The examples of quaternizing agents which may be used in the reaction are given below:

1. 2-bromoacetyl thiophene
2. 2-chloroacetyl thiopene
3. phenacylbromide
4. phenacylchloride
5. 2,4-dichloropheanacylbromide
6. N- phenyl chloroacetamide
7. N- cyclopropyl chloroacetamide
8. ethylbromoacetate
9. bromo acetylfuran
10. N- isopropylchloroacetamide

11. N- chloroacetyl-2-pyrrolidinone
12. chloroacetic acid
13. N-chloroacetyl-4-carboethoxythiazolidine

**In-vitro screening for AGE-breaking Activity**

The in vitro AGE breaking activity, of the representative compounds of the invention has been studied in the laboratory, by incubating reducing sugar glucose, with protein bovine serum albumin, which resulted in browning of solution and increase in the fluorescence. Fluorescence was used as the criteria to monitor the increased AGE formation.

**Example 1A**

**AGE breaking activity has been confirmed by the screening procedure as mentioned** below:

Materials:

Bovine serum albumin (fraction V) (BSA)
Glucose, analytical grade
Phosphate buffered saline (PBS)

Equipment:

Microplate ELISA Reader - Spectramax Plus (Molecular Devices, USA)
Microplate washer, (Bio -Tec Instruments, USA)
pH meter
Methods of experiment: Elisa (Enzyme Linked Immunosorbent Assay)

160 mg/ml of protein, bovine serum albumin, BSA and 1.6M glucose sugar were dissolved in phosphate buffered saline, PBS. Sodium azide was added at 0.02% concentration as a preservative. The solution was filtered asceptically through a 0.22 μM filter and kept for aging at 37°C for 16 weeks. After 16 weeks the solution was dialyzed against PBS, aliquoted and stored at - 20°C.

To determine the AGE breaking activity, 10 μg/ml of the 16 weeks AGE-BSA was incubated with different concentrations of the test compounds at 37°C for 24 hours and AGE breaking activity of the test compounds by ELISA was determined.

ELISA was performed as follows:

1. Different concentrations of 16 weeks AGE-BSA were coated on a microtitre plate as standard. Each concentration is coated in triplicates.
2. The test samples were coated on microtitre plate at a concentration of 5 ng. to 20 ng per well in triplicates.
3. The plate was incubated at 37°C for one hour.
4. After incubation the plate was washed with PBST (PBS with 0.05% Tween 20).
5. Blocking with 5% skimmed milk in PBS at 37°C for one hour was done.
6. The plate was washed with PBST.
7. Primary antibody against AGE-BSA was added and the plate is incubated at 37°C for one hour.
8. The plate was washed with PBST
9. Secondary antibody anti rabbit HRPO (Horse-Radish Per Oxidase) conjugate was added and the plate is incubated at 37°C for one hour.
10. The plate was washed with PBST.
11. Colour development with OPD (orthophenylenediamine dihydrochloride) and hydrogen peroxide was done.
12. OD (optical density) at (450nm reading - 620nm reading) was measured after incubation at 37°C for 15 minutes with Microplate ELISA Reader.

The breaker activity of the compounds were determined by the following formula:

$$\% \text{ Breaker activity} = \frac{OD_{450\text{-}620}\text{Control} - OD_{450\text{-}620}\text{Test}}{OD_{450\text{-}620}\text{Control}} \times 100$$

$OD_{450\text{-}620}$Control= Absorbance of 20ng AGE-BSA after incubation at 37°C for 24 hours without test compound
$OD_{450\text{-}620}$Test= Absorbance of 20ng AGE-BSA after incubation at 37°C for 24 hours with required concentration of test compound

Using specific examples, the % AGE breaking activity was calculated and recorded in Table 2.

Table 2

| **Sample** | **Concentration** | **% Breakage** |
|---|---|---|
| PTB | 10 mM<br>20 mM | 27<br>47 |
| Compound 1 | 5 mM | 13 |
| Compound 4 | 10 mM | 30 |
| Compound 5 | 10 mM<br>50 mM | 16<br>68 |
| Compound 6 | 5 mM | 53 |
| Compound 7 | 20 mM | 36 |
| Compound 16 | 10 mM | 16 |
| Compound 17 | 10 mM | 19 |
| Compound 22 | 10 mM<br>25 mM | 13<br>41 |
| Compound 23 | 10 mM<br>25 mM | 37<br>90 |
| Compound 32 | 10 mM | 14 |
| Compound 33 | 5 mM | 20 |
| Compound 38 | 5mM | 17.66 |
| Compound 39 | 5mM | 22.8 |
| Compound 40 | 10mM | 12.38 |
| Compound 42 | 10mM | 12.51 |
| Compound 43 | 10mM | 10.85 |
| Compound 45 | 10mM | 17.53 |
| Compound 47 | 10mM | 32.38 |
| Compound 49 | 2.5 mM | 85.67 |
| Compound 50 | 10 mM | 31.45 |
| Compound 51 | 10 mM | 20.94 |
| Compound 52 | 10 mM | 25.34 |
| Compound 53 | 2.5 mM | 29.36 |
| Compound 54 | 10 mM | 33.43 |
| Compound 55 | 10 mM | 40.85 |
| Compound 56 | 10 mM | 75.92 |
| Compound 57 | 1.0 mM | 77.69 |

Table 2 (continued)

| Sample | Concentration | % Breakage |
|---|---|---|
| Compound 58 | 10 mM | 81.95 |
| Compound 59 | 10 mM | 20.31 |
| Compound 60 | 1 mM | 95.36 |
| Compound 61 | 10 mM | 25.06 |
| Compound 62 | 10 mM | 78.41 |
| Compound 63 | 10 mM | 25.17 |
| Compound 64 | 10 mM | 60.94 |
| Compound 65 | 2.5 mM | 68.35 |
| Compound 66 | 10 mM | 19.07 |
| Compound 67 | 1 mM | 42.01 |
| Compound 68 | 10 mM | 92.64 |
| Compound 85 | 10 mM | 3 |
| Compound 87 | 10 mM | 43 |
| Compound 90 | 10 mM | 50 |
| Compound 94 | 10 mM | 27 |
| Compound 93 | 10 mM | 57 |
| Compound 95 | 20 mM | 48 |

Hence compounds 4,6, 23,33,39, 47, 49, 50, 53-58, 60, 62, 64, 65, 67 and 68 have superior AGE breaking activity compared to PTB, of which the potency of compounds 49, 56-58,60 62, 64, 65, 67 and 68 are significantly much higher.

**Example 1B**

**In-vivo AGE breaking**

Diabetes and aging process bears a good degree of resemblance in a sense, that both are degenerative processes where normal proteins, like collagen become cross linked to form AGE. These AGE formulations will in turn result in complication of cosmetic compliance. Further collagenous proteins are especially prone to non-enzymatic glycation because they contain several diabetic amino acid residues with free amino groups, have very slow turnover rate and are exposed to ambient levels of glucose. Advanced Glycation has been shown to induce a decrease in susceptibility of collagen to digestion i.e., collagen becomes less soluble. Such cross linked collagen increases complications of aging.

To evaluate AGE breaker efficacy of compounds of formula (I) the process of aging was mimicked by induction of diabetes in male wister rats. Diabetes was induced in male wister rats (by repeated STZ injections) to mimic the process of aging and formation of AGE. Diabetic male wister rats were treated with compounds of formula (I) for a period of 8 weeks after induction of diabetes and collagen solubility was measured in Rat-Tail Tendon. Rat Tail Tendon were selected because of their similarity with skin collagen (i.e. Type I collagen). After 8 weeks of treatment the "control" and "treated" groups of animals were sacrificed and tail tendons were homogenized, digested with hydrochloric acid and the supernatant of both the groups of animals was assayed for hydroxyprolin for acid soluble collagen contents as per method of Stegemann and Stalder (clinica Chimica Acta, 18 (1967) 267-273). The animals treated with compounds of formula (I) demonstrated the increase in collagen solubility versus the diabetic control group. The results are mentioned in Table 3 below:

| Sr. No. | Compound No. | Concentration | % Improvement in collagen solubility with respect to untreated control subject. |
|---|---|---|---|
| 1. | 5 | 10.0mg/kg | 88.80 |
| 2. | 6 | 7.5mg/kg | 56.70 |
| 3. | 33 | 15.0 mg/kg | 80.45 |
| 4. | 39 | 6.0mg/kg | 28.00 |

Diabetes and aging are degenerative process where normal proteins like collagen become cross-linked. The cross linking of skin collagen can lead to complications like Aging. To test the ability of compounds of invention to break pre-formed AGE, diabetic animals were treated for 8 weeks with the compounds. After the treatment the collagen solubility was estimated to determine their AGE breaking activity. The results indicate that the ability of compounds of formula (I) break AGE formed in collagen and improve the solubility of previously cross linked collagen. A brief summary of observation is as follows.

The diabetic rats showed a decrease in the solubility of Rat Tail Tendon Collagen, indicative of AGE cross link formation, as compared to normal age matched controls. The Animals treated for 8 weeks with the compounds of the invention showed an improvement in the collagen solubility, an indication of AGE cross link breakage. This ability of test compounds to break preformed AGE will be beneficial for cosmetic purposes.

<u>**Example 1C**</u>

**Free Radical Scavenging Activity:**

This method measures the relative ability of free radical scavenging substances to scavenge the ABTS $^{\cdot+}$ i. e. 2,2-Azino-bis-(3-ethyl benzo thiazoline-6-sulfonate) radical cation as compared to a standard amount of standard or free radical scavengers antioxidants. Incubation of ABTS with Peroxidase (metmyoglobin) and hydrogen peroxide results in the production of radical cation ABTS$^{\cdot+}$ . This species is blue- green in colour and can be detected at 730nm. Antioxidants or free radical scavengers in the added sample that causes suppression of the color to a degree that is proportional to their concentration.

**Protocol:**

**Preparation of Buffer solutions:**

A. Phosphate Citrate Buffer (pH 5.0): 48.5ml of 0.1M citric acid with sufficient 0.2M disodium hydrogen phosphate to produce 100 ml.
B. Phosphate Buffer Saline (PBS): Dissolve 40.0g of NaCl, 1.0g of KCl, 1.0g of $KH_2PO_4$ and 3.05g of $Na_2HPO_4$ in 1 litre milli-Q water. Dilute 200ml of above solution to 1 litre with milli-Q water (pH 7.4-7.6).
C. 3μM stock solution of Peroxidase was prepared in phosphate buffer saline pH 7.4(PBS).
D. 1.08 mM stock solution of Hydrogen peroxide was prepared in phosphate buffer saline pH 7.4 (PBS).

**Preparation of ABTS Stock solution:**

1 tablet (10mg) was dissolved in phosphate citrate buffer (pH 5.0).

**Preparation of ABTS working solution:**

5.0ml of ABTS stock solution was diluted with PBS to 20 ml.

**Preparation of Horse Radish Peroxidase stock solution:**

320 μg of the enzyme was dissolved in 2.5 ml of PBS.

**Preparation of Hydrogen Peroxide (1.08mM) solution:**

12µl of Hydrogen Peroxide (30%w/v) was diluted to 100ml with PBS.

**Preparation of Drug solutions:**

0.1mM of stock solution of the drug was prepared which was serially diluted in PBS to get solutions of different concentrations.

**Preparation of ABTS radical stock solution:**

To 18ml of ABTS stock solution, 100µl of Horseradish Peroxidase stock solution was added.
As soon as 1.5 ml of hydrogen peroxide solution was added to the above solution, blue-green colour of the ABTS radicals appeared. This solution was incubated at 30°C for 30 min.

**Preparation of control solution:**

980µl of ABTS radical stock solution was added to an eppendorf tube. To it was added 100µl of various PBS solution.

**Preparation of test solution:**

980µl of ABTS radical stock solutions were added to different eppendorf tubes. To it were added 100µl of various concentrations of drug solution.

**Measurement of absorbance (O.D):**

The absorbance of control and test samples was recorded immediately at 730nm taking PBS as blank.
Calculation: The percent antioxidant activity was calculated according to the formula: % Antioxidant activity = [O.D of test sample/O.D of control*100]-100. The results are tabulated in Table 3 below.

TABLE 3

| Sr. No. | Compound No. | Relative Free Radical Scavenging Activity(%) on ABTS | | | | |
|---|---|---|---|---|---|---|
| | | 11.5µM | 23.0µM | 46.0µM | 92.5µM | 925µM |
| 1 | Niacinamide | 0.0 | 2.2 | 3.8 | 3.8 | 9.2 |
| 2 | Compound No.5 | 47.2 | 65.6 | 83.7 | 100 | - |
| 3 | Compound No.33 | 58.4 | 72.8 | 98.5 | 100 | - |
| 4 | Compound No.39 | 43.0 | 62.8 | 95.6 | 98 | - |
| 5 | Compound No.22 | 27.9 | 50.9 | 69.3 | 79.7 | 100 |
| 6 | Compound No.8 | 40.8 | 53.7 | 72.6 | 81.2 | 100 |
| 7 | Compound No. 36 | 27.6 | 59.2 | 74.2 | 83.9 | 100 |
| 8 | Compound No.82 | 26.0 | 44.3 | 62.7 | 75.5 | 100 |
| 9 | Compound No.90 | 47.8 | 58.8 | 78.2 | 99.5 | 100 |
| 10 | Compound No.4 | 58.2 | 64.3 | 69.3 | 98.6 | 100 |
| 11 | Compound No.93 | 8.5 | 8.6 | 9.6 | 11.4 | 25.0 |
| 12 | Compound No.56 | 43.0 | 57.2 | 71.6 | 97.7 | 100 |
| 13 | Compound No.64 | 41.8 | 55.7 | 72.2 | 99.0 | 100 |
| 14 | Compound No.97 | 16.5 | 27 | 37.8 | 44.5 | 97.8 |
| 15 | Compound No.18 | 41.8 | 59.5 | 75.2 | 91.0 | 100 |

TABLE 3 (continued)

| Sr. No. | Compound No. | Relative Free Radical Scavenging Activity(%) on ABTS | | | | |
|---|---|---|---|---|---|---|
| | | 11.5μM | 23.0μM | 46.0μM | 92.5μM | 925μM |
| 16 | Compound No.27 | 43.9 | 55.5 | 75.3 | 99.6 | 100 |
| 17 | Compound No.31 | 41.6 | 57.7 | 66.9 | 99.2 | 100 |
| 18 | Compound No.98 | 7.1 | 26.9 | 51.1 | 78.6 | 99.1 |
| 19 | Compound No.34 | 50.3 | 53.8 | 65.4 | 80.4 | 100 |
| 20 | Compound No.35 | 50.5 | 63.4 | 80.9 | 90.6 | 100 |
| 21 | Compound No.38 | 47.2 | 54.8 | 71.0 | 89.5 | 100 |
| 22 | Compound No.45 | 53.9 | 56.2 | 78.7 | 99.8 | 100 |
| 23 | Compound No.60 | 44.1 | 59.8 | 69.4 | 99.9 | 100 |
| 24 | Compound No.54 | 5.22 | 15.45 | 46.82 | 77.07 | - |
| 25 | Compound No.55 | 46.47 | 84.97 | 99.50 | 99.93 | - |
| 26 | Compound No.63 | 10.75 | 47.20 | 88.64 | 99.18 | - |
| 27 | Compound No.64 | 26.01 | 57.39 | 99.30 | 99.62 | - |
| 28 | Compound No.65 | 27.67 | 59.20 | 94.14 | 100.00 | - |
| 29 | Compound No.67 | 13.11 | 50.70 | 97.46 | 99.43 | - |
| 30 | Compound No.68 | 38.47 | 43.54 | 89.32 | 95.07 | - |
| 31 | Compound No.50 | 3.30 | 23.06 | 54.95 | 89.31 | - |
| 32 | Compound No.51 | 0.00 | 21.64 | 52.82 | 83.94 | - |

## AGE Inhibiting Activity of the Compounds

Apart from the AGE breaking and free radical scavenging activity of the compounds of the invention their potential to inhibit AGE make them ideal for different cosmetic applications as discussed above.

Further in view of the ability of the compounds of the instant invention to prevent the onset of AGE formation by the inhibitory action now discovered, development of pathology condition caused by AGE could be prevented or reduced. The dual activities of the compounds as AGE breaker and also as AGE inhibitor make them even more useful for the disease related to aging and diabetic complications, kidney diseases, nerve damage, retinopathy, neuropathy, endothelial dysfunction, atherosclerosis, micro angiopathy, browning that occurs in the oral cavity like browning of tooth, alzheimer, artirial compliance and distensibility, restenosis, erectile dysfunction and other dysfunction wherein the load of AGE on the cell is very crucial. In fact a triple action of the compounds (a) AGE breaker (b) AGE inhibitor (c) Free radical scavenger can be effectively utilized for reversal of prevention of several pathological conditions as well as reversal and prevention of cosmetic aspects of aging.

The correlation between the onset of AGE with various diseases has been described in various literature as discussed below.

The correlation between the formation of Advanced Glycation End products (AGE) and nephropathy is well established by several research publications. Beisswenger (1995) has shown that AGE concentration in human diabetic subjects correlates with early manifestation of renal diseases. Makita et al (1991) has shown that increase in AGE peptides parallels with the severity of renal dysfunction. The above citations clearly show that AGE is the principal cause of diabetic nephropathy. Yamauchi (1997) showed that prevention of AGE formation by aminoguanidine inhibits development of diabetic nephropathy. Aminoguanidine administration is also shown to ameliorate thickening of glomerular basement membrane of diabetic rats (Ellis 1991). Aminoguanidine is also shown to attenuate the rise in albuminuria in experimental diabetic rats (Soulis-Liparota, 1991).

AGE is also shown to induce expression of vascular endothelial growth factor in retinal muller cells (Hirata, 1997, Murata, 1997) and therefore may promote intraocular neovascularization in diabetic retinopathy. Aminoguanidine treatment is shown to retard progression of diabetic retinopathy in rat model (Hammes, 1991, Hammes, 1994, Roufail, 1998).

Aminoguanidine treatment is also shown to improve nerve conduction velocity in diabetic rats (Kihara, 1991

and Miyauchi, 1996 and Yagihashi, 1992).

Bucala (1996) has extensively reviewed various aspects of development of Atheroscelrosis. They stated that accumulation of AGE can trigger a series of cellular events, such as cellular oxidative stress, expression of adhesion molecules, endothelial transmigration of monocytes, etc. and these events can lead to atherosclerosis. Kirstein (1990) have demonstrated that (i) in vitro and in vivo-formed.

AGE proteins are chemotactic for human blood monocytes, (ii) sub-endothelial AGE can induce monocyte migraton across intact endothelium and (iii) interaction of monocyte with AGE containing matrix results into induction platelet derived growth factor.

Thus, it can be concluded that AGE, upon interaction with endothelial cells through its receptor RAGE, activate nuclear factor Kappa B and induce various genes expressing adhesion molecules. AGE-endothelium interactions also increase oxidative stress, initiate monocyte migration, block endothelial nitric oxide and stimulate angiogenesis. All these conditions result in conditions such as atherosclerosis.

Other dysfunctions demanding lower tissue AGE burden include, Hypertension, Restenosis, Erectile Dysfunction and Alzheimer disease. Similarly, on the other hand, non-enzymatic cross-linking of structural proteins, such as collagen, leads to increased stiffness of arteries and reduce arterial compliance and distensibility. In fact, treatment of AGE-breaker ALT-711 is shown to reverse diabetes induced increase of arterial stiffness and improve arterial compliance (Wolffenbutel 1998). Aronson et al (1996) have reviewed role of AGE in promoting inflammatory cell recruitment and smooth muscle proliferation and suggested it to be a likely reason for greater restenosis rate in diabetic patients.

Seftel (1997) has shown significant elevation of pentosidine in the penile tissue of diabetic patients as compared to non-diabetic. They have speculated a mechanism for AGE mediated erectile dysfunction via upregulation of inducible nitric oxide and downregulation of endothelial nitric oxide in penile tissues.

Vitek et al (1994) have reported that beta amyloid peptides (βAP) aggregate slowly under normal physiological conditions whereas AGE modified (βAP) showed a much more rapid aggregation. Plaque numbers increase in association with neuronal degeneration and cognitive decline in AD. Aggregated but not monomeric βAP is actively neurotoxic. Hence interference with the process by which AGE formation enhances βAP aggregation or inhibition of AGE formation or AGE breaker therapy will provide new therapeutic opportunities to reduce the pathophysiological changes associated with AD.

Hence AGE inhibitors/breakers would be beneficial in reducing the aggregation of βAP, leading to the prevention/ treatment of Alzheimer's disease.

Li et al (1996) have provided evidence for an interrelationship between two key manifestations of physiological aging in the rat cardiovascular and renal decline and the spontaneous age associated biochemical process termed advanced glycation thought to contribute to progressive tissue damage and organ failure. In their study aminoguanidine (an AGE inhibitor) was found to significantly prevent tissue damage as a result of inhibiting AGE formation. Lower tissue AGE burden in rats as a result of aminoguanidine administration was found to preserve an altogether more satisfactory level of cardiovascular and renal function as evidenced by the generally healthier appearance of old rats treated by aminoguanidine as compared to the untreated age and weight matched controls. Hence AGE inhibitors could be used for the prevention of aging related disorders.

The nonenzymatic browning reaction, which occurs in the oral cavity, results in the discoloration of teeth. Antiplaque agents such as chlorhexidine have been reported to accelerate the non-enzymatic browning reaction and further the staining of teeth. (Nordbo, J. Dent. Res., 58, p. 1429 (1979)). Nordbo has proposed that chlorhexidine results in tooth staining in two ways: first, by increasing the formation of pelicle which contains more amino groups, and secondly, by catalysis of the Malliard reaction leading to colored products.

The ability of inhibitors of non-enzymatic browning reaction to prevent the discoloration of protein on a surface, such as that which occurs on the tooth surface has been demonstrated with in vitro experiments in US pat. 5,137,916; US Pat. 5,272,176.

Compounds that have the ability to inhibit or reverse AGE have been claimed to be useful for the inhibiting or reversing the discoloration of teeth resulting from non-enzymatic browning in the oral cavity. (US Pat. 5,272, 176; US Pat. 5,853,703)

All these evidences point out to a common underlying mechanism for the pathophysiological conditions associated with diabetes and that is the formation of Advanced Glycation Endproducts. As the total tissue burden of AGE increases, the severity of the pathological symptoms too increase. On the other hand, if the quantum of AGE is controlled by the compounds like Aminoguanidine, the progression of disease is also retarded. In the present invention, the inhibition of Advanced Glycation Endproducts is described.

Reducing the tissue burden of AGE is expected to reverse these conditions, whereas preventing accumulation upto critical mass could prevent the condition from occurring.

**Example 1D**

**Test for AGE inhibiting activity.**

The following method was used to determine the inhibitory effect of the test compound on Maillard reaction in-vitro. This method is adopted from US Patent No. 5, 514, 676 and European Patent Publication no. 0 339 496 A2.

**Method I**

A solution of Bovine Serum Albumin (BSA), ribose and test compound was prepared in Phosphate Buffer Saline (PBS, pH 7.4) so as to have final concentration of BSA and ribose at 10mg/ml and 500mM respectively. Addition of compound was done in aseptic conditions. Sodium azide (0.02%) was also added in this solution in order to prevent microbial growth. A separate tube containing BSA, ribose and sodium azide in the same concentration and buffer as above, but without any test compound, was also incubated as positive control.

After incubation at 37° C for 7 days, 40 micro litre sample from each tube was removed and diluted with PBS to have final concentration of BSA at 1mg/ml. The fluorescence of all the samples was measured at Excitation Maximum of 355nM and the Emission Maximum of 460nM using f-MAX Fluorimeter (Molecular Device, USA). In order to study the effect of test compound on fluorescence, freshly prepared compound solution was mixed with previously incubated positive control (i.e. BSA + ribose), so as to achieve same concentration of all the components as that of test samples.

The percent inhibition of test compound was measured as follows:

$$\% \text{ Inhibition} = \frac{F4 - F3}{F4} X\ 100$$

**Where F3 = Fluorescence of BSA + ribose + compound,F4 is fluorescence of incubated (BSA + ribose) + freshly added test compound.**

The representative compounds of general formula (I) have been tested for the activity as AGE inhibitor.

Table - 4

| Compound No. | Concentration | %Inhibition (Day 7) |
|---|---|---|
| 5 | 10mM | 70 |
| 33 | 2.5mM | 68.4 |
| 7 | 10mM | 51 |
| 54 | 2.5mM | 46.3 |
| 63 | 6.25mM | 58.15 |

It is thus found that the compounds of general formula (I) as defined above are capable of inhibiting AGE apart from their AGE breaker and free radical scavenging activities.

**Method -II**

**(a) Principle:**

Proteins, in presence of reducing sugars such as glucose or ribose, form crosslinks that result in the formation of multimeric forms of protein such as dimer, trimer, etc.These can be separated on the basis of their molecular weights by a widely employed method termed as sodium dodecyl sulphate-polyacrylamide gel electrophoresis (SDS-PAGE). In short, the protein samples are treated with SDS so as denature them and also confer a net negative charge. The SDS treated protein samples are then run on a polyacrylamide gel in presence of electric current. The protein moieties run through the gel and their migration is directly dependent on their molecular weight. This method is widely employed for analysis of protein and is described by Sambrook,J and Russell,W (1); and is also employed in US Patent 5,853,703 to determine AGE-breaking activity of compounds.

**(b) Procedure:**

Lysozyme (Sigma-Aldrich, USA), ribose and test compound were dissolved in PBS so as to achieve final concentrations of 10mg/ml and 0.5M respectively. 10□g/ml phenylmethylsulfonyl fluoride (PMSF) (Boehringer Mannheim, Germany) and 0.02% sodium azide was added to the above solution so as to prevent protease action and microbial growth respectively. A separate tube containing BSA, ribose and sodium azide in the same concentration and buffer as above, but without any test compound, was also incubated as positive control. The solution was incubated at 37°C for 21 days.

After incubation, equal amount of protein from each reaction i.e. the test and control tubes was removed and loaded on SDS-PAGE. The gel was stained with Coommassie blue and densitometric analysis was carried out using Gel Doc 2000 (Bio Rad, USA). The results of SDS-PAGE is shown in Fig 1 in which Lanes 1 to 6 as marked represent the following:

Lane 1: Molecular weight marker
Lane 2: Control Lysozyme
Lane 3: Lysozyme + ribose
Lane 4: Lysozyme + ribose + Compound No.5 (25mM)
Lane 5: Lysozyme + ribose
Lane 6: Lysozyme + ribose + Compound No. 33 (5mM)

Lysozyme upon incubation with ribose at 37°C for 21 days (AGE-lysosyme) shows three prominent bands when subjected to SDS-PAGE; one that of the native lysozyme and two other bands of molecular weights corresponding to approximately the dimer and trimer of native lysozyme; labelled as peak1 and peak2 respectively. Since the density of the native lysozyme band remains constant in the control and treated samples, it was used for normalization. The inhibitory action of test compounds on AGE formation was determined by analyzing the degree of formation of the dimer (peak1) and trimer (peak2) of lysozyme relative to that in the control, plotted in terms of optical density of each band (Fig 2).

**Discussion of the test results:**

(i) **For Cosmetic Application**

The compounds of present invention have thus demonstrated capability of breaking AGE cross links formed in proteins. The compounds also demonstrated the capability of quenching free radicals, which can cause irreversible damage to proteins nucleic acids, etc. The ability to reverse the formation of Advanced Glycation End products (in skin support protein, like collagen and hair proteins like keratin) in conjunction with free radical quenching, carries with it significant implications and make them useful in cosmetic applications.

The compounds of present invention improves the aesthetic appearance of skin by arresting the complications of skin at more than one crucial stages. It breaks the preformed Advanced Glycation End products (AGE) formed in skin's support proteins and delays intrinsic aging (C.Jeanmaire et.al., British Journal of Dermatology 2001:145:10-18). The compounds of present invention also quenches the free radicals generated by UV exposure, pollutants etc, in the skin thereby prevents extrinsic or photoaging. The free radical quenching will also prevent the irreversible damage caused to proteins and nucleic acid. Moreover, by virtue of free radical quenching, these compounds will reduce the load of free radicals generated by Performed AGE's. The reduction in oxidative stress will in turn reduce the formation of reactive intermediates involved in Amadori Product formation.

The glycation of proteins is a universal phenomenon, well known at the skin level. However, this phenomenon can also occur in other related parts such as the nails or the hair, particularly in the Keratin (EP1068864 A1 and EP 1110539A1).

The glycation of the dermal proteins, particularly the collagen, leads to adverse cosmetic effects for e.g. consequences that damage the skin, the same consequences can be expected as a result of glycation of proteins in skin related parts, such as the nails and /or the hair, and in all the protein system.

(ii) **For Non-Cosmetic Application**

The test compounds listed in the table above exhibit invitro free radical scavenging (antioxidant) activity. Execessive production of free radicals reactive oxidative species (ROS) results in oxidative stress. Therefore, these molecules would be very effective in reducing oxidative stress by their ability to trap ROS. Antioxidants (free radicals scavengers) are reported to be effective in the management of various diseases linked with oxidative stress.

The following examples give method of preparation of the specific compounds useful for the composition of the invention as given in Table 1. The following compounds suggested are by way of example alone and in no way restrict the invention.

## Example 2

**Preparation of N,N'-bis [3-carbonyl-1- (2-phenyl-2-oxoethyl) pyridinium] hydrazine dibromide (compound 1):**

To a boiling solution of N, N-bis-(nicotinyl)hydrazine (1.21 g., 0.005 mol.) in methanol (20 ml.), a solution of phenacyl bromide (1.99 g., 0.01 mol.) in isopropanol (10 ml.) was added and the reaction mixture was refluxed for 6 hrs. The reaction mixture was concentrated under vacuum (~10 ml.) and filtered. The obtained residue was washed with hot ethylacetate and then the isolated solid was powdered. It was recrystallised from a mixture of methanol and ethylacetate (3:1, 20 ml) to afford a pale yellow solid.

| | |
|---|---|
| Yield | 60% |
| m.p. | 260 - 262°C (decomp.) |
| IR(KBr, $cm^{-1}$) | 1696 and 1680 |
| $^1H$ NMR ($DMSOd_6$, 400MHz) δ | 11.65(2H,s), 9.56(2H,s), 9.21-9.16(4H,m), 8.49-8.45 (2H,m), 8.08-8.05 (4H,d), 7.81-7.77(2H,m), 7.68-7.64 (4H,m), 6.58 (4H,s) |
| Mass (m/z) | 479, 480 |

According to the above mentioned procedure the following compounds are synthesized by reacting the corresponding pyridine derivatives with appropriate reagents by refluxing in methanol, ethanol, propanol, toluene or xylene for 6 - 48 hrs. to get the desired compounds:

## Example 3

**N,N'-Bis[3-carbonyl-1- (2- ethoxy -2-oxoethyl) pyridinium] hydrazine dibromide (compound 2):**

| | |
|---|---|
| Yield | 47% |
| m.p. | 180 - 182°C (decomp.) |
| IR(KBr, $cm^{-1}$) | 1744, 1664 |
| $^1H$ NMR ($DMSOd_6$, 400MHz) δ | 11.65 (2H,s), 9.62 (2H,s), 9.28-9.26 (2H,d), 9.17-9.15 (2H,d), 8.47-8.44 (2H,m), 5.77 (4H,s), 4.26 (4H,q), 1.27 (6H,t) |
| Mass (m/z) | 415, 416 |

## Example 4

**N,N'-Bis[3-carbonyl-1- (2- (2,4- dichlorophenyl) -2- oxoethyl) pyridinium] hydrazine dibromide (compound 3):**

| | |
|---|---|
| Yield | 24% |
| m.p. | 225 - 227°C (decomp.) |
| IR(KBr, $cm^{-1}$) | 1702, 1666 |
| $^1H$ NMR ($DMSOd_6$, 400 MHz) δ | 11.69 (2H,s), 9.58 (2H,bs), 9.20-9.18 (4H,m), 8.49-8.47 (2H,m), 8.17-8.15 (2H, d), 7.92 (2H,bs), 7.78-7.76 (2H,d), 6.50 (4H,s) |
| Mass (m/z) | 615, 617, 618, 620. |

**Example 5**

**1- (2- Ethoxy -2- oxoethyl) -3- (2- (2- pyridyl) hydrazinocarbonyl) pyridinium bromide (compound 4):**

| | |
|---|---|
| Yield | 16% |
| m.p. | 210-212°C |
| IR (KBr, $cm^{-1}$) | 3140, 3005, 1732 and 1690 |
| $^1$H NMR (DMSO$d_6$, 400MHz) δ | 9.63 (1H,s), 9.27 (2H,d), 8.49-8.45 (1H,m) 8.13-8.07 (2H,m), 7.32-7.30 (1H,m), 7.12-7.11(1H,m), 5.77 (2H,s), 4.23 (2H,q), 1.25 (3H,t) |
| Mass (m/z) | 301, 302 |

**Example 6**

**1- (2- Thien -2'- yl -2- oxoethyl) -3- (methanesulfonyl hydrazinocarbonyl) pyridinium bromide (compound 5):**

| | |
|---|---|
| Yield | 30 % |
| m.p | 199 - 200 °C |
| IR (KBr, $cm^{-1}$ ) | 1714, 1673 |
| $^1$HNMR (DMSO$d_6$, 400 MHz) δ | 11.38 (1H,s), 9.97 (1H,s) 9.51 (1H,s), 9.16 (1H,d),9.06 - 9.04 (1H,m), 8.43 - 8.39 (1H,m), 8.25 - 8.21 (2H,m), 7.43 - 7.41 (1H,t), 6.45 (2H,s), 3.08 (3H,s). |
| Mass (m/z) | 340, 341, 342 |

**Example 7**

**N,N'-Bis[3-carbonyl-1- (2- thien -2'- yl -2- oxoethyl) pyridinium] hydrazine dibromide (compound 6):**

| | |
|---|---|
| Yield | 33% |
| m.p. | 259 - 261°C (decomp.) |
| IR(KBr, $cm^{-1}$) | 3330, 1702, 1674, 1655 and 1626 |
| $^1$H NMR (DMSO$d_6$, 400 MHz) δ | 11.59 (2H,s), 9.50 (2H,s), 9.15-9.08 (4H,m), 8.40-8.36 (2H,m), 8.17-8.14 (4H, m), 7.33(2H,t), 6.42 (4H,s) |
| Mass (m/z) | 491, 492. |

**Example 8**

**1- (2- Ethoxy -2- oxoethyl) -3- (2- (benzoyloxy) ethylaminocarbonyl) pyridinium bromide (compound 7):**

| | |
|---|---|
| Yield | 85% |
| m.p. | 132-134°C |
| IR (KBr, $cm^{-1}$) | 3210, 3067, 1726, 1687, 1656 |
| $^1$H NMR (DMSO$d_6$, 400 MHz) 6 | 9.46 (1H,s), 9.37 (1H,t), 9.11(1H,t), 8.97 (1H,d), 8.33-8.29 (1H,m) 7.95-7.93 (2H, m), 7.63-7.59 (1H,m), 7.49-7.45 (2H,m), 5.65 (2H,s),4.39 (2H,t), 4.19 (2H,q), 3.70-3.69 (2H,m), 1.20 (3H,t) |
| Mass (m/z) | 357, 358, 359 |

**Example 9**

**1- (2- (2',4' - Dichlorophenyl) -2- oxoethyl) -3- (2-( benzoyloxy)ethyl aminocarbonyl) pyridinium bromide (compound 8):**

| | |
|---|---|
| Yield | 75% |
| m.p. | 102-104°C |
| IR(KBr, $cm^{-1}$) | 1703, 1685, 1675 |
| $^1H$ NMR (DMSO$d_6$, 400 MHz) $\delta$ | 9.41-9.37 (2H,m), 9.03-8.98 (2H,m)8.34-8.30 (1H,m), 8.04 (1H,d), 7.91-7.89 (2H,m), 7.82 (1H,d),7.68-7.65 (1H,m), 7.58-7.55 (1H,m), 7.43 (2H,t), 6.35 (2H, s), 4.36 (2H,t), 3.68-3.64 (2H,m) |
| Mass (m/z) | 457, 458, 459, 460, 461, 462 |

**Example 10**

**1- (2- Thien 2'- yl -2- oxoethyl) -3- (2- (2- pyridyl) hydrazinocarbonyl) pyridinium bromide (compound 9):**

| | |
|---|---|
| Yield | 10% |
| m.p. | 212-214°C (decomp) |
| IR(KBr, $cm^{-1}$) | 1685, 1649 |
| $^1H$ NMR (DMSO$d_6$, 400 MHz) $\delta$ | 11.21 (1H,bs), 9.59 (1H,s), 9.19 (2H,d), 8.44 (1H,t),8.27-8.24 (2H,m), 8.08 (1H, bs), 7.62 (1H,bs), 7.44 (1H,t), 6.85-6.79 (2H,m), 6.50 (2H,s) |
| Mass (m/z) | 339, 340, 341 |

**Example 11**

**1- (2- Phenyl -2- oxoethyl) -3- (2- (2- pyridyl) hydrazinocarbonyl) pyridinium bromide (compound 10):**

| | |
|---|---|
| Yield | 4% |
| m.p. | 190°C (decomp) |
| IR(KBr, $cm^{-1}$) | 1683, 1670, 1648 |
| $^1H$ NMR (DMSO$d_6$, 400 MHz) $\delta$ | 11.14 (1H,bs), 9.53 (1H,s), 9.18-9.13 (2H,m), 8.45- 8.42 (1H,t), 8.08-8.06 (3H, m), 7.80 (1H,t), 7.67 (2H,t), 7.62-7.55 (1H,m), 6.83-6.76 (2H,m), 6.54 (2H,s) |
| Mass (m/z) : | 333, 334, 335 |

**Example 12**

**1-(2-Phenyl-2-oxoethyl) -3- (hydrazinocarbonyl) pyridinium bromide (compound 11).**

| | |
|---|---|
| Yield | 15% |
| m.p. | 215 - 216 °C |
| IR(KBr, $cm^{-1}$) | 1695, 1680 |
| $^1$HNMR (DMSO$d_6$, 400 MHz) $\delta$ | 10.25 (1H,s) 9.65 (1H,s), 9.35 - 9.32 (2H,m), 8.90 - 8.88 (1H,m) 8.50 - 8.46 (2H, d), 8.21 - 8.17 (1H,m), 8.05 - 8.07 (2H,m), 6.50 (2H,s), 4.45 (2H,s). |
| Mass (m/z) | 256, 257. |

**Example 13**

**1- (2- Phenyl -2- oxoethyl) -3- (methanesulfonyl hydrazinocarbonyl) pyridinium bromide (compound 12):**

| | |
|---|---|
| Yield | 35% |
| m.p. | 227 - 228 °C |
| IR(KBr, $cm^{-1}$) | 1710, 1702 |
| $^1$HNMR (DMSO$d_6$, 400 MHz) δ | 11.30, (1H,s), 9.88 (1H,s), 9.41 (1H,s), 9.06- 9.05 (1H,d) 8.98 - 8.96 (1H,d), 8.34 - 8.31 (1H,m), 7.97 (2H,d), 7.72 - 7.69 (1H,t), 7.59 - 7.56 (2H,t), 6.44 (2H,s), 2.99 (3H,s) |
| Mass (m/z) | 334, 335 |

**Example 14**

**1-(2- Ethoxy -2- oxoethyl) -3- (methanesulfonyl hydrazinocarbonyl) pyridinium bromide (compound 13):**

| | |
|---|---|
| Yield | 38% |
| m.p | 75- 76 °C |
| IR(KBr, $cm^{-1}$) | 1739, 1697 |
| $^1$HNMR (DMSO$d_6$, 400 MHz) δ | 11.39 (1H,s), 9.96 (1H,s), 9.56 (1H,s), 9.23 (1H,d), 9.06 (1H,d), 8.40 (1H,t), 5.75 (2H,s), 4.27 - 4.22 (2H,q), 3.08 (3H,s), 1.26 (3H,t) |
| Mass (m/z) | 301, 302, 303 |

**Example 15**

**1-(2-Phenyl-2-oxoethyl)-3-(phenylsulfonylhydrazino carbonyl) pyridinium bromide (compound 14):**

| | |
|---|---|
| Yield | 28% |
| m.p | 187-188°C(dec.) |
| IR(KBr, $cm^{-1}$) | 1700, 1633 |
| $^1$HNMR (DMSO$d_6$, 400 MHz) δ | 11.38 (1H,s), 10.45 (1H,s), 9.33(1 H,s), 9.13 - 9.12 (1H,d), 8.95 (1H, d), 8.38 (1H,t), 8.05 (2H,d), 7.89 (2H,d), 7.80 (1H,t), 7.66 (3H,t), 7.57 (2H,t), 6.50 (2H,s). |
| Mass (m/z) | 396, 397, 398 |

**Example 16**

**1- (2-Phenyl-2-oxoethyl)-2-chloro-3-(phenylsulfonylhydrazino carbonyl) pyridinium bromide (compound 15):**

| | |
|---|---|
| Yield | 23% |
| m.p. | 247 - 250°C (decomp) |
| IR(KBr, $cm^{-1}$) | 1685 , 1679, |
| $^1$HNMR (DMSO$d_6$, 400 MHz) δ | 11.12 (1H,s), 9.49 (1H,s), 9.07 - 9.03(1H,m), 8.44 (1H, t), 8.07 (2H,d), 7.80 (1H, t), 7.67 (2H,t), 7.18 (2H,t), 6.87 (2H,d), 6.77 (1H,t), 6.50 (2H,s). |
| Mass (m/z) | 430, 431, 432 |

**Example 17**

**1-(2- Phenyl -2- oxoethyl) -3- (2- (acetoxy) ethyloxy carbonyl) pyridinium bromide (compound 16):**

| | |
|---|---|
| Yield | 40% |
| m.p. | 152-153°C |
| IR(KBr, $cm^{-1}$) | 1737, 1691, 1635 |
| $^1$HNMR (DMSO$d_6$, 400 MHz) δ | 9.63(1H,s), 9.24(1H,d), 9.12(1H,d), 8.43(1H,t), 8.07(2H,d), 7.80(1H,t), 7.67(2H, t), 6.59(2H,s), 4.62-4.60 (2H,m), 4.39 - 4.37(2H,m), 2.03 (3H,s) |
| Mass (m/z) | 328, 329 |

**Example 18**

**1- (2- Ethoxy -2- oxoethyl) -3- (2- (benzoyloxy) ethyloxycarbonyl) pyridinium bromide (compound 17):**

| | |
|---|---|
| Yield | 35% |
| m.p. | 142-143°C |
| IR(KBr, $cm^{-1}$) | 1736, 1718, 1636 |
| $^1$HNMR (DMSO$d_6$, 400 MHz) δ | 9.60(1H,s), 9.20-9.18(1H,d), 9.04-9.02(1H,d), 8.33- 8.29(1H,m), 7.90-7.88(2H, d), 7.58-7.57(1H,m), 7.46-7.42(2H,m), 5.67(2H,s), 4.71- 4.68(2H,m), 4.58-4.56 (2H,m), 4.15(2H,q), 1.16(3H,t) |
| Mass (m/z) | 358, 359, 360 |

**Example 19**

**1- (2- Thien -2'- yl -2- oxoethyl)-4-(2-(benzoyloxy) ethylaminocarbonyl) pyridinium bromide (compound 18):**

| | |
|---|---|
| m.p. | 210 - 211°C |
| IR(KBr, $cm^{-1}$) | 1723, 1680, 1668 |
| $^1$HNMR (DMSO$d_6$ 400 MHz) δ, | 9.52 (1H,t), 9.14 (2H,d), 8.50 (2H,d), 8.25 - 8.21 (2H,m), 8.01 - 7.99 (2H,d), 7.67 (1H,t), 7.55 - 7.51 (2H,m), 7.42- 7.40 (1H,m), 6.42 (1H,s) 4.47 - 4.45 (2H,t), 3.77 - 3.73 (2H, m). |
| Mass (m/z) | 395, 396 |

**Example 20**

**1-(2-Ethoxy-2-oxoethyl)-4-(phenylsulfonyl hydrazino carbonyl) pyridinium bromide (Compound 19):**

| | |
|---|---|
| Yield | 60% |
| m.p. | 171 - 173°C. |
| IR (KBr, $cm^{-1}$) | 1745, 1685, 1645. |
| $^1$HNMR (DMSO$d_6$, 400 MHz) δ | 11.41 (1H, s), 10.39 (1H, s), 9.10 (2H, d), 8.27 (2H, d), 7.82 - 7.80 (2H, d), 7.60 - 7.57 (1H, t), 7.50 - 7.46 (2H, t), 5.63 (2H, s), 4.18 - 4.12 (2H, q), 1.19 - 1.15 (3H, t). |
| Mass (m/z) | 364, 365, 366 |

**Example 21**

**1-(2-Phenylamino-2-oxoethyl)-4-(phenylsulfonyl hydrazino carbonyl) pyridinium chloride (Compound 20):**

| | |
|---|---|
| Yield | 10% |
| m.p. | 225 - 227°C. |
| IR (KBr, $cm^{-1}$) | 1693, 1642, 1592 |
| $^1$HNMR(DMSO$d_6$, 400 MHz) δ | 11.55 (1H, s), 10.99 (1H, s), 10.49 (1H, s), 9.20 (2H, d), 8.34 (2H, d), 7.89 (2H, d), 7.73 - 7.64 (1H, t), 7.61 - 7.56 (4H, m), 7.37 - 7.33 (2H, t), 7.12 - 7.09 (1H, t), 5.73 (2H, s). |
| Mass (m/z) | 411, 412, 413, 414 |

**Example 22**

**1-(2-Ethoxy-2-oxoethyl)-3-(phenylsulfonylhydrazino carbonyl) pyridinium bromide (Compound 21):**

| | |
|---|---|
| Yield | 75% |
| m.p. | 145 - 147 °C. |
| IR(KBr $cm^{-1}$) | 1744, 1713, 1633 |
| $^1$HNMR (DMSO$d_6$, 400MHz) δ | 11.27(1H,s),10.36 (1H, s), 9.28(1H,s), 9.09(1H,d), 8.83(1H,d),8.27 - 8.24 (1H,m), 7.82 - 7.79 (2H,m), 7.58 (1H,t), 7.48 (2H, t), 5.59 (2H,s), 4.17 - 4.12 (2H, q), 1.16 (3H,t). |
| Mass (m/z) | 364, 365, 366 |

**Example 23**

**1-(2-(2',4'-Dichlorophenyl)-2-oxoethyl)-3- (2(methoxy)ethyloxycarbonyl) pyridinium bromide (Compound 22):**

| | |
|---|---|
| Yield | 25% |
| m.p. | 156 - 158°C. |
| IR (KBr, $cm^{-1}$) | 1731, 1706, 1640 |
| $^1$HNMR (DMSO $d_6$,400 MHz)δ | 9.61 (1H, s),9.20(1H, d),9.13 (1H, d), 8.45 - 8.41 (1H, m),8.15 (1H, d),7.92(1H, d),7.78 - 7.76 (1H, m), 6.49 (2H, s), 4.56-4.54 (2H, m), 3.72 - 3.69 (2H, q), 3.31 (3H, s). |
| Mass (m/z) | 368, 369, 370, 371 |

**Example 24**

**1-(2-Phenylamino-2-oxoethyl)-3-(2-(benzoyloxyl) ethylaminocarbonyl) pyridinium chloride (Compound 23):**

| | |
|---|---|
| Yield | 70% |
| m.p. | 171 - 172°C |
| IR (KBr, $cm^{-1}$) | 1720, 1692, 1668 |
| $^1$HNMR :(DMSO$d_6$, 400 MHz) δ | 11.06 (1H, s), 9.67 (1H, t), 9.59 (1H, s), 9.20 (1H,d), 9.11 (1H, d), 8.36 - 8.32 (1H, m), 8.00 (2H, d), 7.66 - 7.61 (3H, m),7.51 (2H, t),7.34 (2H, t), 7.10 (1H, t), 5.77 (2H,s), 4.45 (2H,t), 3.76 - 3.72 (2H, q). |
| Mass (m/z) | 404, 405, 406, 407 |

**Example 25**

**1-(2-Thien-2'-yl-2-oxoethyl)-3-(phenylaminocarbonyl hydrazinocarbonyl) pyridinium bromide (Compound 24):**

| | |
|---|---|
| Yield | 0% |
| m.p. | 202 - 204°C. |
| IR (KBr, $cm^{-1}$) | 1718, 1673 |
| $^1$HNMR : (DMSO$d_6$, 400 MHz) δ | 11.03 (1H, s), 9.55 (1H, s), 9.18 (1H, d), 9.10 (1H, d), 9.00 (1H, s),8.57 (1H,s), 8.46 - 8.42 (1H, t), 8.25 - 8.22 (2H, m), 7.47 - 7.45 (2H, d), 7.43 - 7.41 (1H, t), 7.29 - 7.25 (2H, t), 7.0 - 6.96 (1H, t), 6.46 (2H, s). |
| Mass (m/z) | 381, 382, 383 |

**Example 26**

**1-(2-Phenyl-2-oxoethyl)-3-(2-(acetoxy) ethylaminocarbonyl) pyridinium bromide (Compound 25):**

| | |
|---|---|
| Yield | 55% |
| m.p. | 186-188 °C |
| IR (KBr, $cm^{-1}$) | 1734, 1697, 1679 |
| HNMR (DMSO$d_6$, 400 MHz) δ | 9.47(1H,s), 9.36 (1H,t), 9.13 - 9.05 (2H, m), 8.42 - 8.38 (1H, m), 8.06 (2H, d), 7.80 (1H, t), 7.67 (2H, t), 6.54 (2H, s), 4.18 (2H,t), 3.61 - 3.57 (2H,q), 2.02 (3H,s). |
| Mass (m/z) | 327, 328, 329. |

**Example 27**

**1-(2-Phenylamino-2-oxoethyl)-3-(phenyl sulfonyl hydrazino carbonyl) pyridinium chloride (Compound 26):**

| | |
|---|---|
| Yield | 38% |
| m.p. | 232 - 234°C. |
| IR(KBr, $cm^{-1}$) | 1689, 1636, 1596 |
| $^1$HNMR (DMSO$d_6$, 400 MHz) δ | 11.30 (1H, s), 10.80 (1H, s), 10.37 (1H, s), 9.29 (1H, s), 9.09 (1H, d), 8.81 (1H, d), 8.25- 8.21 (1H, t), 7.82 - 7.80 (2H, d), 7.59 - 7.46 (5H, m), 7.28 - 7.24 (2H, t), 7.04 - 7.00 (1H, t), 5.62 (2H,s). |
| Mass (m/z) | 411, 412, 413, 414 |

**Example 28**

**1-(2-Phenylamino-2-oxoethyl)-3-((4-methylphenyl)sulfonyl hydrazino carbonyl) pyridinium chloride (Compound 27):**

| | |
|---|---|
| Yield | 48% |
| m.p. | 205 - 206°C |
| IR(KBr, $cm^{-1}$) | 1712, 1681, 1632 |
| $^1$HNMR (DMSO$d_6$, 400 MHz) δ | 11.35 (1H, s), 10.86 (1H, s), 10.36 (1H, s), 9.38 (1H, s), 9.17 (1H, d), 8.90 (1H, d), 8.34 - 8.30 (1H, m), 7.78 (2H,d), 7.59 (2H, d), 7.37- 7.33 (4H,m), 7.11 (1H, t), 5.70 (2H,s), 2.36 (3H, s). |
| Mass (m/z) | 425, 426, 427, 428 |

**Example 29**

**1-(2-Phenyl-2-oxoethyl)-3-(2-(benzoyloxy)ethyloxy carbonyl) pyridinium bromide (Compound 28):**

| | |
|---|---|
| Yield | 35% |
| m.p. | 132 - 134°C. |
| IR (KBr, $cm^{-1}$) | 1730, 1705, 1690 |
| $^1$HNMR ($DMSOd_6$, 400 MHz) δ | 9.80 (1H, s), 9.36 (1H, d), 9.30 (1H, d), 8.58 (1H, t), 8.21 (2H, d), 8.12 (2H, d), 7.95 (1H, t), 7.85 - 7.80 (3 H, m), 7.68 (2H, t), 6.71 (2H, s), 4.95 - 4.93 (2H, m), 4.82 - 4.80 (2H, m). |
| Mass (m/z) | 390, 391, 392. |

**Example 30**

**1-(2-Thien-2'-yl-2-oxoethyl)-3-(phenylcarbonyl hydrazino carbonyl) pyridinium bromide (Compound 29):**

Yield : 45%
m.p. : 80 - 81°C
IR(KBr $Cm^{-1}$) : 1700, 1663, 1631
$^1$HNMR ($DMSOd_6$, 400MHz) δ : 11.49 (1H, s), 10.95 (1H, s), 9.67 (1H, s), 9.34 (1H, d), 9.27 (1H, d), 8.52 - 8.48 (1H, m), 8.29 - 8.28 (2H, m), 8.00 (2H, d), 7.68 (1H, t), 7.59 (2H, t), 7.46 (1H, t), 6.63 (2H,s)
Mass (m/z) : 366, 367, 368, 369

**Example 31**

**1-(2-Ethoxy-2-oxoethyl)-3-((phenylmethyl)sulfonyl hydrazino carbonyl) pyridinium bromide (Compound 30):**

Yield : 50%
m.p. : 147-148°C
IR (KBr, $cm^{-1}$) : 1749, 1698, 1640
$^1$HNMR ($DMSOd_6$, 400 MHz) δ : 11.57 (1H, s), 10.21 (1H,s), 9.75 (1H,s), 9.38 (1H, d), 9.24 (1H, d), 8.59 - 8.56 (1H, m), 7.67 - 7.65 (2H, m), 7.58 - 7.52 (3H, m), 5.90(2H, s),4.68 (2H, s), 4.45 - 4.39(2H, q),1.43 (3H, t).
Mass (m/z) : 377, 378, 379

**Example 32**

**1-(2-Phenyl-2-oxoethyl)-3-((phenylmethyl)sulfonyl hydrazino carbonyl)pyridinium bromide (Compound 31):**

Yield : 80%
m.p. : 205 - 207° C
IR (KBr, $Cm^{-1}$) : 1687, 1637
$^1$HNMR ($DMSOd_6$, 400 MHz) δ : 11.59 (1H,s), 10.20 (1H,s), 9.71 (1H,s), 9.33 (1H, d), 9.27 (1H, d), 8.62 - 8.59(1H, m), 8.25 - 8.23 (2H, d), 7.99 -7.95 (1H, t), 7.86 - 7.82 (2H, t), 7.67- 7.65 (2H, m), 7.57 - 7.52 (3H, m),6.72 (2H, s), 4.69 (2H, s).
Mass (m/z) : 410, 411, 412, 413

**Example 33**

**N, N' - Bis [3-carbonyl-1-(2-furan-2'-yl-2-oxoethyl) pyridinium] hydrazine dibromide. (Compound No: 32)**

Yield : 23%
m.p. : 267-269 °C (dec)
IR (KBr, $cm^{-1}$) : 1687, 1660
$^1$H NMR (DMSO $d_6$, 400 MHz) δ; 11.65 (2H,s), 9.56 (2H,s), 9.21 - 9.15 (4H,m), 8.48- 8.44 (2H,t), 8.23 (2H,s), 7.74 - 7.73 (2H,d), 6.91 - 6.90 (2H,d) 6.34 (4H,s)
Mass (m/z) :459, 460, 461

**Example 34**

**N,N'-Bis [3-carbonyl -1- (2-thien-2'-yl-2-oxoethyl) pyridinium] hydrazine dichloride.(Compound No: 33)**

Yield : 35%
m.p. : 275-277 °C
IR (KBr, $cm^{-1}$) : 3374, 1665,1632, 1410
$^1$H NMR (DMSO $d_6$, 400 MHz) δ: 11.88 (2H,s), 9.66 (2H,s), 9.29 - 9.24 (4H,m), 8.48 - 8.44 (2H,m), 8.25 - 8.23 (4H, m), 7.43 - 7.41 (2H,m), 6.53 (4H,s).
Mass (m/z) : 491, 492, 493, 494

**Example 35**

**1-(2-Thien-2'-yl-2-oxoethyl)-3-((2-(1-oxo-3-cyclohexyl)-propyl)-hydrazino carbonyl)-pyridinium bromide ( Compound No: 34);**

| | |
|---|---|
| Yield | 15% |
| m.p. | 217 - 219 °C ( dec) |
| IR (KBr, $cm^{-1}$) | 3190, 1708, 1667 and 1404 |
| $^1$H NMR (DMSO $d_6$, 400 MHz) δ | 11.07 (1H,s), 10.22 (1H,s), 9.51 (1H,s), 9.16 - 9.15 (1H,d), 9.06 - 9.04 (1H,d), 8.42 - 8.40 (1H,m), 8.25 - 8.21 (2H,m), 7.43 - 7.40 (1H,m), 6.44 (2H,s), 2.25 - 2.22 (2H,t), 1.72 - 1,.60 (5H,m), 1.49 - 1.43 (2H,q), 1.24 - 1.10 (4H,m), 0.9 - 0.85 (2H,m) |
| Mass (m/z) | 400,401,402 and 403 |

**Example 36**

**1-(2-Phenylamino-2-oxo ethyl)-3-({2-(1-oxo-3-cyclohexyl)-propyl} -hydrazino-carbonyl}-pyridinium bromide. (Compound No: 35);**

| | |
|---|---|
| Yield | 25% |
| m.p | 234-236 °C (dec) |
| IR (KBr, $cm^{-1}$) | 1689, 1652 and 1625 |
| $^1$H NMR (DMSO $d_6$, 400 MHz) δ | 11.11 (1H,s), 10.95 (1H,s), 10.23 (1H,s), 9.56 (1H,s), 9.23 - 9.21 (1H,d), 9.06 - 9.04 (1H,d), 8.38-8.35 (1H,m), 7.62 - 7.60 (2H,d), 7.37 - 7.33 (2H,t), 7.12 - 7.09 (1H,t), 5.75 (2H,s), 2.25 - 2.22 (2H,t), 1.72 - 1.60 (5H,m) 1.49 - 1.43 (2H, m), 1.25 - 1.10 (4H,m), 0.91 - 0.83 (2H,m) |

(continued)

| | |
|---|---|
| Mass (m/z) | 409, 410, 411 and 412 |

**Example 37**

**1-(2-Thien-2'-yl-2-oxoethyl)-3-[2-(benzoyloxy)ethylamino carbonyl]-pyridinium bromide (Compound No:36);**

| | |
|---|---|
| Yield | 40% |
| m.p. | 125-127°C |
| IR (KBr, $cm^{-1}$) | 1710 and 1675 |
| $^1H$ NMR (DMSO $d_6$, 400 MHz) δ | 9.48 (1H,s), 9.43 - 9.41 (1H,t), 9.12 - 9.11 (1H,d), 9.05 - 9.02 (1H,d), 8.40 - 8.36 (1H,m), 8.25 - 8.20 (2H,m), 8.00 - 7.98 (2H,m), 7.68 - 7.64 (1H,m), 7.54-7.50 (2H,m), 7.42 - 7.40 (1H,m), 6.43 (2H,s), 4.46-4.43 (2H,t), 3.77- 3.73 (2H,q) |
| Mass (m/z) | 395, 396, 397 and 398 |

**Example 38**

**1-(4-Ethoxy-2,4-dioxobutyl)-3-(2-(benzoxyloxy)ethylamino carbonyl)-pyridinium chloride. (Compound No: 37);**

| | |
|---|---|
| Yield | 35% |
| m.p. | 147-149°C |
| IR (KBr, $cm^{-1}$) | 1743, 1720, 1680 and 1627 |
| $^1H$ NMR (DMSO $d_6$, 400 MHz) δ | 9.62 - 9.59 (1H,t), 9.32 - 9.29 (1H,s), 9.05 - 9.03 (1H,d), 8.93 - 8.90 (1H,d), 8.27 - 8.24 (1H,m), 7.92 - 7.89 (2H,d), 7.59 - 7.55 (1H,m), 7.45 - 7.41 (2H,m), 5.82 (2H,s), 4.37-4.34 (2H,t), 4.08-4.03 (2H,q), 3.80 (2H,s), 3.67- 3.63 (2H,q), 1.15-1.11 (3H,t), |
| Mass (m/z) | 399, 400 and 401 |

**Example 39**

**1-(2',4'-Dichlorophenyl-2-oxoethyl)-3-(2-methoxyethyl aminocarbonyl)-pyridinium bromide. (Compound No: 38);**

| | |
|---|---|
| Yield | 70% |
| m.p. | 93-95 °C |
| IR (KBr, $cm^{-1}$) | 1704, 1664 and 1636 |
| $^1H$ NMR (DMSO $d_6$, 400 MHz) δ | 9.48 (1H,s), 9.29 (1H,bs), 9.11 - 9.08 (2H,m), 8.41 - 8.38 (1H,m), 8.15 - 8.13 (1H,d), 7.92 - 7.91 (1H,t), 7.78 - 7.75 (1H,m), 6.44 (2H,s) 3.52 (2H,bs), 3.51 (2H,bs), 3.28 (3H,s) |
| Mass (m/z) | 367,368,369 and 370 |

**Example 40**

**N,N'-Bis-[3-carbonyl-1-(2-cyclopropylamino-2-oxoethyl) pyridinium] hydrazine dichloride. (Compound No: 39);**

| | |
|---|---|
| Yield | 40% |

(continued)

| | |
|---|---|
| m.p. | 228-230 °C |
| IR (KBr $cm^{-1}$) : | 1675, 1636 and 1298 |
| $^1H$ NMR (DMSO $d_6$, 400 MHz) δ | 11.85 (2H,s), 9.59 (2H,s), 9.25 - 9.19 (4H,m), 9.00 - 8.99 (2H,d), 8.39 - 8.36 (2H,m), 5.53 (4H,s), 2.73 - 2.66 (2H,m), 0.78 - 0.62 (4H,m), 0.53 - 0.49 (4H,m) |
| Mass (m/z) | 437, 438 and 439 |

**Example 41**

**1-(2-Cyclopropylamino-2-oxoethyl)-3-(2-methoxyethylaminocarbonyl)-pyridinium chloride. (Compound No: 40);**

| | |
|---|---|
| Yield | 10% |
| m.p. | 122-124 °C |
| IR(KBr, $cm^{-1}$) | 1661, 1633, 1549 and1121 |
| $^1H$ NMR (DMSO $d_6$, 400 MHz) δ | 9.40 (1H,s), 9.08 - 9.02 (2H,m), 8.28 - 8.25 (1H,m), 5.53 (2H,s), 3.66 - 3.61 (4H,m), 3.39 (3H,s), 2.78 - 2.74 (1H,m), 0.80 - 0.75 (2H,m), 0.64 - 0.61 (2H,m) |
| Mass (m/z) | 278, 279 and 280 |

**Example 42**

**N-N'-Bis [3-carbonyl-1-(2-isopropylamino-2-oxoethyl) pyridinium] hydrazine dichloride. (Compound No: 41);**

| | |
|---|---|
| Yield | 35% |
| m.p. | 114-116 °C (dec) |
| IR (KBr, $cm^{-1}$) | 1707, 1668 and 1637 |
| $^1H$ NMR (DMSO $d_6$, 400 MHz) δ | 11.84 (2H,s), 9.59 (2H,s), 9.21 - 9.18 (4H,m), 8.74- 8.72 (2H,d), 8.39 - 8.35 (2H,m), 5.53 (4H,s), 3.92 - 3.84 (2H,m), 1.14 - 1.02 (12H,d) |
| Mass (m/z) | 441, 442 and 443 |

**Example 43**

**1-(2-Thien-2'yl-2-oxoethyl)-3-(2-(2-chloro-3-pyridoylhydrazinocarbonyl)-pyridinium chloride. (Compound No: 42);**

| | |
|---|---|
| Yield | 56% |
| m.p. | 233-235 °C |
| IR (KBr, $cm^{-1}$) | 1680, 1637, 1404 and 1293 |
| $^1H$ NMR (DMSO $d_6$, 400 MHz) δ | 11.62 (1H,s), 11.05 (1H,s), 9.62 (1H,s), 9.24 - 9.23 (1H,d), 9.18 - 9.16 (1H,d), 8.58 - 8.56 (1H,m), 8.46 - 8.43 (1H,m), 8.26 - 8.24 (2H,m), 8.02 - 8.00 (1H,m), 7.61-7.58 (1H,m), 7.43 - 7.41 (1H,m), 6.51 (2H,s) |
| Mass (m/z) | 401, 402, 403, 404 and 405 |

**Example 44**

**1-(2-Isopropylamino-2-oxoethyl)-3-(2-methylsulfonylhydrazinocarbonyl)-pyridinium chloride. (Compound No: 43);**

| | |
|---|---|
| Yield | 10% |
| m.p. | 227- 229 °C |
| IR (KBr, $cm^{-1}$) | 1691, 1670, 1566 and 1330 |
| $^1H$ NMR (DMSO $d_6$, 400 MHz) δ | 11.55 (1H,s), 9.94 (1H,s), 9.52 (1H,s), 9.16 - 9.14 (1H,m), 9.09 - 9.07 (1H,m), 8.72 - 8.70 (1H,m), 8.34 - 8.30 (1H,m), 5.50 (2H,s), 3.89 - 3.84 (1H,m), 3.11 (3H,s), 1.13 - 1.12 (6H,d) |
| Mass (m/z) | 315, 316 and 317 |

**Example 45**

**1-(2-(1-Pyrrolidinyl)-2-oxoethyl)-3-(methanesulfonyl hydrazino carbonyl) pyridinium chloride (Compound No: 44);**

| | |
|---|---|
| Yield | 21.00% |
| m.p. | 205-207°C |
| IR (KBr, cm-1) | 1699, 1646 and 1589 |
| $^1H$NMR :(DMSO $d_6$, 400 MHz) δ | 11.50 (1H, s), 9.94 (1H, s), 9.46 (1H, s), 9.11 - 9.06 (2H, m), 8.36 - 8.33 (1H, t), 5.75 (2H, s), 3.55 - 3.48 (3H, m), 3.10 (3H, s), 2.00 - 1.95 (2H, m), 1.87 - 1.81 (2H, m) |
| Mass (m/z) | 327, 328, 329 and 330 |

**Example 46**

**1-(2-Thien-2'-yl-2-oxoethyl)-3-(methanesulfonyl hydrazino carbonyl) pyridinium chloride (Compound No: 45);**

| | |
|---|---|
| Yield | 31.00% |
| m.p. | 215- 217°C |
| IR (KBr, $cm^{-1}$) :1685, | 1666 and 1635 |
| $^1H$NMR :(DMSO $d_6$, 400 $MH_z$) δ | 11.49, (1H, s), 9.96 (1H, s), 9.55 (1H, s), 9.18 (1H, d), 9.10 (1H, d), 8.43 - 8.39 (1H, t), 8.25 - 8.22 (2H, m), 7.42 (1H, t) 6.47 (2H, s), 3.09 (3H, s). |
| Mass (m/z) | 340, 341, 342 and 343 |

**Example 47**

**N,N'-Bis[3-carbonyl-1-(2-hydroxy-2-oxoethyl) pyridinium]hydrazine dichloride (Compound No: 46):**

| | |
|---|---|
| Yield | 43.00% |
| m.p. | 235 - 240°C (d) |
| IR (KBr, $cm^{-1}$) | 1743, 1700 and 1672 |
| $^1H$NMR (DMSO $d_6$, 400 MHz) δ | 11.89 (2H, s), 9.69 (2H, s), 9.31 - 9.29 (2 H, d), 9.25 - 9.23 (2H, d), 8.43 - 8.39 (2H, t) 5.70 (4H, s) |
| Mass (m/z) | 360,361,362 |

**Example 48**

**1-(2-Thien-2'-yl-2-oxoethyl)-3-((2-methoxy ethyl) amino carbonyl)-5-bromo pyridinium chloride (Compound No: 47);**

| | |
|---|---|
| Yield | 31.00% |
| m.p. | 180 - 182°C |
| IR (KBr, $cm^{-1}$) | 1661 and 1620 |
| $^1$HNMR (DMSO $d_6$, 400 $MH_z$) δ | 9.58 - 9.54 (2H, d), 9.43 - 9.39 (2H, d), 8.25 - 8.21 (2H, m), 7.41 (1H, t), 6.43 (2H, s), 3.51 (4H, m), 3.29 (3H, s). |
| Mass (m/z) | 384, 385, 386, 387 and 388 |

**Example 49**

**1-(2-Thien-2'-yl-2-oxoethyl)-3-[1-oxo-1-(2-methoxycarbonyl) pyridyl] hydrazino pyridinium chloride (Compound No: 48);**

| | |
|---|---|
| Yield | 30.00% |
| m.p. | 222 - 225°C |
| IR (KBr, $cm^{-1}$) | 1726, 1708 and 1662 |
| $^1$H NMR (DMSO $d_6$, 400 $MH_z$) δ | 11.47 (1H, s), 11.23 (1H, s), 9.58 (1H, s), 9.22 - 9.15 (3H, m), 8.56 - 8.53 (1H, d), 8.46 - 8.43 (1H, t) 8.25 - 8.21 (3H, m), 7.42 (1H, t), 6.49 (2H, s), 3.95 (3H, s) |
| Mass (m/z) | 425, 426 and 427 |

**Example 50**

**1-[1-(2-Thien-2'-yl-2-oxoethyl)-6methyl-3-carbonyl pyridinium]-2-[1-(2-Thien-2'-yl-2-oxoethyl )-3--carbonyl pyridinium] hydrazine dichloride(compound no: 49),**

| | |
|---|---|
| Yield | 40% |
| M.P. | 76-80 °C (dec) |
| IR ( KBr,$cm^{-1}$) | 1637,1513 |
| $^1$HNMR (DMSO $d_6$, 400 MHz) δ | 11.69(2H,s), 9.59-9.53(2H,d), 9.19(2H,m), 9.05(1H,d) , 8.46-8.43(1H,t) ,8.34 (1H,d), 8.27-8.23(4H,m), 7.45-7.41(2H,m) ,6.56(2H,s) ,6.48(2H,s),2.81(3H,s). |
| Mass(m/z) | 505, 506, 507. |

**Example 51**

**1-(2-Thien-2'-yl-2-oxoethyl)-3-(isopropylsulfonyl hydrazino carbonyl) pyridinium bromide(compound no: 50),**

| | |
|---|---|
| Yield | 70% |
| M.P | 90-95°C (dec) |
| IR ( KBr,$cm^{-1}$) | 1638,1589 |
| $^1$HNMR (DMSO $d_6$, 400 MHz) δ | 11.27(1H,s) ,9.91(1H,s), 9.60(1H,s) ,9.19- 9.15(2H,m), 8.42-8.36(1H,m) , 8.25-8.21(2H,m) ,7.43-7.41(1H,t) ,6.45(2H,s), 1.35- 1.34(6H,d). |
| Mass(m/z) | 368,369,370 |

**Example 52**

**1-(2-(4-Benzyl piperidin-1-yl)-2-oxoethyl)-3-(methanesulfonyl hydrazino carbonyl) pyridinium chloride (compound no: 51),**

| | |
|---|---|
| Yield | 17% |
| M.P | 76-78°C |
| IR ($KBr, cm^{-1}$) | 1684,1650,1556,1540. |
| $^1HNMR$ (DMSO $d_6$, 400 MHz) $\delta$ | 11.46(1H,s),9.55(1H,s), 9.46(1H,s),9.09- 9.03(2H,m), 8.36-8.32(1H,t), 7.33-7.29(2H,m), 7.23-7.19(3H,m), 5.88-5.79(2H,m) ,4.30- 4.27(1H,d) , 3.76-3.73(1H,d), 3.10(4H,m),2.64(1H,t),2.57-2.55(2H,d), 1.85(1H,bs) , 1.72-1.63(2H,t) ,1.36-1.28(1H,q) ,1.13-1.03(1H,m) |
| Mass(m/z) | 431,432,433 |

**Example 53**

**1-(2-(2-Ethoxy carbonyl pyrrolidin-1-yl)-2-oxoethyl)-3-(methanesulfonyl hydrazino carbonyl) pyridinium chloride. (compound no: 52),**

| | |
|---|---|
| Yield | 14% |
| M.P | 88-91°C |
| IR ($KBr, cm^{-1}$) | 1735,1665,1539 |
| $^1HNMR$ (DMSO $d_6$, 400 MHz) $\delta$ | 11.48(1H,s) ,9.96(1H,s),9.46(1H,s) ,9.09- 9.05(2H,m) ,8.38-8.34(1H,t), 5.94-5.80(2H,q) ,4.37-4.36(1H,d), 4.08-4.06(2H,d), 3.68- 3,65(2H,m), 3.09(4H, m) ,2.23-2.18(2H,m), 2.04 -1.93(3H,m) ,1.18-1.09(3H,t) |
| Mass(m/z) | 399,400,401 |

**Example 54**

**1-(2-Thien-2'-yl-2-oxoethyl)-3-(methanesulfonyl hydrazino carbonyl )-5-bromo pyridinium bromide. (compound no: 53),**

| | |
|---|---|
| Yield | 54% |
| M.P | Above 190-195°C(dec) |
| IR ( $KBr, cm^{-1}$) | 1682,1557,1540,1520 |
| $^1HNMR$ (DMSO $d_6$, 400 MHz) $\delta$ | 11.35(1H,s),10.01(1H,s) ,9.57- 9.54(2H,d), 9.32(1H,s), 8.26-8.22(2H,m),7.42 (1H,s),6.39(2H,s), 3.08(3H,s) |
| Mass (m/z) | 418,419,420 |

**Example 55**

**1-(2-Thien-2'-yl-2-oxoethyl)-3-(ethoxycarbonyl hydrazino carbonyl ) pyridinium bromide. (compound no: 54),**

| | |
|---|---|
| Yield | 69% |
| M.P | 155-157°C |
| IR($KBr, cm^{-1}$) | 1731,1665,1637 |

(continued)

| | |
|---|---|
| $^1$HNMR (DMSO $d_6$, 400 MHz) δ | 11.04(1H,s), 9.59(1H,s), 9.53(1H,s), 9.18(1H,s), 9.05-9.04(1H,d), 8.42(1H,s), 8.25-8.23(2H,m) , 7.43(1H,s), 6.46(2H,s), 4.12-4.11(2H,s), 1.23(3H,s) |
| Mass (m/z) | 334,335,336 |

**Example 56**

**1-(2-(5-chloro-thien-2-yl)-2-oxoethyl)-3-(methanesulfonyl hydrazino carbonyl) pyridinium bromide (compound no: 55),**

| | |
|---|---|
| Yield | 87% |
| M.P | 228-230°C |
| IR(KBr,$cm^{-1}$) | 1708,1664,1631,1550 |
| $^1$HNMR (DMSO $d_6$, 400 MHz) δ | 11.40(1H,s), 9.98(1H,s), 9.50(1H,s), 9.15(1H,d), 9.061H,d), 8.43-8.39(1H,t), 8.16-8.15(1H,d), 7.51-7.50(1H,d), 6.41(2H,s), 3.09 (3H,s) |
| Mass(m/z) | 374,375,376,377 |

**Example 57**

**N-N'-Bis[3-carbonyl-1-(2-(4-nitro-thien-2-yl)-2-oxoethyl)pyridinium] hydrazine dichloride. (compound no: 56),**

| | |
|---|---|
| Yield | 27% |
| M.P | 204-207°C |
| IR(KBr,$cm^{-1}$) | 1681,1539,1514 |
| $^1$HNMR (DMSO $d_6$, 400 MHz) δ | 11.90(2H,s), 9.63(2H,s) , 9.31-9.30(4H,m), 9.24 -9.22(2H,m), 8.87(2H,s), 8.49-8.46(2H,t), 6.56 (4H,s) |
| Mass (m/z) | 581,582,583 |

**Example 58**

**1-(2-Thien-2'-yl-2-oxoethyl)-3-(methanesulfonyl hydrazino carbonyl) -6-methyl pyridinium bromide. (compound no: 57),**

| | |
|---|---|
| Yield | 14% |
| M.P | 90-95°C(dec) |
| IR(KBr,$cm^{-1}$) | 1677,1575 |
| $^1$HNMR (DMSO $d_6$, 400 MHz) δ | 11.32(1H,s),9.97(1H,s) 9.52(1H,s), 8.94- 8.92(1H,d), 8.32-8.24(3H,m), 7.44 (1H,t), 6.54(2H,s), 3.08(3H,s), 2.79(3H,s) |
| Mass(m/z) | 354,355,356 |

## Example 59

**N-N'-Bis[3-carbonyl-1-(2-(5-methyl-thien-2-yl)-2-oxoethyl)pyridinium] hydrazine dichloride. (compound no: 58),**

| | |
|---|---|
| Yield | 37% |
| M.P | Above 166-168°C(dec) |
| IR(KBr,$cm^{-1}$) | 1666,1500 |
| $^1$HNMR (DMSO $d_6$, 400 MHz) δ | 11.73(2H,s), 9.59(2H,s), 9.19-9.15(4H,d) 8.45-8.42(2H,t), 8.06-8.05(2H,d) , 7.15-7.14(2H,d), 6.43 (4H,s), 2.59(6H,s) |
| Mass(m/z) | 519,520,521,522 |

## Example 60

**N-N'-Bis[3-carbonyl-1-(2-(2-ethoxycarbonyl pyrrolidin-1-yl)-2-oxoethyl) pyridinium] hydrazine dichloride. (compound no: 59),**

| | |
|---|---|
| Yield | 28% |
| M.P | 118-120°C |
| IR(KBr,$cm^{-1}$) | 1660,1510 |
| $^1$HNMR (DMSO $d_6$, 400 MHz) δ | 11.75(2H,s), 9.51(2H,s), 9.20-9.10(4H,m) 8.43-8.40(2H,t), 5.97-5.83(4H,m) , 4.39-4.36(2H,m), 4.27-4.22(1H,q), 4.12-4.05(4H,m), 3.71-3.63(4H,m), 3.48-3.40(1H,m), 2.26-2.19(2H,m), 2.05-1.91(5H,m), 1.30-1.27(1H,t), 1.19-1.15(5H,t) |
| Mass(m/z) | 609,610,611 |

## Example 61

**1-[1-(2-Thien-2'-yl-2-oxoethyl)-5-aminocarbonyl-3-carbonyl pyridinium]-2-[1-(2-Thien-2'-yl-2-oxoethyl )-3-carbonyl pyridinium] hydrazine dichloride (compound no: 60),**

| | |
|---|---|
| Yield | 54% |
| M.P | Above 127-129°C(dec) |
| IR(KBr,$cm^{-1}$) | 1678,1513 |
| $^1$HNMR (DMSO $d_6$, 400 MHz) δ | 11.86(2H,s), 9.83-9.64(4H,t), 9.24 - 9.23(2H,s), 8.82(1H,s), 8.48-8.45(1H,t), 8.34(1H,s) 8.26-8.24(4H,m), 7.44 -7.42(2H,d), 6.52-6.46(4H,d) |
| Mass (m/z) | 534,535,536 |

## Example 62

**1-(2-(4-carboethoxy-thiazolidin-3yl)-2-oxoethyl)-3-(methanesulfonyl hydrazino carbonyl) pyridinium chloride (compound no: 61),**

| | |
|---|---|
| Yield | 29% |
| M.P | 190-192°C |
| IR (KBr,$cm^{-1}$) | 1673,1541 |

(continued)

| | |
|---|---|
| $^1$HNMR (DMSO $d_6$, 400 MHz) δ | 11.50(1H,s), 9.55(1H,s), 9.48(1H,s), 9.12- 9.08(2H,m) , 8.39-8.34(1H,t), 6.04 - 5.99(2H,m), 4.94 -4.91(1H,m), 4.87-4.84(1H,d), 4.73-4.71(1H,d), 4.28-4.23(1H, q), 4.14 -4.09(1H,q), 3.43-3.38(1H,m),3.27-3.22(1H,m), 3.10(3H,s) ,1.30-1.27 (1H,t), 1.20-1.17(2H,m) |
| Mass(m/z) | 439,440,441 |

**Example 63**

**N-N'-Bis[3-carbonyl-1-(2-(5-chloro-thien-2-yl)-2-oxoethyl) pyridinium] hydrazine dichloride. (compound no: 62),**

| | |
|---|---|
| Yield | 35% |
| M.P | Above 200-205°C (dec) |
| IR ( KBr,cm$^{-1}$) | 1674,1590,1500 |
| $^1$HNMR (DMSO $d_6$, 400 MHz) δ | 11.90(2H,s), 9.64 -9.61(2H,d) , 9.29- 9.20(4H,m) , 8.47-8.44(2H,t) , 8.18-8.17 (2H,d),7.51-7.50(2H,d), 6.49-6.48(4H,s) |
| Mass(m/z) | 559,560,561,562,563,564 |

**Example 64**

**1-(2-(5-Methyl-thien-2-yl)-2-oxoethyl)-3-(methanesulfonyl hydrazino carbonyl ) pyridinium chloride (compound no: 63),**

| | |
|---|---|
| Yield | 22% |
| M.P | 196-198°C |
| IR(KBr,cm$^{-1}$) | 1689,1657 |
| $^1$HNMR (DMSO $d_6$, 400 MHz) δ | 11.47(1H,s) ,9.98(1H,s) ,9.53(1H,s) ,9.17- 9.16(1H,d), 9.09-9.07(1H,d), 8.42-8.38(1H,t), 8.06-8.05(1H,d), 7.15-7.14(1H,d), 6.41(2H,s), 3.09(3H,s), 2.59 (3H,s) |
| Mass(m/z) | 354,355,356,357 |

**Example 65**

**1-(2-(4-Nitro-thien-2-yl)-2-oxoethyl)-3-(methanesulfonyl hydrazino carbonyl ) pyridinium bromide. (compound no: 64),**

| | |
|---|---|
| Yield | 52% |
| M.P | Above 200-205°C(dec) |
| IR ( KBr,cm$^{-1}$) | 1688,1631,1541 |
| $^1$HNMR (DMSO $d_6$, 400 MHz) δ | 11.41(1H,s), 9.50(1H,s) 9.309-9.306(1H,d), 9.17-9.15(1H,d) ,9.09-9.07(1H,d), 8.866-8.862(1H,d) ,8.45-8.41(1H,t) , 6.50(2H,s), 3.09(3H,s) |
| Mass(m/z) | 385,386,387 |

**Example 66**

**1-(2-Phenylamino-2-oxoethyl)-3-(phenyl hydrazino carbonyl) pyridinium chloride (compound no: 65),**

| | |
|---|---|
| Yield | 45% |
| M.P | 165-167°C |
| IR ( KBr,$cm^{-1}$) | 1679,1626,1600,1497 |
| $^1$HNMR (DMSO $d_6$, 400 MHz) δ | 11.18(1H,s) , 11.10(1H,s), 9.62(1H,s), 9.24-9.22(1H,d), 9.17-9.15(1H,d), 8.40-8.36(1H,t), 8.19(1H,s), 7.63-7.61(2H,d) , 7.37- 7.33(2H,t) , 7.20-7.16(2H, t), 7.12-7.09(1H,t) , 6.88-6.86(2H,d), 6.78-6.74(1H,t) , 5.78(2H,s) |
| Mass(m/z) | 347,348,349 |

**Example 67**

**1-(2-Phenylamino-2-oxoethyl)-4 -[ 2-(benzoyloxy) ethylamino carbonyl ] pyridinium chloride (compound no: 66),**

| | |
|---|---|
| Yield | 40% |
| M.P | 178-180°C |
| IR (KBr,$cm^{-1}$) | 1700,1666,1559 |
| $^1$HNMR (DMSO $d_6$, 400 MHz) δ | 11.13(1H,s), 9.74-9.71(1H,t), 9.23- 9.22(2H,d) , 8.52-8.50(2H,d) , 8.01-7.99 (2H,d) , 7.68-7.60(3H,m) , 7.54-7.51(2H,t) , 7.36-7.32(2H,t) , 7.12-7.08 (1H,t) , 5.75 (2H,s) , 4.47-4.45(2H,t) , 3.77-3.72(2H,q). |
| Mass (m/z) | 404,405,406 |

**Example 68**

**1-2-(5-Nitro-thien-2-yl)-2-oxoethyl)-3-(methanesulfonyl hydrazino carbonyl ) pyridinium chloride (compound no: 67),**

| | |
|---|---|
| Yield | 10% |
| M.P | Above 105-110°C(dec) |
| IR ( KBr,$cm^{-1}$) | 1680,1620 |
| $^1$HNMR (DMSO $d_6$, 400 MHz) δ | 11.48(1H,s) , 9.98(1H,s), 9.52(1H,s), 9.16- 9.10(2H,m),8.45-8.41(1H,t) , 8.35-8.34(1H,d) ,8.25-8.24(1H,d), 6.50(2H,s), 3.09(3H,s). |
| Mass (m/z) | 385,386,387 |

**Example 69**

**1-(2-Thien-2'-yl-2-oxoethyl)3-(Trifluromethanesulfonyl hydrazino carbonyl)-pyridinium bromide (compound no: 68),**

| | |
|---|---|
| Yield | 22% |
| M.P | 77-79°C |
| IR ( KBr,$cm^{-1}$) | 2960, 1690, 1673, 1591 |
| $^1$HNMR (DMSO $d_6$, 400 MHz) δ | 11.76(1H,s), 11.27(1H,s), 9.61(1H,s),, 9.20-9.19(1H,d) ,9.07-9.05(1H,d), 8.44-8.41(1H,t) ,8.25-8.22(2H,m), 7.34 - 7.41 (1H,m), 6.46 (2H,s). |

(continued)

| | |
|---|---|
| Mass (m/z) | 394, 395, 396 |

**Example 70**

**1-(2-Thien-2'-yl-2-oxoethyl)-3-(phenyl hydrazino carbonyl) pyridinium bromide (compound no. 69),**

| | |
|---|---|
| Yield | 10% |
| M.P | 192-194°C |
| IR(KBr,$cm^{-1}$) | 1669,1663,1603, |
| $^1$HNMR (DMSO $d_6$, 400 MHz) $\delta$ | 10.99(1H,s), 9.54(1H,s), 9.17-9.14(2H,t), 8.44-8.41(1H,t), 8.25-8.22(3H,m), 7.43-7.41(1H,t), 7.20-7.16(2H,t), 6.87-6.85(2H,d), 6.79-6.75(1H,t), 6.46(2H,s) |
| Mass(m/z) | 338,339,340 |

**Example 71**

**1-(2-Thien-2'-yl-2-oxoethyl)-3-(p-methoxy phenyl sulfonyl hydrazino carbonyl) pyridinium bromide (compound no. 70),**

| | |
|---|---|
| Yield | 28% |
| M.P | 126-128°C |
| IR(KBr,$cm^{-1}$) | 1672,1653,1596 |
| $^1$HNMR (DMSO $d_6$, 400 MHz) $\delta$ | 11.34-11.33(1H,d), 10.27-10.26(1H,d), 9.34(1H,s), 9.13-9.12(1H,d), 8.94-8.92 (1H,d), 8.38-8.34(1H,t) ,8.24-8.19(2H,m),7.82- 7.75(2H,m) ,7.42-7.40(1H,t), 7.07-7.04(2H,d) ,6.40(2H,s), 3.81(3H,s). |
| Mass(m/z) | 432,433,434 |

**Example 72**

**1-(2-Ethoxy-2-oxoethyl)-3-(phenyl aminocarbonyl hydrazino carbonyl) pyridinium bromide. (compound no. 71),**

| | |
|---|---|
| Yield | 25% |
| M.P | 183-185°C |
| IR(KBr,$cm^{-1}$) | 1746,1717,1682 |
| $^1$HNMR (DMSO $d_6$, 400 MHz) $\delta$ | 11.02(1H,s), 9.57(1H,s), 9.22-9.21(1H,d), 9.11-9.09(1H,d), 9.00(1H,s), 8.57 (1H,s) ,8.44-8.41(1H,m),7.47-7.45(2H,d) ,7.29- 7.25(2H,t), 7.00-6.96(1H,t), 5.74(2H,s), 4.28-4.23(2H,q), 1.28-1.25(3H,t). |
| Mass(m/z) | 343,344,345,346 |

**Example 73**

**1-(2-Ethoxy-2-oxoethyl)-3-(p-toluene sulfonyl hydrazino carbonyl) pyridinium bromide. (compound no. 72),**

| | |
|---|---|
| Yield | 54% |
| M.P | 174-176°C |
| IR (KBr,$cm^{-1}$) | 1746,1712,1634 |

(continued)

| | |
|---|---|
| $^1$HNMR (DMSO $d_6$, 400 MHz) δ | 11.33(1H,s), 10.36(1H,s), 9.37(1H,s), 9.18-9.16(1H,d), 8.93-8.91(1H,d), 8.37-8.33(1H,t), 7.78-7.76(2H,d), 7.37-7.35(2H,d) , 5.68 (2H,s), 4.26-4.20(2H, q) ,2.37 (3H,s),1.27-1.23(3H,t). |
| Mass(m/z) | 378,379,380,381 |

**Example 74**

**1-(2-Phenyl-2-oxoethyl)-3-(phenylamino carbonyl hydrazino carbonyl) pyridinium bromide. (compound no. 73),**

| | |
|---|---|
| Yield | 70% |
| M.P | 206-208°C |
| IR ( KBr,cm$^{-1}$) | 1713,1684,1634 |
| $^1$HNMR (DMSO $d_6$, 400 MHz) δ | 11.05(1H,s), 9.55(1H,s), 9.18-9.13(2H,m),9.02(1H,s), 8.59(1H,s), 8.49-8.45 (1H,m) ,8.09-8.07(2H,d), 7.84-7.80(1H,t), 7.71-7.67(2H,t), 7.49-7.47(2H,d), 7.30-7.26(2H,t), 7.01-6.97 (1H,t) ,6.56(2H,s). |
| Mass(m/z) | 375,376,377 |

**Example 75**

**1-(2-Phenylamino-2-oxoethyl)-3-(benzyl sulfonyl hydrazino carbonyl) pyridiniumchloride. (compound no. 74),**

| | |
|---|---|
| Yield | 48% |
| M.P | 208-210°C |
| IR ( KBr,cm$^{-1}$) | 1712,1681,1632 |
| $^1$HNMR (DMSO $d_6$, 400 MHz) δ | 11.46(1H,s),10.80(1H,s), 9.59(1H,s), 9.22- 9.20(1H,d) ,9.08-9.06(1H,d), 8.38-8.36(1H,t),7.60-7.58(2H,d) ,7.49(2H,m), 7.39- 7.34(5H,m),7.13-7.10(1H, t), 5.74(2H,s), 4.52(2H,s). |
| Mass(m/z) | 425,426,427,428 |

**Example 76**

**1-(2-Phenyl-2-oxoethyl)-4-(methanesulfonyl hydrazino carbonyl) pyridinium bromide (compound no. 75),**

| | |
|---|---|
| Yield | 10% |
| M.P | 190-192°C |
| IR ( KBr,cm$^{-1}$) | 1679,1630,1650 |
| $^1$HNMR (DMSO $d_6$, 400 MHz) δ | 11.54(1H,s), 10.03(1H,s), 9.20-9.18(2H,d), 8.59-8.57(2H,d), 8.10-8.08(2H,d), 7.84 -7.80(1H,t), 7.71-7.67(2H,t), 6.56(2H,s), 3.08(3H,s). |
| Mass(m/z) | 334,335,336 |

**Example 77**

**1-(2-Phenyl-2-oxoethyl)-3-(phenyl hydrazino carbonyl) pyridinium bromide (compound no. 76),**

| | |
|---|---|
| Yield | 36% |

(continued)

| | |
|---|---|
| M.P | 204-206°C |
| IR ( KBr,cm$^{-1}$) | 1686,1653,1630 |
| $^1$HNMR (DMSO $d_6$, 400 MHz) δ | 11.01(1H,s) ,9.53(1H,s) ,9.17-9.16(2H,m), 8.46-8.42(1H,t), 8.09-8.07(2H,d), 7.82-7.78(1H,t), 7.69-7.65(2H,t), 7.20-7.16(2H,t), 6.88-6.86(2H,d), 6.79-6.75 (1H,t), 6.56(2H,s) |
| Mass(m/z) | 332,333 |

**Example 78**

**1-(2-Ethoxy-2-oxoethyl)-4-[2-(benzoyloxy) ethyl amino carbonyl ] pyridinium bromide (compound no. 77),**

| | |
|---|---|
| Yield | 82% |
| M.P | 154-156°C |
| IR ( KBr,cm$^{-1}$) | 1742,1719,1707,1675 |
| $^1$HNMR (DMSO $d_6$, 400 MHz) δ | 9.57-9.54(1H,t), 9.22-9.20(2H,d), 8.51- 8.49(2H,d), 8.00-7.98(2H,d), 7.68-7.64 (1H,t), 7.54-7.51(2H,t), 5.72(2H,s), 4.47- 4.44(2H,t) , 4.27-4.21(2H,q), 3.76-3.72(2H,q), 1.27-1.24. (3H,t) |
| Mass(m/z) | 357,358,359. |

**Example 79**

**1-(2-Ethoxy-2-oxoethyl)-3-(phenyl hydrazino carbonyl) pyridinium bromide.(compound no. 78),**

| | |
|---|---|
| Yield | 37% |
| M.P | 185-187°C |
| IR(KBr,cm$^{-1}$) | 1740,1690,1630. |
| $^1$HNMR (DMSo $d_6$, 400 MHz) δ | 11.01(1H,s), 9.58(1H,s), 9.23-9.14(2H,m), 8.42- 8.39(1H,t), 8.19(1H,s), 7.20-7.16(2H,t), 6.87-6.85(2H,d), 6.78-6.75(1H,t), 5.75(2H,s), 4.28-4.22(2H,q), 1.28-1.24(3H,t) |
| Mass(m/z) | 300,301,302. |

**Example 80**

**1-(2-Phenyl-2-oxoethyl)-3-(p-methoxyphenyl sulfonyl hydrazino carbonyl ) pyridinium bromide (compound no. 79),**

| | |
|---|---|
| Yield | 59% |
| M.P | 188-190°C |
| IR ( KBr,cm$^{-1}$) | 1671,1634,1580. |
| $^1$HNMR (DMSO $d_6$, 400 MHz) δ | 11.26-11.25(1H,d), 10.17- 10.16(1H,d) , 9.24(1H,s) , 9.03-9.01(1H,d), 8.87-8.85(1H,d) , 8.31-8.27(1H,t) , 7.97-7.96(2H,d) , 7.74 -7.69(3H,m) , 7.60-7.56(2H,t) , 6.99-6.97(2H,d) , 6.40(2H,s) n, 3.73(3H,s). |
| Mass(m/z) | 426,427,428,429 |

**Example 81**

**1-(2-Phenyl-2-oxoethyl)- 4-[2-(benzoyloxy) ethyl amino carbonyl ] pyridinium bromide (compound no. 80),**

| | |
|---|---|
| Yield | 92% |
| M.P | 202-204°C |
| IR ( KBr,$cm^{-1}$) | 1715,1692,1650 |
| $^1$HNMR (DMSO $d_6$, 400 MHz) δ | 9.55(1H,s) , 9.14 -9.13(2H,d) , 8.52- 8.51(2H,d), 8.07-7.99(4H,m) , 7.80-7.51 (6H,m) , 6.52(2H,s), 4.46(2H,s), 3.76-3.75(2H,s). |
| Mass(m/z) | 389,390,391,392 |

**Example 82**

**1-(2-Ethoxy-2-oxoethyl)-4-(p-methanesulfonyl hydrazino carbonyl) pyridinium bromide. (compound no. 81),**

| | |
|---|---|
| Yield | 45% |
| M.P | 94-96°C |
| IR (KBr,$cm^{-1}$) | 1726,1681,1643 |
| $^1$HNMR (DMSO $d_6$, 400 MHz) δ | 11.49(1H,s) ,9.98(1H,s),9.23-9.21(2H,d), 8.54-8.52(2H,d), 5.73(2H,s), 4.28-4.22(2H,q), 3.09(3H,s), 1.28-1.25(3H,t). |
| Mass(m/z) | 302,303,304,305. |

**Example 83**

**Preparation of 3-carbonylamino-1-(2-(2,4-dichlorophenyl) -2- oxoethyl) pyridinium bromide (compound 82)**

Nicotinamide (1.22g, 0.01 mol) was dissolved in refluxing toluene (40 ml) and a solution of 2,4-dichlorophenacyl bromide (3.0g, 0.012mol) in 10ml of toluene was added. The reaction mixture was refluxed for 7.5 hours and cooled. The precipitated solid was filtered and dissolved in methanol, decolourized with activated charcoal and concentrated under vacuum to one-fourth volume. It was cooled in ice - salt mixture and the precipitated solid was filtered and washed with methanol (3x10ml) to afford a pure solid.

| | |
|---|---|
| Yield | 39% |
| m.p. | 237-239°C |
| IR(KBr,$cm^{-1}$) | 3331, 3133, 1706, 1678 $^1$H NMR (DMSO $d_6$, 400 MHz) δ 9.54(1H,s), 9.18-9.11(2H,m),8.67(1H,s), 8.40(1H,t), 8.42-8.38(2H,m), 7.88(1H,s), 7.75 -7.72(1H,m), 6.49(2H,s) |
| Mass (m/z) | 309,310,311,312,187,159 |

According to the above mentioned procedure the following compounds are synthesized by reacting the corresponding pyridine derivatives with appropriate reagents by refluxing in alcoholic solvents like methanol, ethanol, propanol, etc. and high boiling solvents like toluene or xylene for 6-48 hours to give the desired compounds:

**Example 84**

**3-(Tetrahydrobenzothiazol-2-yl) aminocarbonyl)-1-(2-(2,4-dichlorophenyl)-2-oxoethyl) pyridinium bromide (compound 83):**

| | |
|---|---|
| Yield | 48% |
| m.p. | 165 - 167 °C(decomp.) |

(continued)

| | |
|---|---|
| IR(KBr,$cm^{-1}$) | 3333, 1714, 1684, 1635 |
| $^1H$ NMR($CD_3OD$, 400MHz) δ | 9.45(1H,s), 9.27-9.24(1H,m), 8.92-8.91(1H, m), 8.24 - 8.21(1H, m), 8.01 - 7.99 (1H, m), 7.72 - 7.71(1H, m) 7.57-7.54(1H,m), 2.59-2.57 (4H,m), 1.85(4H,m) |
| Mass (m/z) | 446, 447, 448, 449, 416, 307 and 266 |

**Example 85**

**1-(2- Phenyl -2- oxoethyl) -3- ((2- hydroxyethyl) aminocarbonyl) pyridinium bromide (compound 84):**

| | |
|---|---|
| Yield | 98% |
| m.p. | 182 - 184°C(decomp.) |
| IR(KBr,$cm^{-1}$) | 3289, 3241, 1690 and 1660 |
| $^1H$ NMR(DMSO $d_6$, 400MHz) δ | 9.47(1H,s), 9.21(1H,t), 9.09(2H,t),8.41-8.37(1H,m), 8.08-8.04(2H,m), 7.82-7.78 (1H,m), 7.69-7.65(2H,m), 6.52(2H,s), 4.86(1H,t), 3.58- 3.54(2H,m), 3.42-3.38 (2H,m) |
| Mass (m/z) : | 285,242,149,119,91 |

**Example 86**

**3-Carbonylamino-1-(2-thien-2'-yl-2-oxoethyl)-pyridinium bromide (compound 85 ).**

| | |
|---|---|
| Yield | 35% |
| m.p. | 212 - 215°C (decomp.) |
| IR(KBr,$cm^{-1}$) | 3295, 3126, 1680, 1671, 1640 |
| $^1H$ NMR ($DMSO_{d6}$, 400 MHz) δ | 9.49(1H,s), 9.13-9.11(1H,d), 9.07-9.05(1H,d), 8.60(1H,bs), 8.40-8.38(1H,m), 8.25-8.19(3H,m),7.43-7.40,(1H,t), 6.44(2H,s) |
| Mass(m/z) | 247,248,249,193 |

**Example 87**

**1- (2-Phenyl -2- oxoethyl)-3- ((p-sulfonamidophenylene) aminocarbonyl) pyridinium bromide (compound 86):**

| | |
|---|---|
| Yield | 44% |
| m.p. : | 188-190°C |
| IR(KBr,$cm^{-1}$) | 3296, 1700, 1679. |
| $^1H$ NMR ($DMSO_{d6}$, 400MHz) δ | 11.25 (1H,s), 9.58 (1H,s), 9.25 (1H,d), 9.16 (1H, d), 8.45 (1H,t), 8.10 (2H,d), 7.94 (2H,d), 7.86 (2H,d), 7.82(1H,t), 7.68(2H,t), 7.36(2H,s), 6.5(2H,s) |
| Mass (m/z) | 396, 277 |

**Example 88**

**1- (2- Ethoxy -2- oxoethyl) -3- ((2- hydroxyethyl) aminocarbonyl) pyridinium bromide (compound 87):**

| | |
|---|---|
| Yield | 87% |
| m.p. | 138-140°C |

(continued)

| | |
|---|---|
| IR(KBr, $cm^{-1}$) | 1748, 1669 |
| $^1H$ NMR ($CD_3OD$, 400 MHz) δ: | 9.43 (1H,s), 9.09-9.02 (2H,m), 8.26 (1H,m), 5.64 (2H,s), 4.31 (2H,q), 3.73 (2H, t), 3.54 (2H,t), 1.32 (3H,t) |
| Mass (m/z) | 251, 252, 165, 166 |

**Example 89**

**1- (2- Phenyl -2- oxoethyl) -3- (isopropyloxycarbonyl) pyridinium bromide (compound 88):**

| | |
|---|---|
| Yield | 46% |
| m.p. | 172-174°C |
| IR(KBr, $cm^{-1}$) | 1726, 1692 |
| $^1H$ NMR ($DMSO_{d6}$, 400MHz) δ | 9.55 (1H,s), 9.16 (1H,d), 9.08 (1H,d), 8.39-8.36 (1H,m), 8.04 (2H,d), 7.77 (1H, t), 7.64 (2H,t), 6.53 (2H,s), 5.25-5.19 (1H,m), 1.34 (6H,d) |
| Mass : (m/z) | 284, 285, 242 |

**Example 90**

**1- (2- Methyl-2-oxoethyl) -3- ((2- hydroxyethyl) aminocarbonyl) pyridinium chloride (compound 89):**

| | |
|---|---|
| Yield | 47% |
| m.p. | 178-180°C |
| IR(KBr,$cm^{-1}$) | 1727, 1660 |
| $^1H$ NMR ($DMSO_{d6}$, 400 MHz) δ: | 9.33 (1H,t), 9.30 (1H,s), 9.06 (1H,d), 8.90 (1H,d), 8.25-8.21 (1H,m), 5.75 (2H, s), 4.84 (1H,bs), 3.47 (2H,t), 3.30 (2H,t), 2.23 (3H,s) |
| Mass(m/z) | 223, 224, 225 |

**Example 91**

**1- (2- Thien -2'-yl -2- oxoethyl) -3- ((2- hydroxyethyl) aminocarbonyl) pyridinium bromide (compound 90):**

| | |
|---|---|
| Yield | 60% |
| m.p. | 207-209°C |
| IR(KBr, $cm^{-1}$) | 1673, 1656 |
| $^1H$ NMR ($DMSO_{d6}$, 400MHz) δ | 9.47 (1H,s), 9.18-9.05 (3H,m), 8.38-8.34 (1H,m), 8.23-8.19 (2H,m), 7.39 (1H,t), 6.44 (2H,s), 3.55-3.50 (2H,m), 3.40-3.37 (2H,m) |
| Mass (m/z) | 291, 292, 293 |

**Example 92**

**1- (2- (2,4- Dichlorophenyl) -2- oxoethyl) -3- (isopropyloxycarbonyl) pyridinium bromide (compound 91):**

| | |
|---|---|
| Yield | 26% |
| m.p. | 160-162°C |
| IR (KBr, $cm^{-1}$) | 1726, 1705 |

(continued)

| | |
|---|---|
| $^1H$ NMR ($DMSO_{d6}$, 400 MHz) δ | 9.55 (1H,s), 9.15 (1H,d), 9.08 (1H,d), 8.40-8.36 (1H,m), 8.11 (1H,d), 7.89 (1H, bs), 7.75- 7.72 (1H,m), 6.44 (2H,s), 5.26-5.20 (1H,m), 1.34 (6H,d). |
| Mass (m/z) | 352, 353, 354, 310 |

**Example 93**

**1- (2- Phenyl -2- oxoethyl) -3- ((4- methylthiazol -2- yl) aminocarbonyl) pyridinium bromide (compound 92):**

| | |
|---|---|
| Yield | 30% |
| m.p. | 165-167°C |
| IR (KBr, $cm^{-1}$) | 3409, 3319 and 1698 |
| $^1H$ NMR ($DMSO_{d6}$, 400 MHz) δ | 9.58 (1H,s), 9.22 (1H,d), 9.11 (1H,d), 8.42-8.38 (1H,m), 8.07 (2H,d), 7.81 (1H, t), 7.68 (2H,t), 6.86 (1H,bs), 6.56 (2H,s), 2.30 (3H,s) |
| Mass (m/z) | 337, 338, 232, 105. |

**Example 94**

**1- (2- Phenylamino -2- oxoethyl) -3- (n- butoxycarbonyl) pyridinium chloride (compound 93):**

| | |
|---|---|
| Yield | 10% |
| m.p. | 150-152°C |
| IR (KBr, $cm^{-1}$) | 3228, 1742, 1678 (bs) |
| $^1H$ NMR ($DMSO_{d6}$, 400 MHz) δ | 10.96 (1H,s), 9.65 (1H,s), 9.28 (1H,t), 9.09 (1H,d), 8.37 - 8.34 (1H,m), 7.62-7.59 (2H,m), 7.37 - 7.33 (2H,m), 7.11 (1H,t), 5.79 (2H,s), 4.41(2H,t), 1.76-1.72 (2H,m), 1.48-1.43 (2H,m), 0.94 (3H,t) |
| Mass(m/z) | 314, 315 |

**Example 95**

**1- (2- Phenylamino -2- oxoethyl) -3- (n- butylaminocarbonyl) pyridinium chloride (compound 94):**

| | |
|---|---|
| Yield | 37% |
| m.p. | 182-185°C |
| IR (KBr, $cm^{-1}$) | 3245, 1742, 1679 |
| $^1H$ NMR ($DMSO_{d6}$, 400MHz) δ | 10.97 (1H,s), 9.50(1H,s), 9.24 (1H,t), 9.13 (1H,d), 9.02 (1H,d), 8.28-8.25 (1H, m), 7.57 (2H,d), 7.30 (2H,t), 7.05 (1H,t), 5.70 (2H,s), 3.30 - 3.26 (2H,m), 1.52 - 1.48 (2H,m), 1.34 - 1.30 (2H,m), 0.86 (3H,t) |
| Mass (m/z) | 312, 313 |

**Example 96**

**1- (2- Phenylamino -2- oxoethyl) -3- ((2- hydroxyethyl) aminocarbonyl) pyridinium chloride (compound 95):**

| | |
|---|---|
| Yield | 58% |
| m.p. | 225-227°C |

(continued)

| | |
|---|---|
| IR (KBr, $cm^{-1}$) | 3448, 3271, 1702 and 1663 |
| $^1$H NMR ($DMSO_{d6}$,400 MHz) δ | 11.07 (1H,s), 9.58 (1H,s) 9.35 (1H,t), 9.17 (1H,d), 9.11 (1H,d), 8.33-8.29 (1H,m), 7.60 (2H,d), 7.32 (2H,t), 7.08 (1H,t), 5.75 (2H,s), 4.90 (1H,t), 3.57-3.53 (2H,m), 3.40-3.36 (2H,m) |
| Mass (m/z) | 300, 301, 302 |

**Example 97**

**1- (2- (2,4- Dichlorophenyl) -2- oxoethyl) -3- (n- butoxycarbonyl) pyridinium bromide (compound 96):**

| | |
|---|---|
| Yield | 38% |
| m.p. | 154-156°C |
| IR(KBr, $cm^{-1}$): | 3435, 3389, 1731 and 1704 |
| $^1$H NMR ($DMSO_{d6}$, 400 MHz) δ | 9.60 (1H,s), 9.21 (1H,d), 9.14 (1H,d), 8.43 (1H,t), 8.16(1H,d), 7.92 (1H,s), 7.78-7.76 (1H,m), 6.51 (2H,s), 4.42 (2H,t), 1.76-1.72 (2H,m), 1.48-1.42(2H,m), 0.94 (3H,t) |
| Mass (m/z) | 366, 367, 368, 369, 370 |

**Example 98**

**1- (2- (2,4- Dichlorophenyl) -2- oxoethyl) -3- (n-butylaminocarbonyl) pyridinium bromide (compound 97):**

| | |
|---|---|
| Yield | 35% |
| m.p. | 142-144°C |
| IR(KBr, $cm^{-1}$) | 3382, 1698, 1672 |
| $^1$H NMR ($DMSO_{d6}$, 400 MHz) δ | 9.37 (1H,s), 9.07 (1H,t), 8.99 (2H,t), 8.31-8.28 (1H,m), 8.04 (1H,d) 7.82-7.81 (1H,d), 7.68- 7.65 (1H,m), 6.34(2H,s), 3.27-3.24 (2H,m), 1.47-1.43 (2H,m), 1.29-1.24 (2H,m), 0.81 (3H,t) |
| Mass (m/z) | 365, 366, 367, 368, 369 |

**Cosmetic Preparation**

The preparation for use as a free radical scavenger and AGE breaker may contain one or more concentration of the compound in a cosmetically acceptable vehicle. The amount of the compound of invention will preferably range between 0.005 to 50% by weight (unless otherwise noted, all fraction amounts are expressed in weight percent), more preferably between 0.25% and 5.0%. The composition should be applied based on the requirement to an affected area.

Suitable vehicles or carriers for storage and/or delivery of the novel compound of this invention may be provided in lotion, liquid, ointment, gels, creams, spray, poultice or other forms, and will preferably have a lipophilic, hydrophilic or amphiphilic character. Suitable carriers include petrolatum, triglycerides, various esters, fatty alcohols, fatty acid, alkylene glycols, and ethanol, of which polyethylene glycol, polypropylene glycol, and polyethylene glycol are most preferred; if desired, compatible combinations of these vehicles are also suitable.

Further more the vehicles are present as needed for the desired delivery system. The vehicles or carriers can also have additional agents according to conventional practice. For example, the final composition may contain various emollients, emulsifiers, alcohols, colorants, fragrances, thickeners (such as xanthan gum), preservatives, humectants, surfactants (anionic, cationic, nonionic, amphoteric combinations), agents which modify skin differentiation and/or proliferation and/or pigmentation, antiparasitic agents, dispersants, opacifier, gelling agent, hydrating, agent, additional antioxidants, the typical botanical extracts such as those derived from aloe, citrus fruits, Witch Hazel, chamomile, and other like e.g., those having an astringent, antiseptic, sunscreens or suntan effects, skin toners, silicones, exfoliating agents, keratolytic agnets, retinoids, skin penetration enhancers, vitamins, thrombolytic agents, anticlotting agents, capillary protectants, hormones, antibacterial agents, antiviral agents, steroidal anti-inflammatory agents, anaesthetics,

anti-seborrhoeic agents, anti-dandruff agents, anti -acne agents, anti-free radical agents, analgesics, lipophilic compounds, antihistamine agents, insect repellants, skin cooling compounds, lubricants, anti-fungal agents or mixtures thereof. The composition may likewise include a penetration enhancer such as, but not limited to, Oleic acid, DMSO (dimethyl sulfoxide), alcohols, N-methylpyrolidone, dimethyl isosorbide. It may also include one or more additional active ingredients such as anti-inflammatory agents, antibiotic, astringents, growth factors, tocopherols, retinols, free radical scavengers.

**Example 99**

| | |
|---|---|
| Compound of invention | 0.25%w/w |
| Oleic acid | 10.0%w/w |
| Propylene Glycol | 70.0%w/w |
| Tween 80 | 0.1%w/w |
| Absolute ethanol.qs | 100.0%w/w |

**Example 100**

| | |
|---|---|
| Compound of invention | 0.25%w/w |
| Oleic acid | 10.0%w/w |
| Colliodalsilicon Dioxide | 6.0%w/w |
| Tween 80 | 0.1%w/w |
| Caprylic capricTriglyceride qs | 100.0%w/w |

A cosmetically acceptable organic fatty acid can optionally be present independently in the composition in an amount, preferably a bioactively effective amount, of 0.1%to 10.0%; the addition of fatty acid is a preferred ingredient.

It is believed that the effect of the compound of invention will be synergistically improved when combined with a humectant, an emollient, additional antioxidants or an anti-inflammatory agent.

**Example 101**

| | |
|---|---|
| Compound of invention | 0.5%w/w |
| Fatty acid | 4.0%w/w |
| Mineral oil | 5.0%w/w |
| Isocetyl stearate | 1.0%w/w |
| Antioxidant | 0.05%w/w |
| Xanthan gum | 0.2%w/w |
| Glycerol | 50.0%w/w |
| Diazolidinyl urea | 0.2%w/w |
| Lemon peel Extract | 0.02%w/w |
| Alcohol | 2.0%w/w |
| Purified water q.s. | 100.0%w/w |

The addition of humectants and emollients to the antioxidant composition is expected to aid in the rehydration and maintenance of hydration of the skin under consideration. Improved hydration of the skin is believed to both increase the absorbence of the free radical scavenger by the skin and helps in the delivery of the free radical scavenger to the active site.

Examples of the emollients which can be used are: mineral oil, petrolatum, paraffin, ceresin, ozokerite, microcrystalline wax, perhydrosqualene dimethyl polysiloxanes, methylphenyl polysiloxanes, silicone, silicone-glycol copolymers, triglyceride esters, acetylated monoglycerides, ethoxylated glycerides, alkyl esters of fatty acids, fatty acids

and alcohols, lanolin and lanolin derivatives, polyhydric alcohol esters, sterols, beeswax derivatives, polyhydric alcohols and polyethers, and amides of fatty acids . Although various emollients known in the art would be useful in the present invention, the preferred emollient is silicone.

Humectants known in the art to increase skin hydration when applied topically, such as polyhydric alcohols, are appropriate. Examples of suitable humectants are: glycerin, propylene glycol, butylene glycol, diglycerol, or ester derivatives thereof. However, the preferred humectant is glycerin.

The topical preparation of the present invention may contain a single antioxidant, apart from the compound of the invention or a combination of antioxidants, thus an antioxidant blend. The term "antioxidant" as used herein is intended to encompass both a single antioxidant as well as an antioxidant blend. The antioxidant may also be incorporated into various vehicles to facilitate topical application.

In order to obtain elegant, topical compositions in the form of cream, emulsions, lotions or gels, such compositions may include from about 0.001 wt% to about 50 wt% of an antioxidant.

The topical compositions of the present invention can be made as lotions and creams. The free radical scavenger can be combined with most emulsifiers that are used to make lotions, creams and other suitable topical vehicles. The emulsifiers can be cationic, anionic, nonionic, amphoteric, or a combination thereof. Nonionic emulsifiers are preferred. Exemplary nonionic emulsifiers are commercially available sorbitans, alkoxylated fatty alcohols and alkyl polyglycosides. Anionic emulsifiers may include soaps, alkyl sulfates, monoalkyl and dialkyl phosphates, alkyl sulphonates and acyl isothionates, an amphoteric emulsifier that may be used is lactamidopropyl trimonium chloride.

Suitable vehicles for the present invention may also contain thickeners. Examples of suitable thickeners include cellulose derivatives, such as hydroxyethyl cellulose and hydroxypropyl cellulose, as well as polyacrylic acid polymers.

Examples of preservatives that are suitable for use with the compositions include alkanols, especially ethanol and benzyl alcohol; parabens; sorbates; urea derivatives; and, isothiazolinones.

Lotions or creams according to the present invention can be made using conventional homogenization methods known to those skilled in the art. It is also possible to use a process of microfluidization that involves co-mixing the aqueous phase and the oil phase of such creams and lotions in a high-pressure homogenizer that reduces the emulsion particle size dramatically to about several microns of those in creams and lotions prepared without applying high pressure. Microfluidization allows one to prepare elegant stable creams and lotions containing effective amounts of the compound without the use of traditional emulsifiers and surfactants.

The topical compositions of the present invention can also be formulated as a microemulsion, which is a subcategory of emulsions, oils that may be used are mineral oil and silicone oil. Examples of alcohols that may be used are cetyl alcohol, isostearyl alcohol, stearyl alcohol, dodecanol and dodecenol. Nonionic surfactants may be fatty esters, esters of fatty alcohols or ethoxylated alcohols. Examples of nonionic surfactants are polyethylene glycol, isopropyl myristate, cetyl isooctadecanoate, polypropylene glycols, sorbitants and isopropyl oleate.

**Example 102**

| | |
|---|---|
| Compound of invention | 0.25%w/w |
| Fatty acid | 1.5%w/w |
| Surfactant | 3.0%w/w |
| Cosolvent | 70.0%w/w |
| Purified water ....qs | 100.0%w/w |

The topical compositions of the invention can be formulated as oil-in-water or water-in-oil emulsions. The compositions can also be in the form of a multiphase emulsion, such as a water-in-oil-in-water type emulsion

The compositions of the invention can also be made as a liposomal formulation. In such compositions, compound solution can be entrapped inside the liposomal vesicles with the shell of the liposome being a phospholipid or other suitable lipids (e.g. skin lipids). To form a topical composition, the liposomes can then be added to any carrier system described above according, to the preparation modes, uses and compositions of topical liposomes.

**Example 103**

| | |
|---|---|
| Compound of invention | 0.5%w/w |
| Phospholipid | 6.0%w/w |
| Antioxidants | 05%w/w |
| Ethanol | 15.0%w/w |
| Hydrophilic mediumqs | 100.0%w/w |

Solutions of compound and antioxidants can also be entrapped in polymeric vesicles with a shell consisting of a suitable polymeric material, such as gelatin, cross-linked gelatin, polyamide, polyacrylates and the like to form a vesicle that is then incorporated into the topical composition.

The composition according to the instant invention can be used for one or more of the following cosmetic applications, namely (a) reversing and preventing wrinkles b) reversing and preventing fine lines (c) promoting epidermal growth (d) photo protection (e) reversing and preventing skin discoloration (f) reversing and preventing age spots (g) conditioning and preventing dryness (h) reversing and preventing stretch marks (i) reversing and preventing blemishes (j) skin care/skin conditioning (k) reversing and preventing senile xerosis (l) conditioning and preventing sun burns (m) preventing and reversing the loss of collagen (n) improving skin texture (o) improving skin tone (p) enhancing skin thickness (q) decreasing pore size (r) restoring skin luster (s) minimising signs of fatigue (t) reducing acne, (u) treatment of Telangiectasia and (v) improving asthetic appearance of hair and nail.

**Non-Cosmetic Application of the Free-radical scavenging (anti-oxidant) property of the molecules.**

Apart from the use of the compounds of the invention for cosmetic applications based on their AGE-breaking and free-redical scavenging activities, the latter activity of these compounds can be used in strategies directed at control of oxidative stress for effective management of conditions discussed below:

***Neuro-degenerative disorders such as Alzheimer's disease (A.D.), Parkinson's disease (P. D.), Huntington's disease (H.D.), Motor neuron disease (M.N.D), Prion disease***

As people age, their antioxidant levels diminish and these low levels are directly linked to the many diseases associated with aging such as Alzheimer's and Parkinson's disease. One of the leading hypotheses is that oxidative stress induced by Relative Oxygen Species (ROS) damages essential components of the neurons, resulting ultimately in the neuronal death. Oxidative stress is involved in various divergent events leading to neuronal damage, including an increase in membrane rigidity, DNA strand break, and impairment in glucose uptake. Several potential sources of oxidative stress in different eurodegenerative disorders have been well identified **[Munch G, et al. 1998].**

In A.D. mitochondrial dysfunction, amyloid beta mediated processes; transition metal accumulation and genetic factors are responsible for the redox imbalance **[Smith MA, et al. 2000].**

Point mutations in Superoxide Dismutase enzymes are known in the familial form of MND.

Disturbances of neuronal energy metabolism have been implicated as a pathogenetic mechanism for H.D. **[Browne SE, et al. 1999]**

***Diabetes and Diabetic Vascular Complications (DVCs)***

The cause of oxidative stress in diabetes is not yet fully understood but is thought to be due to mitochondrial dysfunction, direct enzyme inhibition by hyperglycemia, auto-oxidation of glucose, and activation of nicotinamide-adenine dinucleotide phosphate (NADPH)-oxidase. Oxidative stress in diabetes is also increased due to weakened defenses due to reduced endogenous antioxidants. The oxidative stress manifests itself as elevated concentrations of lipid peroxidation products, erythrocyte fragility, and decreases in the antioxidant enzyme systems (CAT, GSH Px, SOD). Recent studies also have shown a positive correlation between blood glucose concentration and oxidant-induced lymphocyte DNA damage **[E.J. Harper The 24th Annual WALTHAM®/OSU SYMPOSIUM]**

ROS are generated during glucose oxidation and formation of advanced glycosylation end products (AGE). Evidence has accumulated indicating that the generation of ROS plays an important role in the development of DVCs. Many biochemical pathways associated with hyperglycemia such as advanced glycosylation, glucose auto oxidation, and polyol pathway can increase the production of free radicals. Hyperglycemia in diabetic patients leads to excess auto-oxidation of glucose thereby reducing molecular oxygen and yielding oxidizing intermediates such as superoxide ions ($O_2^-$), hydroxyl radicals (OH), and hydrogen peroxide ($H_2O_2$). Free radicals accelerate the formation of advanced glycosylation end products (AGE), because fragmentation and conformational changes occurring during glycosylation and glucose oxidation have been shown to be dependent upon free radicals. AGEs in turn supply more free radicals;

this process is termed as oxidative glycosylation or glycoxidation. These free radicals impair vascular relaxation by inactivating or quenching nitric oxide (NO) and also adversely affect the endothelial function. Evidence also suggests that Maillard reaction acts as an amplifier of oxidative damage in aging and diabetes **[D. Guigliano et al, 1996].**

***Intestinal diseases***

Oxidative stress is an important cause of tissue injury that occurs in inflammation and ischemia. ***Intestinal ischemia, radiation enteritis, inflammatory bowel disease,*** and promotion of ***gastric and colorectal cancers*** are some of the gastro-intestinal conditions where oxidative stress is implicated in the pathogenesis.

***Liver diseases***

**Alcoholic liver disease-** Ethanol induces an increase in lipid peroxidation either by enhancing ROS or decreasing the level of endogenous antioxidants. Ethanol also induces variety of cytochrome P450 enzymes in microsomes and xanthine oxidases in cytosol. The role of these enzymes in the generation of oxidative stress has been well established in various studies **[Ishii H, et al. 1997].**

Chronic hepatitis C- Enhanced oxidative stress initiates a fibrogenesis cascade in the liver of patients with chronic hepatitis C. Evidences are coming up supporting an oxidative stress pathway leading to active fibrogenesis in chronic hepatitis C. This fibrogenesis cascade characteristic of severe chronic hepatitis C (e.g., oxidative stress, induction of c-myb, activation of stellate cells, and collagen gene expression) is stimulated by ROS.

***Cancers***

Oxidative damage to DNA is a result of interaction of DNA with ROS, in particular the hydroxyl radical. The hydroxyl radicals produce multiple modifications in DNA. Oxidative attack by OH radical on the deoxyribose moiety leads to the release of free bases from DNA, generating strand breaks with various sugar modifications and simple abasic (AP) sites

ROS also interact with and modify cellular protein, lipid, and DNA, which results in altered target cell function. The accumulation of oxidative damage has been implicated in both acute and chronic cell injury including possible participation in the formation of cancer. Acute oxidative injury may produce selective cell death and a compensatory increase in cell proliferation. This stimulus may result in the formation of newly initiated preneoplastic cells and/or enhance the selective clonal expansion of latent initiated preneoplastic cells. Similarly, sublethal acute oxidative injury may produce unrepaired DNA damage and result in the formation of new mutations and, potentially, new initiated cells. ROS, therefore, can have multiple effects in the initiation stage of carcinogenesis by mediating carcinogen activation, causing DNA damage, and interfering with the repair of the DNA damage.

Benefits of various antioxidants in preventing or treating following cancers have been extensively studied.

**1) Lung cancer**
**2) Colorectal cancer**
**3) Cervical cancer**
**4) Breast cancer**
**5) Malignant melanoma**

***Oxidative stress in cardiac diseases***

Lifelong high levels of antioxidant nutrients are supposed to protect against the development of heart disease. High doses of antioxidants in the month following an acute **heart attack** have been shown to significantly reduce the number of deaths, as well as the extent of cardiac damage in non-fatal cases.

It is currently thought that increase in oxidative stress is involved in the pathophysiology of **endothelial dysfunction** that accompanies a number of cardiovascular risk factors including hypercholesterolemia, hypertension and cigarette smoking. It also plays a pivotal role in the evolution of clinical conditions such as atherosclerosis and heart failure. Oxidative stress can activate redox-sensitive kinase cascades and transcription factors such as $NF_KB$ and AP-1, with resulting increases in the expression of factors associated with an inflammatory response and cellular proliferation. There are three enzyme systems producing reactive oxygen species in the vascular wall: NADH/NADPH oxidase, xanthine oxidoreductase, and endothelial nitric oxide synthase (**Zalba Get al, 2000, Rosenfeld ME, 1998).**

Atherogenesis is regarded as the outcome of interactions among multiple stimuli. Endothelial dysfunction plays a key role in the development of **atherosclerosis**. Elevated homocysteine concentrations are associated with rapid onset of endothelial dysfunction, which is another mechanism by which increased oxidative stress contributes to

atherosclerosis. Oxidation of low-density lipoprotein plays an important role at several steps in atherogenesis. Oxidative stress also activates $NF_KB$, which induces expression of genes controlling cytokine expression and leukocyte adhesion to vascular wall. **(Maxwell, et al. 1997).**

Animal studies have provided evidence by suggesting that free radicals may promote thrombosis, directly damage vascular cells and other tissues, and interfere with vasomotor regulation with the clinical sequelae of **myocardial infarction and ischemic stroke.**

In tissues where oxygen supply becomes used up following ischemia, as in myocardial ischemia, the enzyme xanthine oxidase is changed to a form that has potential to reduce oxygen to superoxides. On readmission of oxygen e.g. by reperfusion there is a burst of free radical generation. ROS are formed at an accelerated rate in post-ischemic myocardium. Thus biochemical damage due to free radicals contributes to the ischemic injury.

Oxidative stress also seems to be one of the mechanisms that may produce membrane defects and result in intracellular calcium overload, and cardiac contractile dysfunction in the stunned myocardium.

***Macular degeneration and cataract***

Oxidative damage to lens of the eye with increase in age has a major contribution in cataract formation. Macular degeneration is also being recognized as a consequence of oxidative damage.

***HIV disease***

Perturbation of anti-oxidant defense system has been observed in various tissues in HIV patients. Oxidative stress may contribute to several aspects of HIV disease pathogenesis such as viral replication, inflammatory response, and decreased immune cell proliferation, loss of immune function, apoptosis, chronic weight loss. Antioxidants may offer a promising treatment to HIV patients.

***Chronic obstructive pulmonary diseases (COPD)***

Alteration in the alveolar and lung metabolism of glutathione is widely recognized as a central feature of many inflammatory lung diseases including COPD. These changes are a result of the alteration in the gene expression of the gamma- glutamyl cystine synthase (Gamma-GCS), the rate-limiting enzyme in glutathione synthesis. Oxidative stress is implicated in the pathogenesis of COPD, since it results in inactivation of anti proteinases, airspace epithelial injury, mucus hypersecretion, increased influx of neutrophils into the lungs, transcription factor activation and gene expression of pro-inflammatory mediators **[MacNee W, et al. 2001].**

***Renal Disease***

ROS have been implicated not only in the genesis of different forms of renal disease, predominantly experimentally induced **glomerulonephritis**, but also in different forms of **acute renal failure**.

***Asthma***

Although the pathogenesis of asthma is not fully defined, a typical feature is an increase in the number of inflammatory cells in the lung. Such cells generate ROS, which are involved in the pathophysiology of asthma, including airway smooth muscle contraction, increased airway reactivity, and increased vascular permeability.

**Effect of antioxidant status on immunologic function**

The immune system is particularly sensitive to oxidative stress, primarily because immune cells rely heavily on cell-to-cell communication to work effectively. Peroxidation of cell membranes compromises membrane integrity and disrupts intracellular signaling.

***Cataract:***

Oxidative damage to lens of eye with increase in age has been a major contribution in cataract formation.

Thus, by scavenging the free radicals, the following diseases can be managed.

1) Neurodegenerative disorders

(a) Alzheimer's Disease
(b) Parkinson's Disease
(c) Huntington's Disease
(d) Motor Neuron Disease
(e) Prion Disease

2) Diabetes and Diabetic Vascular Complications
3) Intestinal Diseases

(a) Intestinal Ischemia
(b) Radiation Enteritis
(c) Inflammatory Bowel Disease
(d) Gastric and Colorectal Cancers

4) Liver Diseases

(a) Alcoholic Liver Disease
(b) Chronic Hepatitis C

5) Cancers

(a) Lung Cancer
(b) Colorectal Cancer
(c) Cervical Cancer
(d) Breast Cancer
(e) Malignant Melanoma

6) Cardiac Diseases

(a) Atherosclerosis
(b) Myocardial Infarction
(c) Ischemic Stroke
(d) Endothelial dysfunction

7) Opthalmic Disorders

(a) Cataract formation
(b) Macular degeneration

8) HIV Disease
9) Respiratory Diseases

(a) Chronic Obstructive Pulmonary Diseases (COPD)
(b) Asthma

10) Renal Diseases

(a) Glomerulonephritis
(b) Acute Renal failure

**Pharmaceutical Compositions**

Pharmaceutical compositions effective for scavenging free radicals and/or inhibiting AGE may be prepared with a pharmaceutically effective quantity of compounds of general formula I, individually or in combination. The following pharmaceutical formulations suggested are by way of example alone and in no way restrict the forms in which they can be used.

**Oral formulations**

Oral formulations may be administered as solid dosage forms for example pellets, powders, sachets or discreet units such as tablets or capsules and like. Other orally administered pharmaceutical preparations include monophasic and biphasic liquid dosage forms either in ready to use form or forms suitable for reconstitution such as mixtures, syrups, suspensions or emulsions. The preparations in addition may contain diluents, dispersing agents, buffers, stabilizers, solubilizers, surfactants, preservatives, chelating agents and/or other pharmaceutical additives as are used. Aqueous or non aqueous vehicle or their combination may be used and if desired may contain suitable sweetener, flavouring agent or similar substances. In case of suspension or emulsion a suitable thickening agent or suspending agent or emulsifying agent may be present in addition. Alternatively, the compounds may be administered as such in their pure form unassociated with other additives for example as capsules or sachets. It may also be administered with a vehicle. Pharmaceutical preparations can have a slow, delayed or controlled release of active ingredients as is provided by a matrix or diffusion controlled system.

When the present invention or its salts or suitable complexes is presented as a discreet unit dosage like tablet, it may contain in addition medically inert excipients as are used in the art. Diluents such as starch, lactose, dicalcium phosphate, talc, magnesium stearate, polymeric substances like methyl cellulose, fatty acids and derivatives, sodium starch glycollate, etc. may also be used.

**Example 104**

**Preparation of oral dosage form:**

A typical tablet has the following composition:

| | |
|---|---|
| Active ingredient of formula I | an effective amount |
| Lactose | 135 mg |
| Starch | 76 mg |
| Polyvinyl pyrolidone (K-30) | 2 mg |
| Talc | 1.5 mg |
| Magnesium Stearate | 1.0 mg |

**Parenteral Formulations**

For parenteral administration, the compounds or their salts or suitable complexes thereof may be present in a sterile vehicle which may be an aqueous or non aqueous vehicle or a combination thereof. The examples of vehicles are water, ethyl oleate, oils and derivatives of polyols, glycols and their derivatives. It may contain additives common in injectable preparations like stabilizers, solubilizers, pH modifiers, buffers, antioxidants, cosolvents, complexing agents, tonicity modifiers, etc.

Some suitable additives are for example tartrate, citrate or similar buffers, alcohol, sodium chloride, dextrose and high molecular weight polymers. Another alternative is sterile powder reconstitution. The compound may be administered in the form of injection for more than once daily administration, or intravenous infusion/drip of suitable depot preparation.

**Example 105**

**Preparation suitable for parenteral administration has the following composition:**

| | |
|---|---|
| Active ingredient of formula I | an effective amount |
| Polethylene glycol (400) | 0.75 ml |
| Sodium metabisulphite | 0.01% |
| Isotonic saline/WFI | q.s. |

**Other Formulations.**

For the dermatological application and for the discoloration of teeth, the recommended formulations are lotions, oral rinse and toothpaste containing appropriate amount of the compounds of the general formula I.

The above examples are presented by way of illustration alone and in no way limit the scope of the invention.

## Claims

1. A cosmetic composition comprising an effective amount of a compound with free radical scavenging, AGE-breaking and AGE-inhibiting activity having the formula (I),

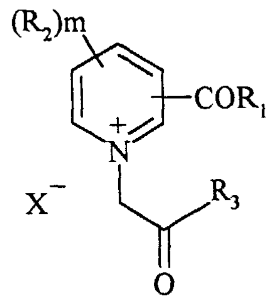

**(I)**

or its cosmetically acceptable salts contained in a cosmetically acceptable carrier
wherein
$R_1$ is $-R_4-R_5$ or $-N(R_7)$ N $(R_7)$ $R_9$; and Y-$R_{11}$
$R_4$ is selected from the group consisting of $-N(R_7)R_6O-$,
$-N(R_7)R_6N(R_7)$,
$-OR_6O$, and $-OR_6N(R_7)-$,
where $R_6$ is alkyl with $C_2$-$C_8$ Carbon atoms;
$R_5$ is selected from the group consisting of alkyl, aryl including heteroaryl, $-COR_7$, $-SO_2R_7$, -C(S) $NHR_7$, $-C(NH)NHR_7$, $-COR_{10}$,

$-C(O)NHR_7$ and 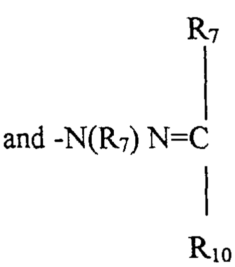

where $R_7$ is selected from the group consisting of H, alkyl and aryl including heteroaryl, provided $R_7$ may be the same or different for $R_1$ and $R_3$ in the same compound;
$R_2$ is selected from the group consisting of F, Cl, Br, I, $OR_7$, $NO_2$, alkyl, aryl including heteroaryl, formyl, acyl, C (O)$NR_7R_{10}$, C(O)$OR_7$, $NR_7R_{10}$, $N=C(R_7)(R_{10})$, $SR_7$, $SO_2NH_2$, SO2 alkyl and $SO_2$aryl;
m is 0, 1 or 2;
$R_3$ is selected from the group consisting of $R_7$, $OR_7$, $N(R_7)$ $(R_{10})$, $N=C(R_7)$ $(R_{10})$, $N(R_7)$ $N(R_7)$ $(R_{10})$, $N(R_7)$ N-C $(R_7)$ $(R_{10})$ and $CH(R_7)C(O)R_8$

where $R_8$ is selected from the group consisting of $R_7$, $OR_7$ and $NR_7R_{10}$; $R_9$ is selected from the group consisting of hydrogen, alkyl, aryl including heteroaryl, $C(O)R_{10}$, $-SO_2R_{10}$, $C(S)NHR_{10}$, $C(NH)NH$ ($R_{10}$) and C(O) $NHR_{10}$; $R_{10}$ is selected from the group consisting of H, alkyl and aryl, including heteroaryl and in each case may be the same or different from substituent $R_7$, provided $R_{10}$ may be the same or different for $R_1$ and $R_3$ in the same compound;
Y is selected from oxygen, NH, $NR_{12}$ and null;
$R_{11}$ and $R_{12}$ are independently selected from hydrogen, alkyl and aryl.
X is selected from group consisting of a halide ion, acetate ion, perchlorate ion, sulfonate ion, oxalate ion, citrate ion, tosylate ion, maleate ion, mesylate ion, carbonate ion, sulfite ion, phosphoric hydrogen ion, phosphonate ion, phosphate ion, $BF_4$- and $PF_6$-; with proviso that,

(i) when two alkyl groups are present on the same carbon or nitrogen, they may be linked together to form a cyclic structure;
(ii) the nitrogen of heteroaryl ring of $R_{10}$, when present, may be quaternized;
(iii) when $R_3$ is $OR_7$ and $R_1$ is $-NHNH_2$ then $R_7$ is not alkyl and
(iv) when $R_3$ is $OR_7$, $R_1$ is $N(R_7)(NR_7)R_9$ and $R_9$ is $C(O)R_{10}$ where

$R_{10}$ is alkyl, then $R_7$ is not hydrogen.

**2.** The composition as claimed in claim 1, wherein $-C(O)R_1$ group of said compound is at position 3 or 4.

**3.** The composition as claimed in claim 2, wherein $-C(O)R_1$ group of said compound is at position 3.

**4.** The composition as claimed in claim 1, wherein for the said compound m is 0 or 1.

**5.** The composition as claimed in claim 2, wherein for the said compound m is 0 or 1.

**6.** The composition as claimed in claim 3, wherein for the said compound m is 0 or 1.

**7.** The composition as claimed in claim 1, wherein for the said compound m is 0

**8.** The composition as claimed in claim 2, wherein for the said compound m is 0.

**9.** The composition as claimed in claim 3, wherein for the said compound m is 0.

**10.** The composition as claimed in claim 1, wherein for the said compound X is a halide ion.

**11.** The composition as claimed in claim 1, wherein said compound is selected from the group consisting of:

(a) N,N'-bis[3-carbonyl-1-(2-thien -2'- yl -2-oxoethyl) -3-pyridinium] hydrazine dibromide or other cosmetically acceptable salts thereof,
(b) 1-(2-ethoxy -2-oxoethyl) -3-(2-(2-pyridyl)hydrazinocarbonyl) pyridinium bromide or other cosmetically acceptable salts thereof,
(c) 1-(2-ethoxy -2-oxoethyl) -3-(2-(benzoyloxy) ethylamino carbonyl) pyridinium bromide or other cosmetically acceptable salts thereof,
(d)N,N'-bis[3-carbonyl-1-(2-phenyl-2-oxoethyl)pyridinium)hydrazine dibromide or other cosmetically acceptable salts thereof,
(e)1-(2-phenyl-2-oxoethyl)-3-(hydrazinocarbonyl)pyridinium bromide or other cosmetically acceptable salts thereof,
(f) 1-(2-thien -2'-yl -2-oxoethyl) -3-(methanesulfonyl hydrazinocarbonyl) pyridinium bromide or other cosmetically acceptable salts thereof,
(g) N,N'-bis[3-carbonyl-1-(2-(2',4'-dichlorophenyl)-2-oxoethyl) pyridinium] hydrazine dibromide or other cosmetically acceptable salts thereof,
(h) 1-(2-phenyl -2-oxoethyl) -3-(methanesulfonyl hydrazinocarbonyl) pyridinium bromide or other cosmetically acceptable salts thereof,
(i) 1-(2-ethox -2-oxoethyl) -3-(methanesulfonyl hydrazinocarbonyl) pyridinium bromide or other cosmetically acceptable salts thereof,
(j)1-(2-phenyl-2-oxoethyl)-3-(phenylsulfonylhydrazino carbonyl) pyridinium bromide or other cosmetically acceptable salts thereof,

(k)1-(2-phenyl-2-oxoethyl)-2-chloro-3-(phenylsulfonyl hydrazino carbonyl) pyridinium bromide or other cosmetically acceptable salts thereof,

(l) 1-(2-thien-2'-yl -2-oxoethyl)-4-(2-(benzoyloxy) ethyl aminocarbonyl) pyridinium bromide or other cosmetically acceptable salts thereof,

(m)1- (2- (2,'4'- dichlorophenyl) -2-oxoethyl) -3-(2- (benzoyloxy) ethylaminocarbonyl) pyridinium bromide or other cosmetically acceptable salts thereof,

(n) 1-(2-phenyl -2-oxoethyl) -3-(2-(acetoxy) ethyloxy) carbonyl) pyridinium bromide or other cosmetically acceptable salts thereof,

(o) 1-(2-ethoxy -2-oxoethyl) -3-(2-(benzoyloxy) ethyloxy carbonyl) pyridinium bromide or other cosmetically acceptable salts thereof,

(p) 1-(2-phenylamino-2-oxoethyl)-4-(phenylsulfonyl hydrazino carbonyl)pyridinium chloride or other cosmetically acceptable salts thereof,

(q) 1-(2-(2,'4'-dichlorophenyl)-2-oxoethyl)-3- (2(methoxy) ethyloxycarbonyl) pyridinium bromide or other cosmetically acceptable salts thereof,

(r) 1-(2-phenylamino-2-oxoethyl)-3-((benzoyloxy) ethylaminocarbonyl) pyridinium chloride or other cosmetically acceptable salts thereof,

(s) 1-(2-thien-2'-yl-2-oxoethyl)-3-(phenylaminocarbonyl hydrazinocarbonyl) pyridinium bromide or other cosmetically acceptable salts thereof,

(t) 1-(2-phenyl-2-oxoethyl)-3-(2-(acetoxy) ethylaminocarbonyl) pyridinium bromide or other cosmetically acceptable salts thereof,

(u) 1-(2-phenylamino-2-oxoethyl)-3-(phenyl sulfonyl hydrazino carbonyl) pyridinium chloride or other cosmetically acceptable salts thereof,

(v) 1-(2-phenylamino-2-oxoethyl)-3-((4-methylphenyl)sulfonyl hydrazinocarbonyl) pyridinium chloride or other cosmetically acceptable salts thereof,

(w) 1-(2-phenyl-2-oxoethyl)-3-(2-(benzoyloxy)ethyloxy carbonyl) pyridinium bromide or other cosmetically acceptable salts thereof,

(x) 1-(2-thien-2'-yl-2-oxoethyl)-3-(phenylcarbonyl hydrazino carbonyl) pyridinium bromide or other cosmetically acceptable salts thereof,

(y) 1-(2-ethoxy-2-oxoethyl)-3-((phenylmethyl)sulfonyl hydrazino carbonyl)pyridinium bromide or other cosmetically acceptable salts thereof,

(z)1-(2-phenyl-2-oxoethyl)-3 -((phenylmethyl)sulfonyl hydrazino carbonyl)pyridinium bromide or other cosmetically acceptable salts thereof,

(aa) N, N' - bis [3-carbonyl-1-(2-furan-2'-yl-2-oxoethyl) pyridinium] hydrazine dibromide or other cosmetically acceptable salts thereof,

(ab) N,N'-bis [3-carbonyl -1- (2-thien-2'-yl-2-oxoethyl) pyridinium] hydrazine dichloride or other cosmetically acceptable salts thereof,

(ac) N,N'-bis-[3-carbonyl-1-(2-cyclopropylamino-2-oxoethyl) pyridinium] hydrazine dichloride or other cosmetically acceptable salts thereof,

(ad) 1-(2',4'-dichlorophenyl-2-oxoethyl)-3-(2-methoxyethyl aminocarbonyl)-pyridinium bromide or other cosmetically acceptable salts thereof,

(ae) 1-(2-thien-2'-yl-2-oxoethyl)-3-((2-methoxy ethyl) amino carbonyl)-5-bromo pyridinium chloride or other cosmetically acceptable salts thereof,

(af) 1-(2-thien-2'-yl-2-oxoethyl)-3-(methanesulfonyl hydrazino carbonyl) pyridinium chloride or other cosmetically acceptable salts thereof,

(ag) 1-(2-thien-2'yl-2-oxoethyl)-3-(2-(2-chloro-3-pyridoylhydrazinocarbonyl)-pyridinium chloride or other cosmetically acceptable salts thereof,

(ah) 1-(2-cyclopropylamino-2-oxoethyl)-3-(2-methoxyethylaminocarbonyl) -pyridinium chloride or other cosmetically acceptable salts thereof,

(ai) 1-(2-isopropylamino-2-oxoethyl)-3-(2-methylsulfonylhydrazinocarbonyl)-pyridinium chloride or other cosmetically acceptable salts thereof,

(aj) 1-(2-phenylamino-2-oxo ethyl)-3-({2-(1-oxo-3-cyclohexyl)-propyl} - hydrazinocarbonyl)-pyridinium bromide or other cosmetically acceptable salts thereof,

(ak) 1-(2-thien-2'-yl-2-oxoethyl)-3-[2-(benzoyloxy)ethylamino carbonyl]-pyridinium bromide or other cosmetically acceptable salts thereof,

(al) 1-(4-ethoxy-2, 4-dioxobutyl)-3-(2-(benzoxyloxy)ethylamino carbonyl)-pyridinium chloride or other cosmetically acceptable salts thereof,

(am)1-(2-thien-2'-yl-2-oxoethyl)-3-[1-oxo-1-(2-methoxy carbonyl) pyridyl] hydrazino pyridinium chloride or other cosmetically acceptable salts thereof,

(an)1-[1-(2-thien-2'-yl-2-oxoethyl)-5-aminocarbonyl-3-carbonyl pyridinium]-2-[1-(2-thien-2'-yl-2-oxoethyl )-3-carbonyl pyridinium] hydrazine dichloride or other cosmetically acceptable salts thereof,

(ao)1-(2-thien-2'-yl-2-oxoethyl)-3-(trifluoromethanesulfonyl hydrazino carbonyl) - pyridinium bromide or other cosmetically acceptable salts thereof,

(ap)1-[1-(2-thien-2'-yl-2-oxoethyl)-6-methyl-3-carbonyl pyridinium]-2-[1-(2-thien-2'-yl-2-oxoethyl )-3-carbonyl pyridinium ] hydrazine dichloride or other cosmetically acceptable salts thereof,

(aq) N,N'-bis[3-carbonyl-1-(2-(5-methyl-thien-2-yl)-2-oxoethyl) pyridinium] hydrazine dichloride or other cosmetically acceptable salts thereof,

(ar) N,N'-bis[3-carbonyl-1-(2-(5-chloro-thien-2-yl)-2-oxoethyl) pyridinium] hydrazine dichloride or other cosmetically acceptable salts thereof,

(as) 1-(2-thien-2'-yl-2-oxoethyl)-3-(methanesulfonyl hydrazino carbonyl )-6-methyl pyridinium bromide or other cosmetically acceptable salts thereof,

(at) N,N'-bis[3-carbonyl-1-(2-(4-nitro-thien-2-yl)-2-oxoethyl)pyridinium] hydrazine dichloride or other cosmetically acceptable salts thereof,

(au) 1-(2-phenylamino-2-oxoethyl)-3-(phenyl hydrazino carbonyl) pyridinium chloride or other cosmetically acceptable salts thereof,

(av) 1-(2-(4-nitro-thien-2-yl)-2-oxoethyl)-3-(methanesulfonyl hydrazino carbonyl ) pyridinium bromide or other cosmetically acceptable salts thereof,

(aw) 1-(2-(5-nitro-thien-2-yl)-2-oxoethyl)-3-(methanesulfonyl hydrazino carbonyl) pyridinium chloride or other cosmetically acceptable salts thereof,

(ax) 1-(2-(5-chloro-thien-2-yl)-2-oxoethyl)-3-(methanesulfonyl hydrazino carbonyl) pyridinium bromide or other cosmetically acceptable salts thereof,

(ay) 1-(2-thien-2'-yl-2-oxoethyl)-3-(ethoxycarbonyl hydrazino carbonyl) pyridinium bromide or other cosmetically acceptable salts thereof,

(az) 1-(2-thien-2'-yl-2-oxoethyl)-3-(isopropylsulfonyl hydrazino carbonyl) pyridinium bromide or other cosmetically acceptable salts thereof,

(ba) 1-(2-thien-2'-yl-2-oxoethyl)-3-(methanesulfonyl hydrazino carbonyl) -5-bromo pyridinium bromide or other cosmetically acceptable salts thereof,

(bb) 1-(2-(2-ethoxy carbonyl pyrrolidin-1-yl)-2-oxoethyl)-3-(methanesulfonyl hydrazino carbonyl) pyridinium chloride or other cosmetically acceptable salts thereof,

(bc) 1-(2-(5-methyl-thien-2-yl)-2-oxoethyl)-3-(methanesulfonyl hydrazino carbonyl) pyridinium chloride or other cosmetically acceptable salts thereof,

(bd) 1-(2-(4- carboethoxy-thiazolidin-3-yl)-2-oxoethyl) -3-(methanesulfonyl hydrazino carbonyl ) pyridinium chloride or other cosmetically acceptable salts thereof,

(be) 1-(2-(4-benzyl piperidin-1-yl)-2-oxoethyl)-3-(methanesulfonyl hydrazino carbonyl) pyridinium chloride or other cosmetically acceptable salts thereof,

(bf) N,N(-bis[3-carbonyl-1-(2-(2-ethoxycarbonyl pyrrolidin-1-yl)-2-oxoethyl) pyridinium] hydrazine dichloride or other cosmetically acceptable salts thereof,

(bg) 1-(2-phenylamino-2-oxoethyl)-4 -[ 2-(benzoyloxy) ethylamino carbonyl ] pyridinium chloride or other cosmetically acceptable salts thereof,

(bh) 1-(2-thien-2(-yl-2-oxoethyl)-3-(phenyl hydrazino carbonyl) pyridinium bromide or other cosmetically acceptable salts thereof,

(bi) 1-(2-thien-2(-yl-2-oxoethyl)-3-(p-methoxy phenyl sulfonyl hydrazino carbonyl) pyridinium bromide or other cosmetically acceptable salts thereof,

(bj) 1-(2-ethoxy-2-oxoethyl)-3-(phenyl aminocarbonyl hydrazino carbonyl ) pyridinium bromide or other cosmetically acceptable salts thereof,

(bk) 1-(2-ethoxy-2-oxoethyl)-3-(p-toluene sulfonyl hydrazino carbonyl) pyridinium bromide or other cosmetically acceptable salts thereof,

(bl) 1-(2-phenyl-2-oxoethyl)-3-(phenylamino carbonyl hydrazino carbonyl ) pyridinium bromide or other cosmetically acceptable salts thereof,

(bm) 1-(2-phenylamino-2-oxoethyl)-3-(benzyl sulfonyl hydrazino carbonyl) pyridiniumchloride or other cosmetically acceptable salts thereof,

(bn) 1-(2-phenyl-2-oxoethyl)-4-(methanesulfonyl hydrazino carbonyl ) pyridinium bromide or other cosmetically acceptable salts thereof,

(bo) 1-(2-phenyl-2-oxoethyl)-3-(phenyl hydrazino carbonyl ) pyridinium bromide or other cosmetically acceptable salts thereof,

(bp) 1 -(2-ethoxy-2-oxoethyl)-4-[2-(benzoyloxy) ethyl amino carbonyl ] pyridinium bromide or other cosmetically acceptable salts thereof,
(bq) 1-(2-ethoxy-2-oxoethyl)-3-(phenyl hydrazino carbonyl ) pyridinium bromide or other cosmetically acceptable salts thereof,
(br) 1-(2-phenyl-2-oxoethyl)-3-(p-methoxyphenyl sulfonyl hydrazino carbonyl ) pyridinium bromide or other cosmetically acceptable salts thereof,
(bs) 1-(2-phenyl-2-oxoethyl)- 4-[2-(benzoyloxy) ethyl amino carbonyl] pyridinium bromide or other cosmetically acceptable salts thereof,
(bt) 1-(2-ethoxy-2-oxoethyl)- 4-(p-methanesulfonyl hydrazino carbonyl ) pyridinium bromide or other cosmetically acceptable salts thereof,
(bu) 3-carbonylamino-1- (2-(2, 4-dichlorophenyl)-2-oxoethyl)-pyridinium bromide or other cosmetically acceptable salts thereof,
(bv) 3-(tetrahydrobenzothiazol-2-yl) aminocarbonyl -1-(2-(2, 4-dichlorophenyl)-2-oxoethyl)-pyridinium bromide or other cosmetically acceptable salts thereof,
(bw) 1-(2-phenyl-2-oxoethyl)-3-((2-hydroxyethyl) aminocarbonyl) pyridinium bromide or other cosmetically acceptable salts thereof,
(bx) 3-carbonylamino-1- (2-thien-2'-yl-2-oxoethyl)-pyridinium bromide or other cosmetically acceptable salts thereof,
(by) 1-(2-phenyl -2-oxoethyl)-3-((p-sulfonamidophenylene) aminocarbonyl) pyridinium bromide or other cosmetically acceptable salts thereof,
(bz) 1-(2-ethoxy-2-oxoethyl)-3-((2-hydroxyethyl) aminocarbonyl) pyridinium bromide or other cosmetically acceptable salts thereof,
(ca) 1-(2-phenyl-2-oxoethyl)-3-(isopropyloxycarbonyl) pyridinium bromide or other cosmetically acceptable salts thereof,
(cb) 1-(2-oxopropyl)-3-((2-hydroxyethyl) aminocarbonyl) pyridinium chloride or other cosmetically acceptable salts thereof,
(cc) 1-(2-thien-2'-yl-2-oxoethyl)-3-((2-hydroxyethyl) aminocarbonyl) pyridinium bromide or other cosmetically acceptable salts thereof,
(cd) 1-(2-(2,4 - dichlorophenyl-2-oxoethyl) -3- (isopropyloxycarbonyl) pyridinium bromide or other cosmetically acceptable salts thereof,
(ce) 1- (2-phenyl - 2- oxoethyl) - 3- ((4-methylthiazol - 2 -yl) aminocarbonyl) pyridinium bromide or other cosmetically acceptable salts thereof,
(cf) 1-(2- phenylamino -2- oxoethyl ) -3- (n-butyloxycarbonyl) pyridinium chloride or other cosmetically acceptable salts thereof,
(cg) 1- (2- phenylamino - 2- oxoethyl)- 3- ((2-hydroxyethyl) aminocarbonyl) - pyridinium chloride or other cosmetically acceptable salts thereof,
(ch) 1- (2- (2,4 - dichlorophenyl ) - 2-oxoethyl) - 3- (n - butoxycarbonyl) pyridinium bromide or other cosmetically acceptable salts thereof,
(ci) 1-(2 - (2,4 - dichlorophenyl) - 2-oxoethyl) - 3- (n-butylamino-carbonyl) pyridinium bromide or other cosmetically acceptable salts thereof,
(cj) 1-(2-phenyl-2-oxoethyl)-3-(1-phenyl-1-oxomethyl) pyridinium bromide or other cosmetically acceptable salts thereof and
(ck) 1- (2 phenyl - 2-oxoethyl) - 3- (methoxycarbonyl) pyridinium bromide or other cosmetically acceptable salts thereof.

**12.** The composition as claimed in claim 1, which is suitable for

a) reversing and preventing wrinkles,
b) reversing and preventing fine lines,
c) promoting epidermal growth,
d) photo protection,
e) reversing and preventing skin discoloration,
f) reversing and preventing age spots,
g) conditioning and preventing dryness,
h) reversing and preventing stretch marks,
i) reversing and preventing blemishes,
j) skin care/ skin conditioning,
k) reversing and preventing senile xerosis,
l) conditioning and preventing sun burns,

m) preventing and reversing the loss of collagen,
n) improving skin texture,
o) imporving skin tone,
p) enhancing of skin thickness,
q) decreasing pore size,
r) restoring skin luster,
s) minimising signs of fatigue,
t) reducing acne,
u) treatment of Telangiectasia or
v) improving the aesthetic appearance of hair and nails.

**13.** A composition useful for the cosmetic application comprising an effective amount of a compound with free radical scavenger, AGE breaker and AGE inhibitor activity as defined in claim 1 or its cosmetically acceptable salts contained in a cosmetically acceptable carrier wherein said composition is effective for atleast one of the following applications:

a) reversal and prevention of wrinkles,
b) reversal and prevention of fine lines,
c) promotion of epidermal growth,
d) photo protection of skin,
e) reversal and prevention of skin discoloration,
f) reversal and prevention of age spots,
g) conditioning and prevention of dry spot,
h) reversal and prevention of stretch marks,
i) reversal and prevention of blemishes,
j) skin care and conditioning,
k) reversal and prevention of senile xerosis,
l) conditioning and prevention of sun burns,
m) preventing and reversing the loss of collagen,
n) improving skin texture,
o) imporving skin tone,
p) enhancing of skin thickness,
q) decreasing pore size,
r) restoring skin luster,
s) minimising signs of fatigue,
t) reducing acne,
u) treatment of Telangiectasia and
v) improving aesthetic appearance of hair and nails.

**14.** The composition as claimed in claim 13, in the form of a solution, gel, ointment, lotion, cream, microemulsion aerosol, dispersion or milk.

**15.** A method of cosmetic application with reversing and preventing effects on aging and wrinkling of the skin comprising applying an effective amount of a cosmetic composition comprising a compound with free radical scavenger, AGE-breaker and AGE-inhibitor activity having the formula (I) as defined in Claim 1 or its cosmetically acceptable salts contained in a cosmetically acceptable carrier.

**16.** The method as claimed in claim 15, wherein the effective amount is effective for aging.

**17.** The method as claimed in claim 16, wherein aging is extrinsic aging and intrinsic aging.

**18.** The method as claimed in claim 16, wherein aging is extrinsic aging.

**19.** A method of cosmetic application with reversing and preventing effects on atleast one of the following:

i) fine lines,
ii) skin discoloration
iii) age spots

iv) stretch marks
v) blemishes
vi) senile xerosis and
vii) preventing and reversing loss of collagen

comprising applying an effective amount of a cosmetic composition comprising compound with free radicals scavenger, AGE breaker and AGE inhibitor activity having the formula (I) as defined in claim 1 or its cosmetically acceptable salts contained in a cosmetically acceptable carrier.

**20.** A method of cosmetic application with conditioning and preventing effects in skin dryness and /or sun burns comprising applying an effective amount of a cosmetic composition comprising a compound with free radical scavenger, AGE breaker and AGE inhibitor activity having the formula (I) as defined in claim 1 or cosmetically acceptable salts thereof contained in a cosmetically acceptable carrier.

**21.** A method of cosmetic application with effects of promoting epidermal growth and/or photo protection, improving skin texture, improving skin tone, enhancing skin thickness, decreasing pore size, restoring skin luster, minimizing signs of fatigue, reducing tone, treatment of telangiectasia comprising applying an effective amount of a cosmetic composition comprising a compound with free radical scavenger, AGE breaker and AGE inhibitor activity having the formula (I) as defined in claim 1 or its cosmetically acceptable salts contained in a cosmetically acceptable carrier.

**22.** The method as claimed in claim 15, wherein said compound is selected from the group consisting of the following compounds:

(a) N,N'-bis[3-carbonyl-1-(2-thien -2'- yl -2-oxoethyl) -3-pyridinium] hydrazine dibromide or other cosmetically acceptable salts thereof,
(b) 1-(2-ethoxy -2-oxoethyl) -3-(2-(2-pyridyl)hydrazinocarbonyl) pyridinium bromide or other cosmetically acceptable salts thereof,
(c) 1-(2-ethoxy -2-oxoethyl) -3-(2-(benzoyloxy) ethylamino carbonyl) pyridinium bromide or other cosmetically acceptable salts thereof,
(d)N,N'-bis[3-carbonyl-1-(2-phenyl-2-oxoethyl)pyridinium]hydrazine dibromide or other cosmetically acceptable salts thereof,
(e)1-(2-phenyl-2-oxoethyl)-3-(hydrazinocarbonyl)pyridinium bromide or other cosmetically acceptable salts thereof,
(f) 1-(2-thien -2'-yl -2-oxoethyl) -3-(methanesulfonyl hydrazinocarbonyl) pyridinium bromide or other cosmetically acceptable salts thereof,
(g) N,N'-bis[3-carbonyl-1-(2-(2',4'-dichlorophenyl)-2-oxoethyl) pyridinium] hydrazine dibromide or other cosmetically acceptable salts thereof,
(h) 1-(2-phenyl -2-oxoethyl) -3-(methanesulfonyl hydrazinocarbonyl) pyridinium bromide or other cosmetically acceptable salts thereof,
(i) 1-(2-ethoxy -2-oxoethyl) -3-(methanesulfonyl hydrazinocarbonyl) pyridinium bromide or other cosmetically acceptable salts thereof,
(j)1-(2-phenyl-2-oxoethyl)-3-(phenylsulfonylhydrazino carbonyl) pyridinium bromide or other cosmetically acceptable salts thereof,
(k)1-(2-phenyl-2-oxoethyl)-2-chloro-3-(phenylsulfonyl hydrazino carbonyl) pyridinium bromide or other cosmetically acceptable salts thereof,
(1) 1-(2-thien -2'-yl -2-oxoethyl)-4-(2-(benzoyloxy) ethyl aminocarbonyl) pyridinium bromide or other cosmetically acceptable salts thereof,
(m)1- (2- (2,'4'- dichlorophenyl) -2-oxoethyl) -3-(2- (benzoyloxy) ethylaminocarbonyl) pyridinium bromide or other cosmetically acceptable salts thereof,
(n) 1-(2-phenyl-2-oxoethyl) -3-(2-(acetoxy) ethyloxy) carbonyl) pyridinium bromide or other cosmetically acceptable salts thereof,
(o) 1-(2-ethoxy -2-oxoethyl) -3-(2-(benzoyloxy) ethyloxy carbonyl) pyridinium bromide or other cosmetically acceptable salts thereof,
(p) 1-(2-phenylamino-2-oxoethyl)-4-(phenylsulfonyl hydrazino carbonyl)pyridinium chloride or other cosmetically acceptable salts thereof,
(q) 1-(2-(2,'4'-dichlorophenyl)-2-oxoethyl)-3- (2(methoxy) ethyloxycarbonyl) pyridinium bromide or other cosmetically acceptable salts thereof,

(r) 1-(2-phenylamino-2-oxoethyl)-3-((benzoyloxy) ethylaminocarbonyl) pyridinium chloride or other cosmetically acceptable salts thereof,
(s) 1-(2-thien-2'-yl-2-oxoethyl)-3-(phenylaminocarbonyl hydrazinocarbonyl) pyridinium bromide or other cosmetically acceptable salts thereof,
(t) 1-(2-phenyl-2-oxoethyl)-3-(2-(acetoxy) ethylaminocarbonyl) pyridinium bromide or other cosmetically acceptable salts thereof,
(u) 1-(2-phenylamino-2-oxoethyl)-3-(phenyl sulfonyl hydrazino carbonyl) pyridinium chloride or other cosmetically acceptable salts thereof,
(v) 1-(2-phenylamino-2-oxoethyl)-3-((4-methylphenyl)sulfonyl hydrazinocarbonyl) pyridinium chloride or other cosmetically acceptable salts thereof,
(w) 1-(2-phenyl-2-oxoethyl)-3-(2-(benzoyloxy)ethyloxy carbonyl) pyridinium bromide or other cosmetically acceptable salts thereof,
(x) 1-(2-thien-2'-yl-2-oxoethyl)-3-(phenylcarbonyl hydrazino carbonyl) pyridinium bromide or other cosmetically acceptable salts thereof,
(y) 1-(2-ethoxy-2-oxoethyl)-3-((phenylmethyl)sulfonyl hydrazino carbonyl)pyridinium bromide or other cosmetically acceptable salts thereof,
(z)1-(2-phenyl-2-oxoethyl)-3-((phenylmethyl)sulfonyl hydrazino carbonyl)pyridinium bromide or other cosmetically acceptable salts thereof,
(aa) N, N' - bis [3-carbonyl-1-(2-furan-2'-yl-2-oxoethyl) pyridinium] hydrazine dibromide or other cosmetically acceptable salts thereof,
(ab) N,N'-bis [3-carbonyl -1- (2-thien-2'-yl-2-oxoethyl) pyridinium] hydrazine dichloride or other cosmetically acceptable salts thereof,
(ac) N,N'-bis-[3-carbonyl-1-(2-cyclopropylamino-2-oxoethyl) pyridinium] hydrazine dichloride or other cosmetically acceptable salts thereof,
(ad) 1-(2',4'-dichlorophenyl-2-oxoethyl)-3-(2-methoxyethyl aminocarbonyl)-pyridinium bromide or other cosmetically acceptable salts thereof,
(ae) 1-(2-thien-2'-yl-2-oxoethyl)-3-((2-methoxy ethyl) amino carbonyl)-5-bromo pyridinium chloride or other cosmetically acceptable salts thereof,
(af) 1-(2-thien-2'-yl-2-oxoethyl)-3-(methanesulfonyl hydrazino carbonyl) pyridinium chloride or other cosmetically acceptable salts thereof,
(ag) 1-(2-thien-2'yl-2-oxoethyl)-3-(2-(2-chloro-3-pyridoylhydrazinocarbonyl) - pyridinium chloride or other cosmetically acceptable salts thereof,
(ah) 1-(2-cyclopropylamino-2-oxoethyl)-3-(2-methoxyethylaminocarbonyl) -pyridinium chloride or other cosmetically acceptable salts thereof,
(ai) 1-(2-isopropylamino-2-oxoethyl)-3-(2-methylsulfonylhydrazinocarbonyl)-pyridinium chloride or other cosmetically acceptable salts thereof,
(aj) 1-(2-phenylamino-2-oxo ethyl)-3-({2-(1-oxo-3-cyclohexyl)-propyl}- hydrazinocarbonyl)-pyridinium bromide or other cosmetically acceptable salts thereof,
(ak) 1-(2-thien-2'-yl-2-oxoethyl)-3-[2-(benzoyloxy)ethylamino carbonyl]-pyridinium bromide or other cosmetically acceptable salts thereof,
(al) 1-(4-ethoxy-2, 4-dioxobutyl)-3-(2-(benzoxyloxy)ethylamino carbonyl)-pyridinium chloride or other cosmetically acceptable salts thereof,
(am)1-(2-thien-2'-yl-2-oxoethyl)-3-[1-oxo-1-(2-methoxy carbonyl) pyridyl] hydrazino pyridinium chloride or other cosmetically acceptable salts thereof,
(an)1-[1-(2-thien-2'-yl-2-oxoethyl)-5-aminocarbonyl-3-carbonyl pyridinium]-2-[1-(2-thien-2'-yl-2-oxoethyl )-3-carbonyl pyridinium] hydrazine dichloride or other cosmetically acceptable salts thereof,
(ao)1-(2-thien-2'-yl-2-oxoethyl)-3-(trifluoromethanesulfonyl hydrazino carbonyl) - pyridinium bromide or other cosmetically acceptable salts thereof,
(ap)1-[1-(2-thien-2'-yl-2-oxoethyl)-6-methyl-3-carbonyl pyridinium]-2-[1-(2-thien-2'-yl-2-oxoethyl )-3-carbonyl pyridinium ] hydrazine dichloride or other cosmetically acceptable salts thereof,
(aq) N,N'-bis[3-carbonyl-1-(2-(5-methyl-thien-2-yl)-2-oxoethyl) pyridinium] hydrazine dichloride or other cosmetically acceptable salts thereof,
(ar) N,N'-bis[3-carbonyl-1-(2-(5-chloro-thien-2-yl)-2-oxoethyl) pyridinium] hydrazine dichloride or other cosmetically acceptable salts thereof,
(as) 1-(2-thien-2'-yl-2-oxoethyl)-3-(methanesulfonyl hydrazino carbonyl )-6-methyl pyridinium bromide or other cosmetically acceptable salts thereof,
(at) N,N'-bis[3-carbonyl-1-(2-(4-nitro-thien-2-yl)-2-oxoethyl)pyridinium] hydrazine dichloride or other cosmetically acceptable salts thereof,

(au) 1-(2-phenylamino-2-oxoethyl)-3-(phenyl hydrazino carbonyl) pyridinium chloride or other cosmetically acceptable salts thereof,
(av) 1-(2-(4-nitro-thien-2-yl)-2-oxoethyl)-3-(methanesulfonyl hydrazino carbonyl ) pyridinium bromide or other cosmetically acceptable salts thereof,
(aw) 1-(2-(5-nitro-thien-2-yl)-2-oxoethyl)-3-(methanesulfonyl hydrazino carbonyl) pyridinium chloride or other cosmetically acceptable salts thereof,
(ax) 1-(2-(5-chloro-thien-2-yl)-2-oxoethyl)-3-(methanesulfonyl hydrazino carbonyl) pyridinium bromide or other cosmetically acceptable salts thereof,
(ay) 1-(2-thien-2'-yl-2-oxoethyl)-3-(ethoxycarbonyl hydrazino carbonyl) pyridinium bromide or other cosmetically acceptable salts thereof,
(az) 1-(2-thien-2'-yl-2-oxoethyl)-3-(isopropylsulfonyl hydrazino carbonyl) pyridinium bromide or other cosmetically acceptable salts thereof,
(ba) 1-(2-thien-2'-yl-2-oxoethyl)-3-(methanesulfonyl hydrazino carbonyl) -5-bromo pyridinium bromide or other cosmetically acceptable salts thereof,
(bb) 1-(2-(2-ethoxy carbonyl pyrrolidin-1-yl)-2-oxoethyl)-3-(methanesulfonyl hydrazino carbonyl) pyridinium chloride or other cosmetically acceptable salts thereof,
(bc) 1-(2-(5-methyl-thien-2-yl)-2-oxoethyl)-3-(methanesulfonyl hydrazino carbonyl) pyridinium chloride or other cosmetically acceptable salts thereof,
(bd) 1-(2-(4- carboethoxy -thiazolidin-3-yl)-2-oxoethyl) -3-(methanesulfonyl hydrazino carbonyl ) pyridinium chloride or other cosmetically acceptable salts thereof,
(be) 1-(2-(4-benzyl piperidin-1-yl)-2-oxoethyl)-3-(methanesulfonyl hydrazino carbonyl) pyridinium chloride or other cosmetically acceptable salts thereof,
(bf) N,N(-bis[3-carbonyl-1-(2-(2-ethoxycarbonyl pyrrolidin-1-yl)-2-oxoethyl) pyridinium] hydrazine dichloride or other cosmetically acceptable salts thereof,
(bg) 1-(2-phenylamino-2-oxoethyl)-4 -[2-(benzoyloxy) ethylamino carbonyl ] pyridinium chloride or other cosmetically acceptable salts thereof,
(bh) 1-(2-thien-2(-yl-2-oxoethyl)-3-(phenyl hydrazino carbonyl) pyridinium bromide or other cosmetically acceptable salts thereof,
(bi) 1-(2-thien-2(-yl-2-oxoethyl)-3-(p-methoxy phenyl sulfonyl hydrazino carbonyl) pyridinium bromide or other cosmetically acceptable salts thereof,
(bj) 1-(2-ethoxy-2-oxoethyl)-3-(phenyl aminocarbonyl hydrazino carbonyl ) pyridinium bromide or other cosmetically acceptable salts thereof,
(bk) 1-(2-ethoxy-2-oxoethyl)-3-(p-toluene sulfonyl hydrazino carbonyl) pyridinium bromide or other cosmetically acceptable salts thereof,
(bl) 1-(2-phenyl-2-oxoethyl)-3-(phenylamino carbonyl hydrazino carbonyl ) pyridinium bromide or other cosmetically acceptable salts thereof,
(bm) 1-(2-phenylamino-2-oxoethyl)-3-(benzyl sulfonyl hydrazino carbonyl) pyridiniumchloride or other cosmetically acceptable salts thereof,
(bn) 1-(2-phenyl-2-oxoethyl)-4-(methanesulfonyl hydrazino carbonyl ) pyridinium bromide or other cosmetically acceptable salts thereof,
(bo) 1-(2-phenyl-2-oxoethyl)-3-(phenyl hydrazino carbonyl ) pyridinium bromide or other cosmetically acceptable salts thereof,
(bp) 1 -(2-ethoxy-2-oxoethyl)-4-[2-(benzoyloxy) ethyl amino carbonyl ] pyridinium bromide or other cosmetically acceptable salts thereof,
(bq) 1-(2-ethoxy-2-oxoethyl)-3-(phenyl hydrazino carbonyl ) pyridinium bromide or other cosmetically acceptable salts thereof,
(br) 1-(2-phenyl-2-oxoethyl)-3-(p-methoxyphenyl sulfonyl hydrazino carbonyl ) pyridinium bromide or other cosmetically acceptable salts thereof,
(bs) 1-(2-phenyl-2-oxoethyl)- 4-[2-(benzoyloxy) ethyl amino carbonyl] pyridinium bromide or other cosmetically acceptable salts thereof,
(bt) 1-(2-ethoxy-2-oxoethyl)- 4-(p-methanesulfonyl hydrazino carbonyl ) pyridinium bromide or other cosmetically acceptable salts thereof,
(bu) 3-carbonylamino-1-(2-(2, 4-dichlorophenyl)-2-oxoethyl)-pyridinium bromide or other cosmetically acceptable salts thereof,
(bv) 3-(tetrahydrobenzothiazol-2-yl) aminocarbonyl -1-(2-(2, 4-dichlorophenyl)-2-oxoethyl)-pyridinium bromide or other cosmetically acceptable salts thereof, (bw) 1-(2-phenyl-2-oxoethyl)-3-((2-hydroxyethyl) aminocarbonyl) pyridinium bromide or other cosmetically acceptable salts thereof,
(bx) 3-carbonylamino-1- (2-thien-2'-yl-2-oxoethyl)-pyridinium bromide or other cosmetically acceptable salts

thereof,

(by) 1-(2-phenyl -2-oxoethyl)-3-((p-sulfonamidophenylene) aminocarbonyl) pyridinium bromide or other cosmetically acceptable salts thereof,

(bz) 1-(2-ethoxy-2-oxoethyl)-3-((2-hydroxyethyl) aminocarbonyl) pyridinium bromide or other cosmetically acceptable salts thereof,

(ca) 1-(2-phenyl-2-oxoethyl)-3-(isopropyloxycarbonyl) pyridinium bromide or other cosmetically acceptable salts thereof,

(cb) 1-(2-oxopropyl)-3-((2-hydroxyethyl) aminocarbonyl) pyridinium chloride or other cosmetically acceptable salts thereof,

(cc) 1-(2-thien-2'-yl-2-oxoethyl)-3-((2-hydroxyethyl) aminocarbonyl) pyridinium bromide or other cosmetically acceptable salts thereof,

(cd) 1-(2-(2,4 - dichlorophenyl-2-oxoethyl) -3- (isopropyloxycarbonyl) pyridinium bromide or other cosmetically acceptable salts thereof,

(ce) 1- (2-phenyl - 2- oxoethyl) - 3- ((4-methylthiazol - 2 -yl) aminocarbonyl) pyridinium bromide or other cosmetically acceptable salts thereof,

(cf) 1-(2- phenylamino -2- oxoethyl ) -3- (n-butyloxycarbonyl) pyridinium chloride or other cosmetically acceptable salts thereof,

(cg) 1- (2- phenylamino - 2- oxoethyl)- 3- ((2-hydroxyethyl) aminocarbonyl)-pyridinium chloride or other cosmetically acceptable salts thereof,

(ch) 1- (2- (2,4 - dichlorophenyl ) - 2-oxoethyl) - 3- (n - butoxycarbonyl) pyridinium bromide or other cosmetically acceptable salts thereof,

(ci) 1-(2 - (2, 4- dichlorophenyl) - 2-oxoethyl) - 3- (n-butylamino-carbonyl) pyridinium bromide or other cosmetically acceptable salts thereof,

(cj) 1-(2-phenyl-2-oxoethyl)-3-(1-phenyl-1-oxomethyl) pyridinium bromide or other cosmetically acceptable salts thereof and

(ck) 1- (2 phenyl - 2-oxoethyl) - 3- (methoxycarbonyl) pyridinium bromide or other cosmetically acceptable salts thereof.

**23.** The method as claimed in claim 19, wherein said compound is selected from the group consisting of the following compounds:

(a) N,N'-bis[3-carbonyl-1-(2-thien -2'- yl -2-oxoethyl) -3-pyridinium] hydrazine dibromide or other cosmetically acceptable salts thereof,

(b) 1-(2-ethoxy -2-oxoethyl) -3-(2-(2-pyridyl)hydrazinocarbonyl) pyridinium bromide or other cosmetically acceptable salts thereof,

(c) 1-(2-ethoxy -2-oxoethyl) -3-(2-(benzoyloxy) ethylamino carbonyl) pyridinium bromide or other cosmetically acceptable salts thereof,

(d)N,N'-bis[3-carbonyl-1-(2-phenyl-2-oxoethyl)pyridinium]hydrazine dibromide or other cosmetically acceptable salts thereof,

(e)1-(2-phenyl-2-oxoethyl)-3-(hydrazinocarbonyl)pyridinium bromide or other cosmetically acceptable salts thereof,

(f) 1-(2-thien-2'-yl-2-oxoethyl) -3-(methanesulfonyl hydrazinocarbonyl) pyridinium bromide or other cosmetically acceptable salts thereof,

(g) N,N'-bis[3-carbonyl-1-(2-(2',4'-dichlorophenyl)-2-oxoethyl) pyridinium] hydrazine dibromide or other cosmetically acceptable salts thereof,

(h) 1-(2-phenyl -2-oxoethyl) -3-(methanesulfonyl hydrazinocarbonyl) pyridinium bromide or other cosmetically acceptable salts thereof,

(i) 1-(2-ethoxy -2-oxoethyl) -3-(methanesulfonyl hydrazinocarbonyl) pyridinium bromide or other cosmetically acceptable salts thereof,

(j)1-(2-phenyl-2-oxoethyl)-3-(phenylsulfonylhydrazino carbonyl) pyridinium bromide or other cosmetically acceptable salts thereof,

(k)1-(2-phenyl-2-oxoethyl)-2-chloro-3-(phenylsulfonyl hydrazino carbonyl) pyridinium bromide or other cosmetically acceptable salts thereof,

(l) 1-(2-thien-2'-yl -2-oxoethyl)-4-(2-(benzoyloxy) ethyl aminocarbonyl) pyridinium bromide or other cosmetically acceptable salts thereof,

(m)1- (2- (2,'4'- dichlorophenyl) -2-oxoethyl) -3-(2- (benzoyloxy) ethylaminocarbonyl) pyridinium bromide or other cosmetically acceptable salts thereof,

(n) 1-(2-phenyl -2-oxoethyl) -3-(2-(acetoxy) ethyloxy) carbonyl) pyridinium bromide or other cosmetically acceptable salts thereof,
(o) 1-(2-ethoxy -2-oxoethyl) -3-(2-(benzoyloxy) ethyloxy carbonyl) pyridinium bromide or other cosmetically acceptable salts thereof,
(p) 1-(2-phenylamino-2-oxoethyl)-4-(phenylsulfonyl hydrazino carbonyl)pyridinium chloride or other cosmetically acceptable salts thereof,
(q) 1-(2-(2,'4'-dichlorophenyl)-2-oxoethyl)-3- (2(methoxy) ethyloxycarbonyl) pyridinium bromide or other cosmetically acceptable salts thereof,
(r) 1-(2-phenylamino-2-oxoethyl)-3-((benzoyloxy) ethylaminocarbonyl) pyridinium chloride or other cosmetically acceptable salts thereof,
(s) 1-(2-thien-2'-yl-2-oxoethyl)-3-(phenylaminocarbonyl hydrazinocarbonyl) pyridinium bromide or other cosmetically acceptable salts thereof,
(t) 1-(2-phenyl-2-oxoethyl)-3-(2-(acetoxy) ethylaminocarbonyl) pyridinium bromide or other cosmetically acceptable salts thereof,
(u) 1-(2-phenylamino-2-oxoethyl)-3-(phenyl sulfonyl hydrazino carbonyl) pyridinium chloride or other cosmetically acceptable salts thereof,
(v) 1-(2-phenylamino-2-oxoethyl)-3-((4-methylphenyl)sulfonyl hydrazinocarbonyl) pyridinium chloride or other cosmetically acceptable salts thereof,
(w) 1-(2-phenyl-2-oxoethyl)-3-(2-(benzoyloxy)ethyloxy carbonyl) pyridinium bromide or other cosmetically acceptable salts thereof,
(x) 1-(2-thien-2'-yl-2-oxoethyl)-3-(phenylcarbonyl hydrazino carbonyl) pyridinium bromide or other cosmetically acceptable salts thereof,
(y) 1-(2-ethoxy-2-oxoethyl)-3-((phenylmethyl)sulfonyl hydrazino carbonyl)pyridinium bromide or other cosmetically acceptable salts thereof,
(z)1-(2-phenyl-2-oxoethyl)-3-((phenylmethyl)sulfonyl hydrazino carbonyl)pyridinium bromide or other cosmetically acceptable salts thereof,
(aa) N, N' - bis [3-carbonyl-1-(2-furan-2'-yl-2-oxoethyl) pyridinium] hydrazine dibromide or other cosmetically acceptable salts thereof,
(ab) N,N'-bis [3-carbonyl -1- (2-thien-2'-yl-2-oxoethyl) pyridinium] hydrazine dichloride or other cosmetically acceptable salts thereof,
(ac) N,N'-bis-[3-carbonyl-1-(2-cyclopropylamino-2-oxoethyl) pyridinium] hydrazine dichloride or other cosmetically acceptable salts thereof,
(ad) 1-(2',4'-dichlorophenyl-2-oxoethyl)-3-(2-methoxyethyl aminocarbonyl)-pyridinium bromide or other cosmetically acceptable salts thereof,
(ae) 1-(2-thien-2'-yl-2-oxoethyl)-3-((2-methoxy ethyl) amino carbonyl)-5-bromo pyridinium chloride or other cosmetically acceptable salts thereof,
(af) 1-(2-thien-2'-yl-2-oxoethyl)-3-(methanesulfonyl hydrazino carbonyl) pyridinium chloride or other cosmetically acceptable salts thereof,
(ag) 1-(2-thien-2'yl-2-oxoethyl)-3-(2-(2-chloro-3-pyridoylhydrazinocarbonyl)-pyridinium chloride or other cosmetically acceptable salts thereof,
(ah) 1-(2-cyclopropylamino-2-oxoethyl)-3-(2-methoxyethylaminocarbonyl) -pyridinium chloride or other cosmetically acceptable salts thereof,
(ai) 1-(2-isopropylamino-2-oxoethyl)-3-(2-methylsulfonylhydrazinocarbonyl)-pyridinium chloride or other cosmetically acceptable salts thereof,
(aj) 1-(2-phenylamino-2-oxo ethyl)-3-({2-(1-oxo-3-cyclohexyl)-propyl} - hydrazinocarbonyl)-pyridinium bromide or other cosmetically acceptable salts thereof,
(ak) 1-(2-thien-2'-yl-2-oxoethyl)-3-[2-(benzoyloxy)ethylamino carbonyl]-pyridinium bromide or other cosmetically acceptable salts thereof,
(al) 1-(4-ethoxy-2, 4-dioxobutyl)-3-(2-(benzoxyloxy)ethylamino carbonyl)-pyridinium chloride or other cosmetically acceptable salts thereof,
(am)1-(2-thien-2'-yl-2-oxoethyl)-3-[1-oxo-1-(2-methoxy carbonyl) pyridyl] hydrazino pyridinium chloride or other cosmetically acceptable salts thereof,
(an)1-[1-(2-thien-2'-yl-2-oxoethyl)-5-aminocarbonyl-3-carbonyl pyridinium]-2-[1-(2-thien-2'-yl-2-oxoethyl )-3-carbonyl pyridinium] hydrazine dichloride or other cosmetically acceptable salts thereof,
(ao)1-(2-thien-2'-yl-2-oxoethyl)-3-(trifluoromethanesulfonyl hydrazino carbonyl) - pyridinium bromide or other cosmetically acceptable salts thereof,
(ap)1-[1-(2-thien-2'-yl-2-oxoethyl)-6-methyl-3-carbonyl pyridinium]-2-[1-(2-thien-2'-yl-2-oxoethyl )-3-carbonyl pyridinium ] hydrazine dichloride or other cosmetically acceptable salts thereof,
(aq) N,N'-bis[3-carbonyl-1-(2-(5-methyl-thien-2-yl)-2-oxoethyl) pyridinium] hydrazine dichloride or other cosmetically acceptable salts thereof,

(ar) N,N'-bis[3-carbonyl-1-(2-(5-chloro-thien-2-yl)-2-oxoethyl) pyridinium] hydrazine dichloride or other cosmetically acceptable salts thereof,

(as) 1-(2-thien-2'-yl-2-oxoethyl)-3-(methanesulfonyl hydrazino carbonyl )-6-methyl pyridinium bromide or other cosmetically acceptable salts thereof,

(at) N,N'-bis[3-carbonyl-1-(2-(4-nitro-thien-2-yl)-2-oxoethyl)pyridinium] hydrazine dichloride or other cosmetically acceptable salts thereof,

(au) 1-(2-phenylamino-2-oxoethyl)-3-(phenyl hydrazino carbonyl) pyridinium chloride or other cosmetically acceptable salts thereof,

(av) 1-(2-(4-nitro-thien-2-yl)-2-oxoethyl)-3-(methanesulfonyl hydrazino carbonyl ) pyridinium bromide or other cosmetically acceptable salts thereof,

(aw) 1-(2-(5-nitro-thien-2-yl)-2-oxoethyl)-3-(methanesulfonyl hydrazino carbonyl) pyridinium chloride or other cosmetically acceptable salts thereof,

(ax) 1-(2-(5-chloro-thien-2-yl)-2-oxoethyl)-3-(methanesulfonyl hydrazino carbonyl) pyridinium bromide or other cosmetically acceptable salts thereof,

(ay) 1-(2-thien-2'-yl-2-oxoethyl)-3-(ethoxycarbonyl hydrazino carbonyl) pyridinium bromide or other cosmetically acceptable salts thereof,

(az) 1-(2-thien-2'-yl-2-oxoethyl)-3-(isopropylsulfonyl hydrazino carbonyl) pyridinium bromide or other cosmetically acceptable salts thereof,

(ba) 1-(2-thien-2'-yl-2-oxoethyl)-3-(methanesulfonyl hydrazino carbonyl) -5-bromo pyridinium bromide or other cosmetically acceptable salts thereof,

(bb) 1-(2-(2-ethoxy carbonyl pyrrolidin-1-yl)-2-oxoethyl)-3-(methanesulfonyl hydrazino carbonyl) pyridinium chloride or other cosmetically acceptable salts thereof,

(bc) 1-(2-(5-methyl-thien-2-yl)-2-oxoethyl)-3-(methanesulfonyl hydrazino carbonyl) pyridinium chloride or other cosmetically acceptable salts thereof, (bd) 1-(2-(4- carboethoxy -thiazolidin-3-yl)-2-oxoethyl) -3-(methanesulfonyl hydrazino carbonyl ) pyridinium chloride or other cosmetically acceptable salts thereof,

(be) 1-(2-(4-benzyl piperidin-1-yl)-2-oxoethyl)-3-(methanesulfonyl hydrazino carbonyl) pyridinium chloride or other cosmetically acceptable salts thereof,

(bf) N,N(-bis[3-carbonyl-1-(2-(2-ethoxycarbonyl pyrrolidin-1-yl)-2-oxoethyl) pyridinium] hydrazine dichloride or other cosmetically acceptable salts thereof,

(bg) 1-(2-phenylamino-2-oxoethyl)-4 -[ 2-(benzoyloxy) ethylamino carbonyl ] pyridinium chloride or other cosmetically acceptable salts thereof,

(bh) 1-(2-thien-2(-yl-2-oxoethyl)-3-(phenyl hydrazino carbonyl) pyridinium bromide or other cosmetically acceptable salts thereof,

(bi) 1-(2-thien-2(-yl-2-oxoethyl)-3-(p-methoxy phenyl sulfonyl hydrazino carbonyl) pyridinium bromide or other cosmetically acceptable salts thereof,

(bj) 1-(2-ethoxy-2-oxoethyl)-3-(phenyl aminocarbonyl hydrazino carbonyl ) pyridinium bromide or other cosmetically acceptable salts thereof,

(bk) 1-(2-ethoxy-2-oxoethyl)-3-(p-toluene sulfonyl hydrazino carbonyl) pyridinium bromide or other cosmetically acceptable salts thereof,

(bl) 1-(2-phenyl-2-oxoethyl)-3-(phenylamino carbonyl hydrazino carbonyl ) pyridinium bromide or other cosmetically acceptable salts thereof,

(bm) 1-(2-phenylamino-2-oxoethyl)-3-(benzyl sulfonyl hydrazino carbonyl) pyridiniumchloride or other cosmetically acceptable salts thereof,

(bn) 1-(2-phenyl-2-oxoethyl)-4-(methanesulfonyl hydrazino carbonyl ) pyridinium bromide or other cosmetically acceptable salts thereof,

(bo) 1-(2-phenyl-2-oxoethyl)-3-(phenyl hydrazino carbonyl ) pyridinium bromide or other cosmetically acceptable salts thereof,

(bp) 1 -(2-ethoxy-2-oxoethyl)-4-[2-(benzoyloxy) ethyl amino carbonyl ] pyridinium bromide or other cosmetically acceptable salts thereof,

(bq) 1-(2-ethoxy-2-oxoethyl)-3-(phenyl hydrazino carbonyl ) pyridinium bromide or other cosmetically acceptable salts thereof,

(br) 1-(2-phenyl-2-oxoethyl)-3-(p-methoxyphenyl sulfonyl hydrazino carbonyl ) pyridinium bromide or other cosmetically acceptable salts thereof,

(bs) 1-(2-phenyl-2-oxoethyl)- 4-[2-(benzoyloxy) ethyl amino carbonyl] pyridinium bromide or other cosmetically acceptable salts thereof,

(bt) 1-(2-ethoxy-2-oxoethyl)- 4-(p-methanesulfonyl hydrazino carbonyl ) pyridinium bromide or other cosmetically acceptable salts thereof,

(bu) 3-carbonylamino-1- (2-(2, 4-dichlorophenyl)-2-oxoethyl)-pyridinium bromide or other cosmetically acceptable salts thereof,
(bv) 3-(tetrahydrobenzothiazol-2-yl) aminocarbonyl -1-(2-(2, 4-dichlorophenyl)-2-oxoethyl)-pyridinium bromide or other cosmetically acceptable salts thereof,
(bw) 1-(2-phenyl-2-oxoethyl)-3-((2-hydroxyethyl) aminocarbonyl) pyridinium bromide or other cosmetically acceptable salts thereof,
(bx) 3-carbonylamino-1- (2-thien-2'-yl-2-oxoethyl)-pyridinium bromide or other cosmetically acceptable salts thereof,
(by) 1-(2-phenyl-2-oxoethyl)-3-((p-sulfonamidophenylene) aminocarbonyl) pyridinium bromide or other cosmetically acceptable salts thereof,
(bz) 1-(2-ethoxy-2-oxoethyl)-3-((2-hydroxyethyl) aminocarbonyl) pyridinium bromide or other cosmetically acceptable salts thereof,
(ca) 1-(2-phenyl-2-oxoethyl)-3-(isopropyloxycarbonyl) pyridinium bromide or other cosmetically acceptable salts thereof,
(cb) 1-(2-oxopropyl)-3-((2-hydroxyethyl) aminocarbonyl) pyridinium chloride or other cosmetically acceptable salts thereof,
(cc) 1-(2-thien-2'-yl-2-oxoethyl)-3-((2-hydroxyethyl) aminocarbonyl) pyridinium bromide or other cosmetically acceptable salts thereof,
(cd) 1-(2-(2,4 - dichlorophenyl-2-oxoethyl) -3- (isopropyloxycarbonyl) pyridinium bromide or other cosmetically acceptable salts thereof,
(ce) 1- (2-phenyl - 2- oxoethyl) - 3- ((4-methylthiazol - 2 -yl) aminocarbonyl) pyridinium bromide or other cosmetically acceptable salts thereof,
(cf) 1-(2- phenylamino -2- oxoethyl ) -3- (n-butyloxycarbonyl) pyridinium chloride or other cosmetically acceptable salts thereof,
(cg) 1- (2- phenylamino - 2- oxoethyl)- 3- ((2-hydroxyethyl) aminocarbonyl) - pyridinium chloride or other cosmetically acceptable salts thereof,
(ch) 1- (2- (2,4 - dichlorophenyl )- 2-oxoethyl) - 3- (n - butoxycarbonyl) pyridinium bromide or other cosmetically acceptable salts thereof,
(ci) 1-(2 - (2, 4 - dichlorophenyl) - 2-oxoethyl) - 3- (n-butylamino-carbonyl) pyridinium bromide or other cosmetically acceptable salts thereof,
(cj) 1-(2-phenyl-2-oxoethyl)-3-(1-phenyl-1-oxomethyl) pyridinium bromide or other cosmetically acceptable salts thereof and
(ck) 1- (2 phenyl - 2-oxoethyl) - 3- (methoxycarbonyl) pyridinium bromide or other cosmetically acceptable salts thereof

**24.** The method as claimed in claim 20, wherein said compound is selected from the group consisting of the following compounds:

(a) N,N'-bis[3-carbonyl-1-(2-thien -2'- yl -2-oxoethyl) -3-pyridinium] hydrazine dibromide or other cosmetically acceptable salts thereof,
(b) 1-(2-ethoxy -2-oxoethyl) -3-(2-(2-pyridyl)hydrazinocarbonyl) pyridinium bromide or other cosmetically acceptable salts thereof,
(c) 1-(2-ethoxy -2-oxoethyl) -3-(2-(benzoyloxy) ethylamino carbonyl) pyridinium bromide or other cosmetically acceptable salts thereof,
(d)N,N-bis[3-carbonyl-1-(2-phenyl-2-oxoethyl)pyridinium]hydrazine dibromide or other cosmetically acceptable salts thereof,
(e)1-(2-phenyl-2-oxoethyl)-3-(hydrazinocarbonyl)pyridinium bromide or other cosmetically acceptable salts thereof,
(f) 1-(2-thien -2'-yl -2-oxoethyl) -3-(methanesulfonyl hydrazinocarbonyl) pyridinium bromide or other cosmetically acceptable salts thereof,
(g) N,N'-bis[3-carbonyl-1-(2-(2',4'-dichlorophenyl)-2-oxoethyl) pyridinium] hydrazine dibromide or other cosmetically acceptable salts thereof,
(h) 1-(2-phenyl -2-oxoethyl) -3-(methanesulfonyl hydrazinocarbonyl) pyridinium bromide or other cosmetically acceptable salts thereof,
(i) 1-(2-ethoxy -2-oxoethyl) -3-(methanesulfonyl hydrazinocarbonyl) pyridinium bromide or other cosmetically acceptable salts thereof,
(j)1-(2-phenyl-2-oxoethyl)-3-(phenylsulfonylhydrazino carbonyl) pyridinium bromide or other cosmetically acceptable salts thereof,

(k)1-(2-phenyl-2-oxoethyl)-2-chloro-3-(phenylsulfonyl hydrazino carbonyl) pyridinium bromide or other cosmetically acceptable salts thereof,
(l) 1-(2-thien -2'-yl-2-oxoethyl)-4-(2-(benzoyloxy) ethyl aminocarbonyl) pyridinium bromide or other cosmetically acceptable salts thereof,
(m)1- (2- (2,'4'- dichlorophenyl) -2-oxoethyl) -3-(2- (benzoyloxy) ethylaminocarbonyl) pyridinium bromide or other cosmetically acceptable salts thereof,
(n) 1-(2-phenyl -2-oxoethyl) -3-(2-(acetoxy) ethyloxy) carbonyl) pyridinium bromide or other cosmetically acceptable salts thereof,
(o) 1-(2-ethoxy -2-oxoethyl) -3-(2-(benzoyloxy) ethyloxy carbonyl) pyridinium bromide or other cosmetically acceptable salts thereof,
(p) 1-(2-phenylamino-2-oxoethyl)-4-(phenylsulfonyl hydrazino carbonyl)pyridinium chloride or other cosmetically acceptable salts thereof,
(q) 1-(2-(2,'4'-dichlorophenyl)-2-oxoethyl)-3- (2(methoxy) ethyloxycarbonyl) pyridinium bromide or other cosmetically acceptable salts thereof,
(r) 1-(2-phenylamino-2-oxoethyl)-3-((benzoyloxy) ethylaminocarbonyl) pyridinium chloride or other cosmetically acceptable salts thereof,
(s) 1-(2-thien-2'-yl-2-oxoethyl)-3-(phenylaminocarbonyl hydrazinocarbonyl) pyridinium bromide or other cosmetically acceptable salts thereof,
(t) 1-(2-phenyl-2-oxoethyl)-3-(2-(acetoxy) ethylaminocarbonyl) pyridinium bromide or other cosmetically acceptable salts thereof,
(u) 1-(2-phenylamino-2-oxoethyl)-3-(phenyl sulfonyl hydrazino carbonyl) pyridinium chloride or other cosmetically acceptable salts thereof,
(v) 1-(2-phenylamino-2-oxoethyl)-3-((4-methylphenyl)sulfonyl hydrazinocarbonyl) pyridinium chloride or other cosmetically acceptable salts thereof,
(w) 1-(2-phenyl-2-oxoethyl)-3-(2-(benzoyloxy)ethyloxy carbonyl) pyridinium bromide or other cosmetically acceptable salts thereof,
(x) 1-(2-thien-2'-yl-2-oxoethyl)-3-(phenylcarbonyl hydrazino carbonyl) pyridinium bromide or other cosmetically acceptable salts thereof,
(y) 1-(2-ethoxy-2-oxoethyl)-3-((phenylmethyl)sulfonyl hydrazino carbonyl)pyridinium bromide or other cosmetically acceptable salts thereof,
(z)1-(2-phenyl-2-oxoethyl)-3-((phenylmethyl)sulfonyl hydrazino carbonyl)pyridinium bromide or other cosmetically acceptable salts thereof,
(aa) N, N' - bis[3-carbonyl-1-(2-furan-2'-yl-2-oxoethyl) pyridinium] hydrazine dibromide or other cosmetically acceptable salts thereof,
(ab) N,N'-bis [3-carbonyl -1- (2-thien-2'-yl-2-oxoethyl) pyridinium] hydrazine dichloride or other cosmetically acceptable salts thereof,
(ac) N,N'-bis-[3-carbonyl-1-(2-cyclopropylamino-2-oxoethyl) pyridinium] hydrazine dichloride or other cosmetically acceptable salts thereof,
(ad) 1-(2',4'-dichlorophenyl-2-oxoethyl)-3-(2-methoxyethyl aminocarbonyl)-pyridinium bromide or other cosmetically acceptable salts thereof,
(ae) 1-(2-thien-2'-yl-2-oxoethyl)-3-((2-methoxy ethyl) amino carbonyl)-5-bromo pyridinium chloride or other cosmetically acceptable salts thereof,
(af) 1-(2-thien-2'-yl-2-oxoethyl)-3-(methanesulfonyl hydrazino carbonyl) pyridinium chloride or other cosmetically acceptable salts thereof,
(ag) 1-(2-thien-2'yl-2-oxoethyl)-3-(2-(2-chloro-3-pyridoylhydrazinocarbonyl)-pyridinium chloride or other cosmetically acceptable salts thereof,
(ah) 1-(2-cyclopropylamino-2-oxoethyl)-3-(2-methoxyethylaminocarbonyl) -pyridinium chloride or other cosmetically acceptable salts thereof,
(ai) 1-(2-isopropylamino-2-oxoethyl)-3-(2-methylsulfonylhydrazinocarbonyl)-pyridinium chloride or other cosmetically acceptable salts thereof,
(aj) 1-(2-phenylamino-2-oxo ethyl)-3-({2-(1-oxo-3-cyclohexyl)-propyl} - hydrazinocarbonyl)-pyridinium bromide or other cosmetically acceptable salts thereof, (ak) 1 -(2-thien-2'-yl-2-oxoethyl)-3-[2-(benzoyloxy)ethylamino carbonyl]-pyridinium bromide or other cosmetically acceptable salts thereof,
(al) 1-(4-ethoxy-2,4-dioxobutyl)-3-(2-(benzoxyloxy)ethylamino carbonyl)-pyridinium chloride or other cosmetically acceptable salts thereof,
(am)1-(2-thien-2'-yl-2-oxoethyl)-3-[1-oxo-1-(2-methoxy carbonyl) pyridyl] hydrazino pyridinium chloride or other cosmetically acceptable salts thereof,
(an)1-[1-(2-thien-2'-yl-2-oxoethyl)-5-aminocarbonyl-3-carbonyl pyridinium]-2-[1-(2-thien-2'-yl-2-oxoethyl )-

3-carbonyl pyridinium] hydrazine dichloride or other cosmetically acceptable salts thereof,
(ao)1-(2-thien-2'-yl-2-oxoethyl)-3-(trifluoromethanesulfonyl hydrazino carbonyl) - pyridinium bromide or other cosmetically acceptable salts thereof,
(ap)1-[1-(2-thien-2'-yl-2-oxoethyl)-6-methyl-3-carbonyl pyridinium]-2-[1-(2-thien-2'-yl-2-oxoethyl )-3-carbonyl pyridinium ] hydrazine dichloride or other cosmetically acceptable salts thereof,
(aq) N,N'-bis[3-carbonyl-1-(2-(5-methyl-thien-2-yl)-2-oxoethyl) pyridinium] hydrazine dichloride or other cosmetically acceptable salts thereof,
(ar) N,N'-bis[3-carbonyl-1-(2-(5-chloro-thien-2-yl)-2-oxoethyl) pyridinium] hydrazine dichloride or other cosmetically acceptable salts thereof,
(as) 1-(2-thien-2'-yl-2-oxoethyl)-3-(methanesulfonyl hydrazino carbonyl )-6-methyl pyridinium bromide or other cosmetically acceptable salts thereof,
(at) N,N'-bis[3-carbonyl-1-(2-(4-nitro-thien-2-yl)-2-oxoethyl)pyridinium] hydrazine dichloride or other cosmetically acceptable salts thereof,
(au) 1-(2-phenylamino-2-oxoethyl)-3-(phenyl hydrazino carbonyl) pyridinium chloride or other cosmetically acceptable salts thereof,
(av) 1-(2-(4-nitro-thien-2-yl)-2-oxoethyl)-3-(methanesulfonyl hydrazino carbonyl ) pyridinium bromide or other cosmetically acceptable salts thereof,
(aw) 1-(2-(5-nitro-thien-2-yl)-2-oxoethyl)-3-(methanesulfonyl hydrazino carbonyl) pyridinium chloride or other cosmetically acceptable salts thereof,
(ax) 1-(2-(5-chloro-thien-2-yl)-2-oxoethyl)-3-(methanesulfonyl hydrazino carbonyl) pyridinium bromide or other cosmetically acceptable salts thereof,
(ay) 1-(2-thien-2'-yl-2-oxoethyl)-3-(ethoxycarbonyl hydrazino carbonyl) pyridinium bromide or other cosmetically acceptable salts thereof,
(az) 1-(2-thien-2'-yl-2-oxoethyl)-3-(isopropylsulfonyl hydrazino carbonyl) pyridinium bromide or other cosmetically acceptable salts thereof,
(ba) 1-(2-thien-2'-yl-2-oxoethyl)-3-(methanesulfonyl hydrazino carbonyl) -5-bromo pyridinium bromide or other cosmetically acceptable salts thereof,
(bb) 1-(2-(2-ethoxy carbonyl pyrrolidin-1-yl)-2-oxoethyl)-3-(methanesulfonyl hydrazino carbonyl) pyridinium chloride or other cosmetically acceptable salts thereof,
(bc) 1-(2-(5-methyl-thien-2-yl)-2-oxoethyl)-3-(methanesulfonyl hydrazino carbonyl) pyridinium chloride or other cosmetically acceptable salts thereof,
(bd) 1-(2-(4- carboethoxy -thiazolidin-3-yl)-2-oxoethyl) -3-(methanesulfonyl hydrazino carbonyl ) pyridinium chloride or other cosmetically acceptable salts thereof,
(be) 1-(2-(4-benzyl piperidin-1-yl)-2-oxoethyl)-3-(methanesulfonyl hydrazino carbonyl) pyridinium chloride or other cosmetically acceptable salts thereof,
(bf) N,N(-bis[3-carbonyl-1-(2-(2-ethoxycarbonyl pyrrolidin-1-yl)-2-oxoethyl) pyridinium] hydrazine dichloride or other cosmetically acceptable salts thereof,
(bg) 1-(2-phenylamino-2-oxoethyl)-4-[ 2-(benzoyloxy) ethylamino carbonyl ] pyridinium chloride or other cosmetically acceptable salts thereof,
(bh) 1-(2-thien-2(-yl-2-oxoethyl)-3-(phenyl hydrazino carbonyl) pyridinium bromide or other cosmetically acceptable salts thereof,
(bi) 1-(2-thien-2(-yl-2-oxoethyl)-3-(p-methoxy phenyl sulfonyl hydrazino carbonyl) pyridinium bromide or other cosmetically acceptable salts thereof,
(bj) 1-(2-ethoxy-2-oxoethyl)-3-(phenyl aminocarbonyl hydrazino carbonyl ) pyridinium bromide or other cosmetically acceptable salts thereof,
(bk) 1-(2-ethoxy-2-oxoethyl)-3-(p-toluene sulfonyl hydrazino carbonyl) pyridinium bromide or other cosmetically acceptable salts thereof,
(bl) 1-(2-phenyl-2-oxoethyl)-3-(phenylamino carbonyl hydrazino carbonyl) pyridinium bromide or other cosmetically acceptable salts thereof,
(bm) 1-(2-phenylamino-2-oxoethyl)-3-(benzyl sulfonyl hydrazino carbonyl) pyridiniumchloride or other cosmetically acceptable salts thereof,
(bn) 1-(2-phenyl-2-oxoethyl)-4-(methanesulfonyl hydrazino carbonyl ) pyridinium bromide or other cosmetically acceptable salts thereof,
(bo) 1-(2-phenyl-2-oxoethyl)-3-(phenyl hydrazino carbonyl ) pyridinium bromide or other cosmetically acceptable salts thereof,
(bp) 1 -(2-ethoxy-2-oxoethyl)-4-[2-(benzoyloxy) ethyl amino carbonyl ] pyridinium bromide or other cosmetically acceptable salts thereof,
(bq) 1-(2-ethoxy-2-oxoethyl)-3-(phenyl hydrazino carbonyl ) pyridinium bromide or other cosmetically acceptable salts thereof,

(br) 1-(2-phenyl-2-oxoethyl)-3-(p-methoxyphenyl sulfonyl hydrazino carbonyl ) pyridinium bromide or other cosmetically acceptable salts thereof,

(bs) 1-(2-phenyl-2-oxoethyl)- 4-[2-(benzoyloxy) ethyl amino carbonyl] pyridinium bromide or other cosmetically acceptable salts thereof,

(bt) 1-(2-ethoxy-2-oxoethyl)- 4-(p-methanesulfonyl hydrazino carbonyl ) pyridinium bromide or other cosmetically acceptable salts thereof,

(bu) 3-carbonylamino-1- (2-(2, 4-dichlorophenyl)-2-oxoethyl)-pyridinium bromide or other cosmetically acceptable salts thereof,

(bv) 3-(tetrahydrobenzothiazol-2-yl) aminocarbonyl -1-(2-(2, 4-dichlorophenyl)-2-oxoethyl)-pyridinium bromide or other cosmetically acceptable salts thereof,

(bw) 1-(2-phenyl-2-oxoethyl)-3-((2-hydroxyethyl) aminocarbonyl) pyridinium bromide or other cosmetically acceptable salts thereof,

(bx) 3-carbonylamino-1-(2-thien-2'-yl-2-oxoethyl)-pyridinium bromide or other cosmetically acceptable salts thereof,

(by) 1-(2-phenyl -2-oxoethyl)-3-((p-sulfonamidophenylene) aminocarbonyl) pyridinium bromide or other cosmetically acceptable salts thereof,

(bz) 1-(2-ethoxy-2-oxoethyl)-3-((2-hydroxyethyl) aminocarbonyl) pyridinium bromide or other cosmetically acceptable salts thereof,

(ca) 1-(2-phenyl-2-oxoethyl)-3-(isopropyloxycarbonyl) pyridinium bromide or other cosmetically acceptable salts thereof,

(cb) 1-(2-oxopropyl)-3-((2-hydroxyethyl) aminocarbonyl) pyridinium chloride or other cosmetically acceptable salts thereof,

(cc) 1-(2-thien-2'-yl-2-oxoethyl)-3-((2-hydroxyethyl) aminocarbonyl) pyridinium bromide or other cosmetically acceptable salts thereof,

(cd) 1-(2-(2,4 - dichlorophenyl-2-oxoethyl) -3- (isopropyloxycarbonyl) pyridinium bromide or other cosmetically acceptable salts thereof,

(ce) 1- (2-phenyl - 2- oxoethyl) - 3- ((4-methylthiazol - 2 -yl) aminocarbonyl) pyridinium bromide or other cosmetically acceptable salts thereof,

(cf) 1-(2- phenylamino -2- oxoethyl ) -3- (n-butyloxycarbonyl) pyridinium chloride or other cosmetically acceptable salts thereof,

(cg) 1- (2- phenylamino - 2- oxoethyl)- 3- ((2-hydroxyethyl) aminocarbonyl) - pyridinium chloride or other cosmetically acceptable salts thereof,

(ch) 1- (2- (2,4 - dichlorophenyl ) - 2-oxoethyl) - 3- (n - butoxycarbonyl) pyridinium bromide or other cosmetically acceptable salts thereof,

(ci) 1-(2 - (2, 4 - dichlorophenyl) - 2-oxoethyl) - 3- (n-butylamino-carbonyl) pyridinium bromide or other cosmetically acceptable salts thereof,

(cj) 1-(2-phenyl-2-oxoethyl)-3-(1-phenyl-1-oxomethyl) pyridinium bromide or other cosmetically acceptable salts thereof and

(ck) 1- (2 phenyl - 2-oxoethyl) - 3- (methoxycarbonyl) pyridinium bromide or other cosmetically acceptable salts thereof

**25.** The method as claimed in claim 21, wherein said compound is selected from the group consisting of the following compounds:

(a) N,N'-bis[3-carbonyl-1-(2-thien -2'- yl -2-oxoethyl) -3-pyridinium] hydrazine dibromide or other cosmetically acceptable salts thereof,

(b) 1-(2-ethoxy -2-oxoethyl) -3-(2-(2-pyridyl)hydrazinocarbonyl) pyridinium bromide or other cosmetically acceptable salts thereof,

(c) 1-(2-ethoxy -2-oxoethyl) -3-(2-(benzoyloxy) ethylamino carbonyl) pyridinium bromide or other cosmetically acceptable salts thereof,

(d)N,N'-bis[3-carbonyl-1-(2-phenyl-2-oxoethyl)pyridinium]hydrazine dibromide or other cosmetically acceptable salts thereof,

(e)1-(2-phenyl-2-oxoethyl)-3-(hydrazinocarbonyl)pyridinium bromide or other cosmetically acceptable salts thereof,

(f) 1-(2-thien-2'-yl -2-oxoethyl) -3-(methanesulfonyl hydrazinocarbonyl) pyridinium bromide or other cosmetically acceptable salts thereof,

(g) N,N'-bis[3-carbonyl-1-(2-(2',4'-dichlorophenyl)-2-oxoethyl) pyridinium] hydrazine dibromide or other cosmetically acceptable salts thereof,
(h) 1-(2-phenyl -2-oxoethyl) -3-(methanesulfonyl hydrazinocarbonyl) pyridinium bromide or other cosmetically acceptable salts thereof,
(i) 1-(2-ethoxy -2-oxoethyl) -3-(methanesulfonyl hydrazinocarbonyl) pyridinium bromide or other cosmetically acceptable salts thereof,
(j)1-(2-phenyl-2-oxoethyl)-3-(phenylsulfonylhydrazino carbonyl) pyridinium bromide or other cosmetically acceptable salts thereof,
(k)1-(2-phenyl-2-oxoethyl)-2-chloro-3-(phenylsulfonyl hydrazino carbonyl) pyridinium bromide or other cosmetically acceptable salts thereof,
(l) 1-(2-thien -2'-yl -2-oxoethyl)-4-(2-(benzoyloxy) ethyl aminocarbonyl) pyridinium bromide or other cosmetically acceptable salts thereof,
(m)1- (2- (2,'4'- dichlorophenyl) -2-oxoethyl) -3-(2- (benzoyloxy) ethylaminocarbonyl) pyridinium bromide or other cosmetically acceptable salts thereof,
(n) 1-(2-phenyl -2-oxoethyl) -3-(2-(acetoxy) ethyloxy) carbonyl) pyridinium bromide or other cosmetically acceptable salts thereof,
(o) 1-(2-ethoxy -2-oxoethyl) -3-(2-(benzoyloxy) ethyloxy carbonyl) pyridinium bromide or other cosmetically acceptable salts thereof,
(p)1-(2-phenylamino-2-oxoethyl)-4-(phenylsulfonyl hydrazino carbonyl)pyridinium chloride or other cosmetically acceptable salts thereof,
(q) 1-(2-(2,(4(-dichlorophenyl)-2-oxoethyl)-3- (2(methoxy) ethyloxycarbonyl) pyridinium bromide or other cosmetically acceptable salts thereof,
(r) 1-(2-phenylamino-2-oxoethyl)-3-((benzoyloxy) ethylaminocarbonyl) pyridinium chloride or other cosmetically acceptable salts thereof,
(s) 1-(2-thien-2'-yl-2-oxoethyl)-3-(phenylaminocarbonyl hydrazinocarbonyl) pyridinium bromide or other cosmetically acceptable salts thereof,
(t) 1-(2-phenyl-2-oxoethyl)-3-(2-(acetoxy) ethylaminocarbonyl) pyridinium bromide or other cosmetically acceptable salts thereof,
(u) 1-(2-phenylamino-2-oxoethyl)-3-(phenyl sulfonyl hydrazino carbonyl) pyridinium chloride or other cosmetically acceptable salts thereof,
(v) 1-(2-phenylamino-2-oxoethyl)-3-((4-methylphenyl)sulfonyl hydrazinocarbonyl) pyridinium chloride or other cosmetically acceptable salts thereof,
(w) 1-(2-phenyl-2-oxoethyl)-3-(2-(benzoyloxy)ethyloxy carbonyl) pyridinium bromide or other cosmetically acceptable salts thereof,
(x) 1-(2-thien-2'-yl-2-oxoethyl)-3-(phenylcarbonyl hydrazino carbonyl) pyridinium bromide or other cosmetically acceptable salts thereof,
(y) 1-(2-ethoxy-2-oxoethyl)-3-((phenylmethyl)sulfonyl hydrazino carbonyl)pyridinium bromide or other cosmetically acceptable salts thereof
(z)1-(2-phenyl-2-oxoethyl)-3-((phenylmethyl)sulfonyl hydrazino carbonyl)pyridinium bromide or other cosmetically acceptable salts thereof,
(aa) N, N' - bis [3-carbonyl-1-(2-furan-2'-yl-2-oxoethyl) pyridinium] hydrazine dibromide or other cosmetically acceptable salts thereof,
(ab) N,N'-bis [3-carbonyl -1- (2-thien-2'-yl-2-oxoethyl) pyridinium] hydrazine dichloride or other cosmetically acceptable salts thereof,
(ac) N,N'-bis-[3-carbonyl-1-(2-cyclopropylamino-2-oxoethyl) pyridinium] hydrazine dichloride or other cosmetically acceptable salts thereof,
(ad) 1-(2',4'-dichlorophenyl-2-oxoethyl)-3-(2-methoxyethyl aminocarbonyl)-pyridinium bromide or other cosmetically acceptable salts thereof,
(ae) 1-(2-thien-2'-yl-2-oxoethyl)-3-((2-methoxy ethyl) amino carbonyl)-5-bromo pyridinium chloride or other cosmetically acceptable salts thereof,
(af) 1-(2-thien-2'-yl-2-oxoethyl)-3-(methanesulfonyl hydrazino carbonyl) pyridinium chloride or other cosmetically acceptable salts thereof,
(ag) 1-(2-thien-2'yl-2-oxoethyl)-3-(2-(2-chloro-3-pyridoylhydrazinocarbonyl)-pyridinium chloride or other cosmetically acceptable salts thereof,
(ah) 1-(2-cyclopropylamino-2-oxoethyl)-3-(2-methoxyethylaminocarbonyl) -pyridinium chloride or other cosmetically acceptable salts thereof,
(ai) 1-(2-isopropylamino-2-oxoethyl)-3-(2-methylsulfonylhydrazinocarbonyl)-pyridinium chloride or other cosmetically acceptable salts thereof,
(aj) 1-(2-phenylamino-2-oxo ethyl)-3-({2-(1-oxo-3-cyclohexyl)-propyl}-hydrazinocarbonyl)-pyridinium bromide

or other cosmetically acceptable salts thereof, (ak) 1-(2-thien-2'-yl-2-oxoethyl)-3-[2-(benzoyloxy)ethylamino carbonyl]-pyridinium bromide or other cosmetically acceptable salts thereof,

(al) 1-(4-ethoxy-2, 4-dioxobutyl)-3-(2-(benzoxyloxy)ethylamino carbonyl)-pyridinium chloride or other cosmetically acceptable salts thereof,

(am)1-(2-thien-2'-yl-2-oxoethyl)-3-[1-oxo-1-(2-methoxy carbonyl) pyridyl] hydrazino pyridinium chloride or other cosmetically acceptable salts thereof,

(an)1-[1-(2-thien-2'-yl-2-oxoethyl)-5-aminocarbonyl-3-carbonyl pyridinium]-2-[1-(2-thien-2'-yl-2-oxoethyl )-3-carbonyl pyridinium] hydrazine dichloride or other cosmetically acceptable salts thereof,

(ao)1-(2-thien-2'-yl-2-oxoethyl)-3-(trifluoromethanesulfonyl hydrazino carbonyl) - pyridinium bromide or other cosmetically acceptable salts thereof,

(ap)1-[1-(2-thien-2'-yl-2-oxoethyl)-6-methyl-3-carbonyl pyridinium]-2-[1-(2-thien-2'-yl-2-oxoethyl )-3-carbonyl pyridinium ] hydrazine dichloride or other cosmetically acceptable salts thereof,

(aq) N,N'-bis[3-carbonyl-1-(2-(5-methyl-thien-2-yl)-2-oxoethyl) pyridinium] hydrazine dichloride or other cosmetically acceptable salts thereof,

(ar) N,N'-bis[3-carbonyl-1-(2-(5-chloro-thien-2-yl)-2-oxoethyl) pyridinium] hydrazine dichloride or other cosmetically acceptable salts thereof,

(as) 1-(2-thien-2'-yl-2-oxoethyl)-3-(methanesulfonyl hydrazino carbonyl )-6-methyl pyridinium bromide or other cosmetically acceptable salts thereof,

(at) N,N'-bis[3-carbonyl-1-(2-(4-nitro-thien-2-yl)-2-oxoethyl)pyridinium] hydrazine dichloride or other cosmetically acceptable salts thereof,

(au) 1-(2-phenylamino-2-oxoethyl)-3-(phenyl hydrazino carbonyl) pyridinium chloride or other cosmetically acceptable salts thereof,

(av) 1-(2-(4-nitro-thien-2-yl)-2-oxoethyl)-3-(methanesulfonyl hydrazino carbonyl ) pyridinium bromide or other cosmetically acceptable salts thereof,

(aw) 1-(2-(5-nitro-thien-2-yl)-2-oxoethyl)-3-(methanesulfonyl hydrazino carbonyl) pyridinium chloride or other cosmetically acceptable salts thereof,

(ax) 1-(2-(5-chloro-thien-2-yl)-2-oxoethyl)-3-(methanesulfonyl hydrazino carbonyl) pyridinium bromide or other cosmetically acceptable salts thereof,

(ay) 1-(2-thien-2'-yl-2-oxoethyl)-3-(ethoxycarbonyl hydrazino carbonyl) pyridinium bromide or other cosmetically acceptable salts thereof,

(az) 1-(2-thien-2'-yl-2-oxoethyl)-3-(isopropylsulfonyl hydrazino carbonyl) pyridinium bromide or other cosmetically acceptable salts thereof,

(ba) 1-(2-thien-2'-yl-2-oxoethyl)-3-(methanesulfonyl hydrazino carbonyl) -5-bromo pyridinium bromide or other cosmetically acceptable salts thereof,

(bb) 1-(2-(2-ethoxy carbonyl pyrrolidin-1-yl)-2-oxoethyl)-3-(methanesulfonyl hydrazino carbonyl) pyridinium chloride or other cosmetically acceptable salts thereof,

(bc) 1-(2-(5-methyl-thien-2-yl)-2-oxoethyl)-3-(methanesulfonyl hydrazino carbonyl) pyridinium chloride or other cosmetically acceptable salts thereof,

(bd) 1-(2-(4- carboethoxy -thiazolidin-3-yl)-2-oxoethyl) -3-(methanesulfonyl hydrazino carbonyl ) pyridinium chloride or other cosmetically acceptable salts thereof,

(be) 1-(2-(4-benzyl piperidin-1-yl)-2-oxoethyl)-3-(methanesulfonyl hydrazino carbonyl) pyridinium chloride or other cosmetically acceptable salts thereof,

(bf) N,N(-bis[3-carbonyl-1-(2-(2-ethoxycarbonyl pyrrolidin-1-yl)-2-oxoethyl) pyridinium] hydrazine dichloride or other cosmetically acceptable salts thereof,

(bg) 1-(2-phenylamino-2-oxoethyl)-4 -[ 2-(benzoyloxy) ethylamino carbonyl ] pyridinium chloride or other cosmetically acceptable salts thereof,

(bh) 1-(2-thien-2(-yl-2-oxoethyl)-3-(phenyl hydrazino carbonyl) pyridinium bromide or other cosmetically acceptable salts thereof,

(bi) 1-(2-thien-2(-yl-2-oxoethyl)-3-(p-methoxy phenyl sulfonyl hydrazino carbonyl) pyridinium bromide or other cosmetically acceptable salts thereof,

(bj) 1-(2-ethoxy-2-oxoethyl)-3-(phenyl aminocarbonyl hydrazino carbonyl ) pyridinium bromide or other cosmetically acceptable salts thereof,

(bk) 1-(2-ethoxy-2-oxoethyl)-3-(p-toluene sulfonyl hydrazino carbonyl) pyridinium bromide or other cosmetically acceptable salts thereof,

(bl) 1-(2-phenyl-2-oxoethyl)-3-(phenylamino carbonyl hydrazino carbonyl ) pyridinium bromide or other cosmetically acceptable salts thereof,

(bm) 1-(2-phenylamino-2-oxoethyl)-3-(benzyl sulfonyl hydrazino carbonyl) pyridiniumchloride or other cosmetically acceptable salts thereof,

(bn) 1-(2-phenyl-2-oxoethyl)-4-(methanesulfonyl hydrazino carbonyl ) pyridinium bromide or other cosmetically acceptable salts thereof,
(bo) 1-(2-phenyl-2-oxoethyl)-3-(phenyl hydrazino carbonyl ) pyridinium bromide or other cosmetically acceptable salts thereof,
(bp) 1 -(2-ethoxy-2-oxoethyl)-4-[2-(benzoyloxy) ethyl amino carbonyl ] pyridinium bromide or other cosmetically acceptable salts thereof,
(bq) 1-(2-ethoxy-2-oxoethyl)-3-(phenyl hydrazino carbonyl ) pyridinium bromide or other cosmetically acceptable salts thereof,
(br) 1-(2-phenyl-2-oxoethyl)-3-(p-methoxyphenyl sulfonyl hydrazino carbonyl ) pyridinium bromide or other cosmetically acceptable salts thereof,
(bs) 1-(2-phenyl-2-oxoethyl)- 4-[2-(benzoyloxy) ethyl amino carbonyl] pyridinium bromide or other cosmetically acceptable salts thereof,
(bt) 1-(2-ethoxy-2-oxoethyl)- 4-(p-methanesulfonyl hydrazino carbonyl ) pyridinium bromide or other cosmetically acceptable salts thereof,
(bu) 3-carbonylamino-1- (2-(2, 4-dichlorophenyl)-2-oxoethyl)-pyridinium bromide or other cosmetically acceptable salts thereof,
(bv) 3-(tetrahydrobenzothiazol-2-yl) aminocarbonyl -1-(2-(2, 4-dichlorophenyl)-2-oxoethyl)-pyridinium bromide or other cosmetically acceptable salts thereof,
(bw) 1-(2-phenyl-2-oxoethyl)-3-((2-hydroxyethyl) aminocarbonyl) pyridinium bromide or other cosmetically acceptable salts thereof,
(bx) 3-carbonylamino-1- (2-thien-2'-yl-2-oxoethyl)-pyridinium bromide or other cosmetically acceptable salts thereof,
(by) 1-(2-phenyl -2-oxoethyl)-3-((p-sulfonamidophenylene) aminocarbonyl) pyridinium bromide or other cosmetically acceptable salts thereof,
(bz) 1-(2-ethoxy-2-oxoethyl)-3-((2-hydroxyethyl) aminocarbonyl) pyridinium bromide or other cosmetically acceptable salts thereof,
(ca) 1-(2-phenyl-2-oxoethyl)-3-(isopropyloxycarbonyl) pyridinium bromide or other cosmetically acceptable salts thereof,
(cb) 1-(2-oxopropyl)-3-((2-hydroxyethyl) aminocarbonyl) pyridinium chloride or other cosmetically acceptable salts thereof,
(cc) 1-(2-thien-2'-yl-2-oxoethyl)-3-((2-hydroxyethyl) aminocarbonyl) pyridinium bromide or other cosmetically acceptable salts thereof,
(cd) 1-(2-(2,4 - dichlorophenyl-2-oxoethyl) -3- (isopropyloxycarbonyl) pyridinium bromide or other cosmetically acceptable salts thereof,
(ce) 1- (2-phenyl - 2- oxoethyl) - 3- ((4-methylthiazol - 2 -yl) aminocarbonyl) pyridinium bromide or other cosmetically acceptable salts thereof,
(cf) 1-(2- phenylamino -2- oxoethyl ) -3- (n-butyloxycarbonyl) pyridinium chloride or other cosmetically acceptable salts thereof,
(cg) 1- (2- phenylamino - 2- oxoethyl)- 3- ((2-hydroxyethyl) aminocarbonyl) - pyridinium chloride or other cosmetically acceptable salts thereof,
(ch) 1- (2- (2,4 - dichlorophenyl ) - 2-oxoethyl) - 3- (n - butoxycarbonyl) pyridinium bromide or other cosmetically acceptable salts thereof,
(ci) 1-(2 - (2, 4 - dichlorophenyl) - 2-oxoethyl) - 3- (n-butylamino-carbonyl) pyridinium bromide or other cosmetically acceptable salts thereof,
(cj) 1-(2-phenyl-2-oxoethyl)-3-(1-phenyl-1-oxomethyl) pyridinium bromide or other cosmetically acceptable salts thereof and
(ck) 1- (2 phenyl - 2-oxoethyl) - 3- (methoxycarbonyl) pyridinium bromide or other cosmetically acceptable salts thereof

**26.** The cosmetic composition as claimed in claim 1, wherein the concentration of said compound is between 0.005 to 50% by weight.

**27.** The cosmetic composition as claimed in claim 26, wherein the concentration of said compound is between 0.25% to 5.0% by weight.

**28.** A cosmetic composition comprising the compound of the Formula (I) as defined in claim 1 or other cosmetically acceptable salts thereof and one or more agents selected from the group consisting of emollients, emulsifiers, agents modifying skin differentation and/or proliferation and/or pigmentation, antiparasitic agents, preservatives,

alcohols, fragrances, thickening agents, humectants, colorants, silicones, exfoliating agents, keratolytic agents, retinoids, sunscreens, skin penetration enhancers, anti-inflammatory agents, vitamins, thrombolytic agents, anticlotting agents, capillary protectants, additional antioxidants, hormones, antibacterial agents, antiviral agents, steroidal anti-inflammatory agents, anaesthetics, anti-seborrhoeic agents, anti-dandruff agents, anti-acne agents, antifree radical agents, analgesics, lipophilic compounds, antihistamine agents, insect repellants, skin cooling compounds, lubricants and anti-fungal agents or mixtures thereof.

**29.** A method of cosmetic application comprising applying an effective amount of a composition as claimed in claim 28.

**30.** A pharmaceutical composition for scavenging free radicals in the body cell of a mammal comprising a compound of formula (I) or pharmaceutically acceptable salts thereof.

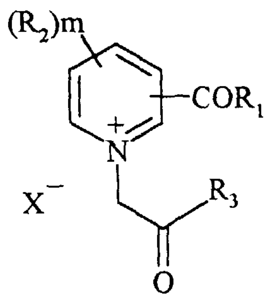

**(I)**

in admixture with pharmaceutically acceptable carrier, diluent, excipient or solvate
wherein
$R_1$ is $-R_4-R_5$ or $-N(R_7)\ N\ (R_7)\ R_9$;
$R_4$ is selected from the group consisting of $-N(R_7)R_6O-$,
$-N(R_7)R_6N(R_7)$,
$-OR_6O$, and $-OR_6N(R_7)-$, and Y-$R_{11}$
where $R_6$ is alkyl
$R_5$ is selected from the group consisting of alkyl, aryl including heteroaryl, $-COR_7$, -
$SO_2R_7$, -C(S) $NHR_7$, -C(NH)$NHR_7$, $-COR_{10}$,

-C(O)$NHR_7$ and 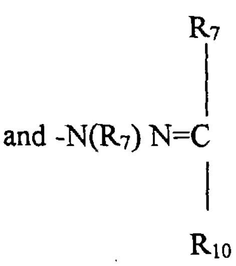

where $R_7$ is selected from the group consisting of H, alkyl and aryl including heteroaryl, provided $R_7$ may be the same or different for $R_1$ and $R_3$ in the same compound; $R_2$ is selected from the group consisting of F, Cl, Br, I, $OR_7$, $NO_2$, alkyl, aryl including heteroaryl, formyl, acyl, C(O)$NR_7R_{10}$, C(O)$OR_7$, $NR_7R_{10}$, N=C($R_7$)($R_{10}$), $SR_7$, $SO_2NH_2$, $SO_2$ alkyl and $SO_2$aryl;
m is 0, 1 or 2;
$R_3$ is selected from the group consisting of $R_7$, $OR_7$, N($R_7$) ($R_{10}$), N=C($R_7$) ($R_{10}$), N($R_7$) N($R_7$) ($R_{10}$), N($R_7$) N=C ($R_7$) ($R_{10}$) and CH($R_7$)C(O)$R_8$

where $R_8$ is selected from the group consisting of $R_7$, $OR_7$ and $NR_7R_{10}$;
$R_9$ is selected from the group consisting of hydrogen, alkyl, aryl including heteroaryl, $C(O)R_{10}$, $-SO_2R_{10}$, C(S) $NHR_{10}$, C(NH) NH ($R_{10}$) and C(O) $NHR_{10}$;
$R_{10}$ is selected from the group consisting of H, alkyl and aryl, including heteroaryl and in each case may be the same or different from substituent $R_7$, provided $R_{10}$ may be the same or different for $R_1$ and $R_3$ in the same compound;
X is selected from group consisting of a halide ion, acetate ion, perchlorate ion, sulfonate ion, oxalate ion, citrate ion, tosylate ion, maleate ion, mesylate ion, carbonate ion, sulfite ion, phosphoric hydrogen ion, phosphonate ion, phosphate ion, $BF_4$- and $PF_6$-;
Y is selected from oxygen, NH, $NR_{12}$ and null; $R_{11}$ and $R_{12}$ are independently selected from hydrogen, alkyl and aryl. with proviso that,

(i) when two alkyl groups are present on the same carbon or nitrogen, they may be linked together to form a cyclic structure;
(ii) the nitrogen of heteroaryl ring of $R_{10}$, when present, may be quatemized;
(iii) when $R_3$ is $OR_7$ and $R_1$ is $-NHNH_2$ then $R_7$ is not alkyl and
(iv) when $R_3$ is $OR_7$, $R_1$ is $N(R_7)(NR_7)R_9$ and $R_9$ is $C(O)R_{10}$ where

$R_{10}$ is alkyl, then $R_7$ is not hydrogen.

**31.** The composition as claimed in claim 30, wherein $-C(O)R_1$ group of said compound is at position 3 or 4.

**32.** The composition as claimed in claim 31, wherein $-C(O)R_1$ group of said compound is at position 3.

**33.** The composition as claimed in claim 30, wherein for the said compound m is 0 or 1.

**34.** The composition as claimed in claim 31, wherein for the said compound m is 0 or 1.

**35.** The composition as claimed in claim 32, wherein for the said compound m is 0 or 1.

**36.** The composition as claimed in claim 30, wherein for the said compound m is 0.

**37.** The composition as claimed in claim 31, wherein for the said compound m is 0.

**38.** The composition as claimed in claim 32, wherein for the said compound m is 0.

**39.** The composition as claimed in claim 30, wherein for the said compound X is a halide ion.

**40.** A composition as claimed in claim 30, comprising compounds selected from the group consisting of

(a) N,N'-bis[3-carbonyl-1-(2-thien -2'- yl -2-oxoethyl) -3-pyridinium] hydrazine dibromide or other pharmaceutially acceptable salts thereof,
(b) 1-(2-ethoxy -2-oxoethyl) -3-(2-(2-pyridyl)hydrazinocarbonyl) pyridinium bromide or other pharmaceutially acceptable salts thereof,
(c) 1-(2-ethoxy -2-oxoethyl) -3-(2-(benzoyloxy) ethylamino carbonyl) pyridinium bromide or other pharmaceutially acceptable salts thereof,
(d)N,N'-bis[3-carbonyl-1-(2-phenyl-2-oxoethyl)pyridinium]hydrazine dibromide or other pharmaceutially acceptable salts thereof,
(e)1-(2-phenyl-2-oxoethyl)-3-(hydrazinocarbonyl)pyridinium bromide or other pharmaceutially acceptable salts thereof,
(f) 1-(2-thien -2'-yl -2-oxoethyl) -3-(methanesulfonyl hydrazinocarbonyl) pyridinium bromide or other pharmaceutially acceptable salts thereof,
(g) N,N'-bis[3-carbonyl-1-(2-(2',4'-dichlorophenyl)-2-oxoethyl) pyridinium] hydrazine dibromide or other pharmaceutially acceptable salts thereof,
(h) 1-(2-phenyl -2-oxoethyl) -3-(methanesulfonyl hydrazinocarbonyl) pyridinium bromide or other pharmaceutially acceptable salts thereof,
(i) 1-(2-ethoxy -2-oxoethyl) -3-(methanesulfonyl hydrazinocarbonyl) pyridinium bromide or other pharmaceutially acceptable salts thereof,

(j)1-(2-phenyl-2-oxoethyl)-3-(phenylsulfonylhydrazino carbonyl) pyridinium bromide or other pharmaceutially acceptable salts thereof,
(k)1-(2-phenyl-2-oxoethyl)-2-chloro-3-(phenylsulfonyl hydrazino carbonyl) pyridinium bromide or other pharmaceutially acceptable salts thereof,
(l) 1-(2-thien -2'-yl -2-oxoethyl)-4-(2-(benzoyloxy) ethyl aminocarbonyl) pyridinium bromide or other pharmaceutially acceptable salts thereof,
(m)1- (2- (2,'4'- dichlorophenyl) -2-oxoethyl) -3-(2- (benzoyloxy) ethylaminocarbonyl) pyridinium bromide or other pharmaceutially acceptable salts thereof,
(n) 1-(2-phenyl -2-oxoethyl) -3-(2-(acetoxy) ethyloxy) carbonyl) pyridinium bromide or other pharmaceutially acceptable salts thereof,
(o) 1-(2-ethoxy -2-oxoethyl) -3-(2-(benzoyloxy) ethyloxy carbonyl) pyridinium bromide or other pharmaceutially acceptable salts thereof,
(p) 1-(2-phenylamino-2-oxoethyl)-4-(phenylsulfonyl hydrazino carbonyl)pyridinium chloride or other pharmaceutially acceptable salts thereof,
(q) 1-(2-(2,'4'-dichlorophenyl)-2-oxoethyl)-3- (2(methoxy) ethyloxycarbonyl) pyridinium bromide or other pharmaceutially acceptable salts thereof,
(r) 1-(2-phenylamino-2-oxoethyl)-3-((benzoyloxy) ethylaminocarbonyl) pyridinium chloride or other pharmaceutially acceptable salts thereof,
(s) 1-(2-thien-2'-yl-2-oxoethyl)-3-(phenylaminocarbonyl hydrazinocarbonyl) pyridinium bromide or other pharmaceutially acceptable salts thereof,
(t) 1-(2-phenyl-2-oxoethyl)-3-(2-(acetoxy) ethylaminocarbonyl) pyridinium bromide or other pharmaceutially acceptable salts thereof,
(u) 1-(2-phenylamino-2-oxoethyl)-3-(phenyl sulfonyl hydrazino carbonyl) pyridinium chloride or other pharmaceutially acceptable salts thereof,
(v) 1-(2-phenylamino-2-oxoethyl)-3-((4-methylphenyl)sulfonyl hydrazinocarbonyl) pyridinium chloride or other pharmaceutially acceptable salts thereof,
(w) 1-(2-phenyl-2-oxoethyl)-3-(2-(benzoyloxy)ethyloxy carbonyl) pyridinium bromide or other pharmaceutially acceptable salts thereof,
(x) 1-(2-thien-2'-yl-2-oxoethyl)-3-(phenylcarbonyl hydrazino carbonyl) pyridinium bromide or other pharmaceutially acceptable salts thereof,
(y) 1-(2-ethoxy-2-oxoethyl)-3-((phenylmethyl)sulfonyl hydrazino carbonyl)pyridinium bromide or other pharmaceutially acceptable salts thereof,
(z)1-(2-phenyl-2-oxoethyl)-3-((phenylmethyl)sulfonyl hydrazino carbonyl)pyridinium bromide or other pharmaceutially acceptable salts thereof,
(aa) N,N' - bis[3-carbonyl-1-(2-furan-2'-yl-2-oxoethyl) pyridinium] hydrazine dibromide or other pharmaceutially acceptable salts thereof,
(ab) N,N'-bis [3-carbonyl-1-(2-thien-2'-yl-2-oxoethyl) pyridinium] hydrazine dichloride or other pharmaceutially acceptable salts thereof,
(ac) N,N'-bis-[3-carbonyl-1-(2-cyclopropylamino-2-oxoethyl) pyridinium] hydrazine dichloride or other pharmaceutially acceptable salts thereof,
(ad) 1-(2',4'-dichlorophenyl-2-oxoethyl)-3-(2-methoxyethyl aminocarbonyl)-pyridinium bromide or other pharmaceutially acceptable salts thereof,
(ae) 1-(2-thien-2'-yl-2-oxoethyl)-3-((2-methoxy ethyl) amino carbonyl)-5-bromo pyridinium chloride or other pharmaceutially acceptable salts thereof,
(af) 1-(2-thien-2'-yl-2-oxoethyl)-3-(methanesulfonyl hydrazino carbonyl) pyridinium chloride or other pharmaceutially acceptable salts thereof,
(ag) 1-(2-thien-2'yl-2-oxoethyl)-3-(2-(2-chloro-3-pyridoylhydrazinocarbonyl)-pyridinium chloride or other pharmaceutially acceptable salts thereof,
(ah) 1-(2-cyclopropylamino-2-oxoethyl)-3-(2-methoxyethylaminocarbonyl) -pyridinium chloride or other pharmaceutially acceptable salts thereof,
(ai) 1-(2-isopropylamino-2-oxoethyl)-3-(2-methylsulfonylhydrazinocarbonyl)-pyridinium chloride or other pharmaceutially acceptable salts thereof,
(aj) 1-(2-phenylamino-2-oxo ethyl)-3-({2-(1-oxo-3-cyclohexyl)-propyl}-hydrazinocarbonyl)-pyridinium bromide or other pharmaceutially acceptable salts thereof,
(ak) 1-(2-thien-2'-yl-2-oxoethyl)-3-[2-(benzoyloxy)ethylamino carbonyl]-pyridinium bromide or other pharmaceutially acceptable salts thereof,
(al) 1-(4-ethoxy-2, 4-dioxobutyl)-3-(2-(benzoxyloxy)ethylamino carbonyl)-pyridinium chloride or other pharmaceutially acceptable salts thereof,

(am)1-(2-thien-2'-yl-2-oxoethyl)-3-[1-oxo-1-(2-methoxy carbonyl) pyridyl] hydrazino pyridinium chloride or other pharmaceutially acceptable salts thereof,
(an)1-[1-(2-thien-2'-yl-2-oxoethyl)-5-aminocarbonyl-3-carbonyl pyridinium]-2-[1-(2-thien-2'-yl-2-oxoethyl )-3-carbonyl pyridinium] hydrazine dichloride or other pharmaceutially acceptable salts thereof,
(ao)1-(2-thien-2'-yl-2-oxoethyl)-3-(trifluoromethanesulfonyl hydrazino carbonyl) - pyridinium bromide or other pharmaceutially acceptable salts thereof,
(ap)1-[1-(2-thien-2'-yl-2-oxoethyl)-6-methyl-3-carbonyl pyridinium]-2-[1-(2-thien-2'-yl-2-oxoethyl )-3-carbonyl pyridinium ] hydrazine dichloride or other pharmaceutially acceptable salts thereof,
(aq) N,N'-bis[3-carbonyl-1-(2-(5-methyl-thien-2-yl)-2-oxoethyl) pyridinium] hydrazine dichloride or other pharmaceutially acceptable salts thereof,
(ar) N,N'-bis[3-carbonyl-1-(2-(5-chloro-thien-2-yl)-2-oxoethyl) pyridinium] hydrazine dichloride or other pharmaceutially acceptable salts thereof,
(as) 1-(2-thien-2'-yl-2-oxoethyl)-3-(methanesulfonyl hydrazino carbonyl )-6-methyl pyridinium bromide or other pharmaceutially acceptable salts thereof,
(at) N,N'-bis[3-carbonyl-1-(2-(4-nitro-thien-2-yl)-2-oxoethyl)pyridinium] hydrazine dichloride or other pharmaceutially acceptable salts thereof,
(au) 1-(2-phenylamino-2-oxoethyl)-3-(phenyl hydrazino carbonyl) pyridinium chloride or other pharmaceutially acceptable salts thereof,
(av) 1-(2-(4-nitro-thien-2-yl)-2-oxoethyl)-3-(methanesulfonyl hydrazino carbonyl ) pyridinium bromide or other pharmaceutially acceptable salts thereof,
(aw) 1-(2-(5-nitro-thien-2-yl)-2-oxoethyl)-3-(methanesulfonyl hydrazino carbonyl) pyridinium chloride or other pharmaceutially acceptable salts thereof,
(ax) 1-(2-(5-chloro-thien-2-yl)-2-oxoethyl)-3-(methanesulfonyl hydrazino carbonyl) pyridinium bromide or other pharmaceutially acceptable salts thereof,
(ay) 1-(2-thien-2'-yl-2-oxoethyl)-3-(ethoxycarbonyl hydrazino carbonyl) pyridinium bromide or other pharmaceutially acceptable salts thereof,
(az) 1-(2-thien-2'-yl-2-oxoethyl)-3-(isopropylsulfonyl hydrazino carbonyl) pyridinium bromide or other pharmaceutially acceptable salts thereof,
(ba) 1-(2-thien-2'-yl-2-oxoethyl)-3-(methanesulfonyl hydrazino carbonyl) -5-bromo pyridinium bromide or other pharmaceutially acceptable salts thereof,
(bb) 1-(2-(2-ethoxy carbonyl pyrrolidin-1-yl)-2-oxoethyl)-3-(methanesulfonyl hydrazino carbonyl) pyridinium chloride or other pharmaceutially acceptable salts thereof,
(bc) 1-(2-(5-methyl-thien-2-yl)-2-oxoethyl)-3-(methanesulfonyl hydrazino carbonyl) pyridinium chloride or other pharmaceutially acceptable salts thereof,
(bd) 1-(2-(4- carboethoxy -thiazolidin-3-yl)-2-oxoethyl) -3-(methanesulfonyl hydrazino carbonyl ) pyridinium chloride or other pharmaceutially acceptable salts thereof,
(be) 1-(2-(4-benzyl piperidin-1-yl)-2-oxoethyl)-3-(methanesulfonyl hydrazino carbonyl) pyridinium chloride or other pharmaceutically acceptable salts thereof,
(bf) N,N(-bis[3-carbonyl-1-(2-(2-ethoxycarbonyl pyrrolidin-1-yl)-2-oxoethyl) pyridinium] hydrazine dichloride or other pharmaceutically acceptable salts thereof,
(bg) 1-(2-phenylamino-2-oxoethyl)-4 -[ 2-(benzoyloxy) ethylamino carbonyl ] pyridinium chloride or other pharmaceutically acceptable salts thereof,
(bh) 1-(2-thien-2(-yl-2-oxoethyl)-3-(phenyl hydrazino carbonyl) pyridinium bromide or other pharmaceutically acceptable salts thereof,
(bi) 1-(2-thien-2(-yl-2-oxoethyl)-3-(p-methoxy phenyl sulfonyl hydrazino carbonyl) pyridinium bromide or other pharmaceutically acceptable salts thereof,
(bj) 1-(2-ethoxy-2-oxoethyl)-3-(phenyl aminocarbonyl hydrazino carbonyl ) pyridinium bromide or other pharmaceutically acceptable salts thereof,
(bk) 1-(2-ethoxy-2-oxoethyl)-3-(p-toluene sulfonyl hydrazino carbonyl) pyridinium bromide or other pharmaceutically acceptable salts thereof,
(bl) 1-(2-phenyl-2-oxoethyl)-3-(phenylamino carbonyl hydrazino carbonyl ) pyridinium bromide or other pharmaceutically acceptable salts thereof,
(bm) 1-(2-phehylamino-2-oxoethyl)-3-(benzyl sulfonyl hydrazino carbonyl) pyridiniumchloride or other pharmaceutically acceptable salts thereof,
(bn) 1-(2-phenyl-2-oxoethyl)-4-(methanesulfonyl hydrazino carbonyl ) pyridinium bromide or other pharmaceutically acceptable salts thereof,
(bo) 1-(2-phenyl-2-oxoethyl)-3-(phenyl hydrazino carbonyl ) pyridinium bromide or other pharmaceutically acceptable salts thereof,

(bp) 1 -(2-ethoxy-2-oxoethyl)-4-[2-(benzoyloxy) ethyl amino carbonyl ] pyridinium bromide or other pharmaceutically acceptable salts thereof,
(bq) 1-(2-ethoxy-2-oxoethyl)-3-(phenyl hydrazino carbonyl ) pyridinium bromide or other pharmaceutically acceptable salts thereof,
(br) 1-(2-phenyl-2-oxoethyl)-3-(p-methoxyphenyl sulfonyl hydrazino carbonyl ) pyridinium bromide or other pharmaceutically acceptable salts thereof,
(bs) 1-(2-phenyl-2-oxoethyl)- 4-[2-(benzoyloxy) ethyl amino carbonyl] pyridinium bromide or other pharmaceutically acceptable salts thereof,
(bt) 1-(2-ethoxy-2-oxoethyl)- 4-(p-methanesulfonyl hydrazino carbonyl ) pyridinium bromide or other pharmaceutically acceptable salts thereof,
(bu) 3-carbonylamino-1- (2-(2, 4-dichlorophenyl)-2-oxoethyl)-pyridinium bromide or other pharmaceutically acceptable salts thereof,
(bv) 3-(tetrahydrobenzothiazol-2-yl) aminocarbonyl -1-(2-(2, 4-dichlorophenyl)-2-oxoethyl)-pyridinium bromide or other pharmaceutically acceptable salts thereof,
(bw) 1-(2-phenyl-2-oxoethyl)-3-((2-hydroxyethyl) aminocarbonyl) pyridinium bromide or other pharmaceutically acceptable salts thereof,
(bx) 3-carbonylamino-1- (2-thien-2'-yl-2-oxoethyl)-pyridinium bromide or other pharmaceutically acceptable salts thereof,
(by) 1-(2-phenyl -2-oxoethyl)-3-((p-sulfonamidophenylene) aminocarbonyl) pyridinium bromide or other pharmaceutically acceptable salts thereof,
(bz) 1-(2-ethoxy-2-oxoethyl)-3-((2-hydroxyethyl) aminocarbonyl) pyridinium bromide or other pharmaceutically acceptable salts thereof,
(ca) 1-(2-phenyl-2-oxoethyl)-3-(isopropyloxycarbonyl) pyridinium bromide or other pharmaceutically acceptable salts thereof,
(cb) 1-(2-oxopropyl)-3-((2-hydroxyethyl) aminocarbonyl) pyridinium chloride or other pharmaceutically acceptable salts thereof,
(cc) 1-(2-thien-2'-yl-2-oxoethyl)-3-((2-hydroxyethyl) aminocarbonyl) pyridinium bromide or other pharmaceutically acceptable salts thereof,
(cd) 1-(2-(2,4 - dichlorophenyl-2-oxoethyl) -3- (isopropyloxycarbonyl) pyridinium bromide or other pharmaceutically acceptable salts thereof,
(ce) 1- (2-phenyl - 2- oxoethyl) - 3- ((4-methylthiazol - 2 -yl) aminocarbonyl) pyridinium bromide or other pharmaceutically acceptable salts thereof,
(cf) 1-(2- phenylamino -2- oxoethyl ) -3- (n-butyloxycarbonyl) pyridinium chloride or other pharmaceutically acceptable salts thereof,
(cg) 1- (2- phenylamino - 2- oxoethyl)- 3- ((2-hydroxyethyl) aminocarbonyl) - pyridinium chloride or other pharmaceutically acceptable salts thereof,
(ch) 1- (2- (2,4 - dichlorophenyl ) - 2-oxoethyl) - 3- (n - butoxycarbonyl) pyridinium bromide or other pharmaceutically acceptable salts thereof,
(ci) 1-(2 - (2, 4 - dichlorophenyl) - 2-oxoethyl) - 3- (n-butylamino-carbonyl) pyridinium bromide or other pharmaceutically acceptable salts thereof,
(cj) 1-(2-phenyl-2-oxoethyl)-3-(1-phenyl-1-oxomethyl) pyridinium bromide or other pharmaceutically acceptable salts thereof and
(ck) 1- (2 phenyl - 2-oxoethyl) - 3- (methoxycarbonyl) pyridinium bromide or other pharmaceutically acceptable salts thereof.

**41.** The pharmaceutical composition as claimed in claim 30, in the form of an oral formulation.

**42.** The pharmaceutical composition as claimed in claim 30, wherein said acceptable carrier, diluent, solvent or excipient is selected from the group consisting of starch, lactose, polyvinyl pyrolidone (K-30), talc and magnesium stearate.

**43.** The pharmaceutical composition as claimed in claim 30, in the form of a parenteral formulation.

**44.** A method for the preparation of a parenteral formulation as claimed in claim 43, which comprises dissolving one or more compounds represented by general formula (I), as defined in claim 30, in polyethylene glycol 400 and diluting the solution so obtained, with an isotonic solution or water to a desired concentration.

**45.** Pharmaceutical composition as claimed in claim 30, in the form of a lotion, oral rinse and toothpaste.

**46.** The use of a compound of formula (I) or its pharmaceutically acceptable salts

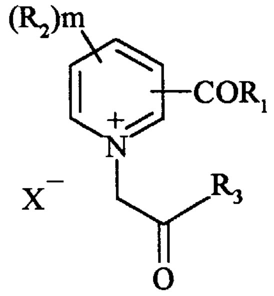

**(I)**

wherein
$R_1$ is $-R_4-R_5$ or $-N(R_7)\ N\ (R_7)\ R_9$;
$R_4$ is selected from the group consisting of $-N(R_7)R_6O-$,
$-N(R_7)R_6N(R_7)$,
$-OR_6O$, and $-OR_6N(R_7)-$,
where $R_6$ is alkyl with $C_2$-$C_8$ Carbon atoms;
$R_5$ is selected from the group consisting of alkyl, aryl including heteroaryl, $-COR_7$, $-SO_2R_7$, $-C(S)\ NHR_7$, $-C(NH)NHR_7$, $-COR_{10}$,
$-C(O)NHR_7$ and

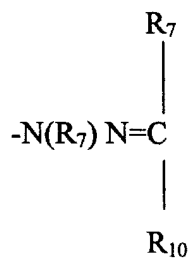

where $R_7$ is selected from the group consisting of H, alkyl and aryl including heteroaryl, provided $R_7$ may be the same or different for $R_1$ and $R_3$ in the same compound;
$R_2$ is selected from the group consisting of F, Cl, Br, I, $OR_7$, $NO_2$, alkyl, aryl including heteroaryl, formyl, acyl, $C(O)NR_7R_{10}$, $C(O)OR_7$, $NR_7R_{10}$, $N=C(R_7)(R_{10})$, $SR_7$, $SO_2NH_2$, $SO_2$ alkyl and $SO_2$aryl;
m is 0, 1 or 2;
$R_3$ is selected from the group consisting of $R_7$, OR7, $N(R_7)(R_{10})$, $N=C(R_7)(R_{10})$, $N(R_7)\ N(R_7)(R_{10})$, $N(R_7)\ N=C(R_7)(R_{10})$ and $CH(R_7)C(O)R_8$
where $R_8$ is selected from the group consisting of $R_7$, OR7 and $NR_7R_{10}$;
$R_9$ is selected from the group consisting of hydrogen, alkyl, aryl including heteroaryl, $C(O)R_{10}$, $-SO_2R_{10}$, $C(S)\ NHR_{10}$, $C(NH)\ NH\ (R_{10})$ and $C(O)\ NHR_{10}$;
$R_{10}$ is selected from the group consisting of H, alkyl and aryl, including heteroaryl and in each case may be the same or different from substituent $R_7$, provided $R_{10}$ may be the same or different for $R_1$ and $R_3$ in the same compound;
X is selected from group consisting of a halide ion, acetate ion, perchlorate ion, sulfonate ion, oxalate ion, citrate ion, tosylate ion, maleate ion, mesylate ion, carbonate ion, sulfite ion, phosphoric hydrogen ion, phosphonate ion, phosphate ion, $BF_4$- and $PF_6$-; with proviso that,

(i) when two alkyl groups are present on the same carbon or nitrogen, they may be linked together to form a cyclic structure;

(ii) the nitrogen of heteroaryl ring of $R_{10}$, when present, may be quaternized;
(iii) when $R_3$ is $OR_7$ and $R_1$ is $-NHNH_2$ then $R_7$ is not alkyl and
(iv) when $R_3$ is $OR_7$, $R_1$ is $N(R_7)(NR_7)R_9$ and $R_9$ is $C(O)R_{10}$ where

$R_{10}$ is alkyl, then $R_7$ is not hydrogen,
for the manufacture of a medicament useful for the treatment of diseases caused by accumulation of free radicals in the body cells.

**47.** The use of a compound as claimed in claim 46, wherein $-C(O)R_1$ group of said compound is at position 3 or 4.

**48.** The use of a compound as claimed in claim 47, wherein $-C(O)R_1$ group of said compound is at position 3.

**49.** The use of a compound as claimed in claim 46, wherein for the said compound m is 0 or 1.

**50.** The use of a compound as claimed in claim 47, wherein for the said compound m is 0 or 1.

**51.** The use of a compound as claimed in claim 48, wherein for the said compound m is 0 or 1.

**52.** The use of a compound as claimed in claim 46, wherein for the said compound m is 0.

**53.** The use of a compound as claimed in claim 47, wherein for the said compound m is 0.

**54.** The use of a compound as claimed in claim 48, wherein for the said compound m is 0.

**55.** The use of a compound as claimed in claim 46, wherein for the said compound X is a halide ion.

**56.** The use of a compound as claimed in claim 46, wherein said compound is selected from the group consisting of:

(a) N,N'-bis[3-carbonyl-1-(2-thien -2'- yl -2-oxoethyl) -3-pyridinium] hydrazine dibromide or other pharmaceutially acceptable salts thereof,
(b) 1-(2-ethoxy -2-oxoethyl) -3-(2-(2-pyridyl)hydrazinocarbonyl) pyridinium bromide or other pharmaceutially acceptable salts thereof,
(c) 1-(2-ethoxy -2-oxoethyl) -3-(2-(benzoyloxy) ethylamino carbonyl) pyridinium bromide or other pharmaceutially acceptable salts thereof,
(d)N,N'-bis[3-carbonyl-1-(2-phenyl-2-oxoethyl)pyridinium]hydrazine dibromide or other pharmaceutially acceptable salts thereof,
(e)1-(2-phenyl-2-oxoethyl)-3-(hydrazinocarbonyl)pyridinium bromide or other pharmaceutially acceptable, salts thereof,
(f) 1-(2-thien -2'-yl -2-oxoethyl) -3-(methanesulfonyl hydrazinocarbonyl) pyridinium bromide or other pharmaceutially acceptable salts thereof,
(g) N,N'-bis[3-carbonyl-1-(2-(2',4'-dichlorophenyl)-2-oxoethyl) pyridinium] hydrazine dibromide or other pharmaceutially acceptable salts thereof,
(h) 1-(2-phenyl -2-oxoethyl) -3-(methanesulfonyl hydrazinocarbonyl) pyridinium bromide or other pharmaceutially acceptable salts thereof,
(i) 1-(2-ethoxy -2-oxoethyl) -3-(methanesulfonyl hydrazinocarbonyl) pyridinium bromide or other pharmaceutially acceptable salts thereof,
(j)1-(2-phenyl-2-oxoethyl)-3-(phenylsulfonylhydrazino carbonyl) pyridinium bromide or other pharmaceutially acceptable salts thereof,
(k)1-(2-phenyl-2-oxoethyl)-2-chloro-3-(phenylsulfonyl hydrazino carbonyl) pyridinium bromide or other pharmaceutially acceptable salts thereof,
(l) 1-(2-thien -2'-yl -2-oxoethyl)-4-(2-(benzoyloxy) ethyl aminocarbonyl) pyridinium bromide or other pharmaceutially acceptable salts thereof,
(m)1- (2- (2,'4'- dichlorophenyl) -2-oxoethyl) -3-(2- (benzoyloxy) ethylaminocarbonyl) pyridinium bromide or other pharmaceutially acceptable salts thereof,
(n) 1-(2-phenyl -2-oxoethyl) -3-(2-(acetoxy) ethyloxy) carbonyl) pyridinium bromide or other pharmaceutially acceptable salts thereof,
(o) 1-(2-ethoxy -2-oxoethyl) -3-(2-(benzoyloxy) ethyloxy carbonyl) pyridinium bromide or other pharmaceutially acceptable salts thereof,

(p) 1-(2-phenylamino-2-oxoethyl)-4-(phenylsulfonyl hydrazino carbonyl)pyridinium chloride or other pharmaceutially acceptable salts thereof,
(q) 1-(2-(2,'4'-dichlorophenyl)-2-oxoethyl)-3- (2(methoxy) ethyloxycarbonyl) pyridinium bromide or other pharmaceutially acceptable salts thereof,
(r) 1-(2-phenylamino-2-oxoethyl)-3-((benzoyloxy) ethylaminocarbonyl) pyridinium chloride or other pharmaceutially acceptable salts thereof,
(s) 1-(2-thien-2'-yl-2-oxoethyl)-3-(phenylaminocarbonyl hydrazinocarbonyl) pyridinium bromide or other pharmaceutially acceptable salts thereof,
(t) 1-(2-phenyl-2-oxoethyl)-3-(2-(acetoxy) ethylaminocarbonyl) pyridinium bromide or other pharmaceutially acceptable salts thereof,
(u) 1-(2-phenylamino-2-oxoethyl)-3-(phenyl sulfonyl hydrazino carbonyl) pyridinium chloride or other pharmaceutially acceptable salts thereof,
(v) 1-(2-phenylamino-2-oxoethyl)-3-((4-methylphenyl)sulfonyl hydrazinocarbonyl) pyridinium chloride or other pharmaceutially acceptable salts thereof,
(w) 1-(2-phenyl-2-oxoethyl)-3-(2-(benzoyloxy)ethyloxy carbonyl) pyridinium bromide or other pharmaceutially acceptable salts thereof,
(x) 1-(2-thien-2'-yl-2-oxoethyl)-3-(phenylcarbonyl hydrazino carbonyl) pyridinium bromide or other pharmaceutially acceptable salts thereof,
(y) 1-(2-ethoxy-2-oxoethyl)-3-((phenylmethyl)sulfonyl hydrazino carbonyl)pyridinium bromide or other pharmaceutially acceptable salts thereof,
(z)1-(2-phenyl-2-oxoethyl)-3-((phenylmethyl)sulfonyl hydrazino carbonyl)pyridinium bromide or other pharmaceutially acceptable salts thereof,
(aa) N, N' - bis [3-carbonyl-1-(2-furan-2'-yl-2-oxoethyl) pyridinium] hydrazine dibromide or other pharmaceutially acceptable salts thereof,
(ab) N,N'-bis [3-carbonyl -1- (2-thien-2'-yl-2-oxoethyl) pyridinium] hydrazine dichloride or other pharmaceutially acceptable salts thereof,
(ac) N,N'-bis-[3-carbonyl-1-(2-cyclopropylamino-2-oxoethyl) pyridinium] hydrazine dichloride or other pharmaceutially acceptable salts thereof,
(ad) 1-(2',4'-dichlorophenyl-2-oxoethyl)-3-(2-methoxyethyl aminocarbonyl)-pyridinium bromide or other pharmaceutially acceptable salts thereof,
(ae) 1-(2-thien-2'-yl-2-oxoethyl)-3-((2-methoxy ethyl) amino carbonyl)-5-bromo pyridinium chloride or other pharmaceutially acceptable salts thereof,
(af) 1-(2-thien-2'-yl-2-oxoethyl)-3-(methanesulfonyl hydrazino carbonyl) pyridinium chloride or other pharmaceutially acceptable salts thereof,
(ag) 1-(2-thien-2'yl-2-oxoethyl)-3-(2-(2-chloro-3-pyridoylhydrazinocarbonyl)-pyridinium chloride or other pharmaceutially acceptable salts thereof,
(ah) 1-(2-cyclopropylamino-2-oxoethyl)-3-(2-methoxyethylaminocarbonyl) -pyridinium chloride or other pharmaceutially acceptable salts thereof,
(ai) 1-(2-isopropylamino-2-oxoethyl)-3-(2-methylsulfonylhydrazinocarbonyl)-pyridinium chloride or other pharmaceutially acceptable salts thereof,
(aj) 1-(2-phenylamino-2-oxo ethyl)-3-({2-(1-oxo-3-cyclohexyl)-propyl} - hydrazinocarbonyl)-pyridinium bromide or other pharmaceutially acceptable salts thereof,
(ak) 1-(2-thien-2'-yl-2-oxoethyl)-3-[2-(benzoyloxy)ethylamino carbonyl]-pyridinium bromide or other pharmaceutially acceptable salts thereof,
(al) 1-(4-ethoxy-2, 4-dioxobutyl)-3-(2-(benzoxyloxy)ethylamino carbonyl)-pyridinium chloride or other pharmaceutially acceptable salts thereof,
(am)1-(2-thien-2'-yl-2-oxoethyl)-3-[1-oxo-1-(2-methoxy carbonyl) pyridyl] hydrazino pyridinium chloride or other pharmaceutially acceptable salts thereof,
(an)1-[1-(2-thien-2'-yl-2-oxoethyl)-5-aminocarbonyl-3-carbonyl pyridinium]-2-[1-(2-thien-2'-yl-2-oxoethyl )-3-carbonyl pyridinium] hydrazine dichloride or other pharmaceutially acceptable salts thereof,
(ao)1-(2-thien-2'-yl-2-oxoethyl)-3-(trifluoromethanesulfonyl hydrazino carbonyl) - pyridinium bromide or other pharmaceutially acceptable salts thereof,
(ap)1-[1-(2-thien-2'-yl-2-oxoethyl)-6-methyl-3-carbonyl pyridinium]-2-[1-(2-thien-2'-yl-2-oxoethyl )-3-carbonyl pyridinium ] hydrazine dichloride or other pharmaceutially acceptable salts thereof,
(aq) N,N'-bis[3-carbonyl-1-(2-(5-methyl-thien-2-yl)-2-oxoethyl) pyridinium] hydrazine dichloride or other pharmaceutially acceptable salts thereof,
(ar) N,N'-bis[3-carbonyl-1-(2-(5-chloro-thien-2-yl)-2-oxoethyl) pyridinium] hydrazine dichloride or other pharmaceutially acceptable salts thereof,

(as) 1-(2-thien-2'-yl-2-oxoethyl)-3-(methanesulfonyl hydrazino carbonyl )-6-methyl pyridinium bromide or other pharmaceutially acceptable salts thereof,
(at) N,N'-bis[3-carbonyl-1-(2-(4-nitro-thien-2-yl)-2-oxoethyl)pyridinium] hydrazine dichloride or other pharmaceutially acceptable salts thereof,
(au) 1-(2-phenylamino-2-oxoethyl)-3-(phenyl hydrazino carbonyl) pyridinium chloride or other pharmaceutially acceptable salts thereof,
(av) 1-(2-(4-nitro-thien-2-yl)-2-oxoethyl)-3-(methanesulfonyl hydrazino carbonyl ) pyridinium bromide or other pharmaceutially acceptable salts thereof,
(aw) 1-(2-(5-nitro-thien-2-yl)-2-oxoethyl)-3-(methanesulfonyl hydrazino carbonyl) pyridinium chloride or other pharmaceutially acceptable salts thereof,
(ax) 1-(2-(5-chloro-thien-2-yl)-2-oxoethyl)-3-(methanesulfonyl hydrazino carbonyl) pyridinium bromide or other pharmaceutially acceptable salts thereof,
(ay) 1-(2-thien-2'-yl-2-oxoethyl)-3-(ethoxycarbonyl hydrazino carbonyl) pyridinium bromide or other pharmaceutially acceptable salts thereof,
(az) 1-(2-thien-2'-yl-2-oxoethyl)-3-(isopropylsulfonyl hydrazino carbonyl) pyridinium bromide or other pharmaceutially acceptable salts thereof,
(ba) 1-(2-thien-2'-yl-2-oxoethyl)-3-(methanesulfonyl hydrazino carbonyl) -5-bromo pyridinium bromide or other pharmaceutially acceptable salts thereof,
(bb) 1-(2-(2-ethoxy carbonyl pyrrolidin-1-yl)-2-oxoethyl)-3-(methanesulfonyl hydrazino carbonyl) pyridinium chloride or other pharmaceutially acceptable salts thereof,
(bc) 1-(2-(5-methyl-thien-2-yl)-2-oxoethyl)-3-(methanesulfonyl hydrazino carbonyl) pyridinium chloride or other pharmaceutially acceptable salts thereof,
(bd) 1-(2-(4- carboethoxy -thiazolidin-3-yl)-2-oxoethyl) -3-(methanesulfonyl hydrazino carbonyl ) pyridinium chloride or other pharmaceutially acceptable salts thereof,
(be) 1-(2-(4-benzyl piperidin-1-yl)-2-oxoethyl)-3-(methanesulfonyl hydrazino carbonyl) pyridinium chloride or other pharmaceutically acceptable salts thereof,
(bf) N,N(-bis[3-carbonyl-1-(2-(2-ethoxycarbonyl pyrrolidin-1-yl)-2-oxoethyl) pyridinium] hydrazine dichloride or other pharmaceutically acceptable salts thereof,
(bg) 1-(2-phenylamino-2-oxoethyl)-4 -[ 2-(benzoyloxy) ethylamino carbonyl ] pyridinium chloride or other pharmaceutically acceptable salts thereof,
(bh) 1-(2-thien-2(-yl-2-oxoethyl)-3-(phenyl hydrazino carbonyl) pyridinium bromide or other pharmaceutically acceptable salts thereof,
(bi) 1-(2-thien-2(-yl-2-oxoethyl)-3-(p-methoxy phenyl sulfonyl hydrazino carbonyl) pyridinium bromide or other pharmaceutically acceptable salts thereof,
(bj) 1-(2-ethoxy-2-oxoethyl)-3-(phenyl aminocarbonyl hydrazino carbonyl ) pyridinium bromide or other pharmaceutically acceptable salts thereof,
(bk) 1-(2-ethoxy-2-oxoethyl)-3-(p-toluene sulfonyl hydrazino carbonyl) pyridinium bromide or other pharmaceutically acceptable salts thereof,
(bl) 1-(2-phenyl-2-oxoethyl)-3-(phenylamino carbonyl hydrazino carbonyl ) pyridinium bromide or other pharmaceutically acceptable salts thereof,
(bm) 1-(2-phenylamino-2-oxoethyl)-3-(benzyl sulfonyl hydrazino carbonyl) pyridiniumchloride or other pharmaceutically acceptable salts thereof,
(bn) 1-(2-phenyl-2-oxoethyl)-4-(methanesulfonyl hydrazino carbonyl ) pyridinium bromide or other pharmaceutically acceptable salts thereof,
(bo) 1-(2-phenyl-2-oxoethyl)-3-(phenyl hydrazino carbonyl ) pyridinium bromide or other pharmaceutically acceptable salts thereof,
(bp) 1 -(2-ethoxy-2-oxoethyl)-4-[2-(benzoyloxy) ethyl amino carbonyl ] pyridinium bromide or other pharmaceutically acceptable salts thereof,
(bq) 1-(2-ethoxy-2-oxoethyl)-3-(phenyl hydrazino carbonyl ) pyridinium bromide or other pharmaceutically acceptable salts thereof,
(br) 1-(2-phenyl-2-oxoethyl)-3-(p-methoxyphenyl sulfonyl hydrazino carbonyl ) pyridinium bromide or other pharmaceutically acceptable salts thereof,
(bs) 1-(2-phenyl-2-oxoethyl)- 4-[2-(benzoyloxy) ethyl amino carbonyl] pyridinium bromide or other pharmaceutically acceptable salts thereof,
(bt) 1-(2-ethoxy-2-oxoethyl)- 4-(p-methanesulfonyl hydrazino carbonyl ) pyridinium bromide or other pharmaceutically acceptable salts thereof,
(bu) 3-carbonylamino-1- (2-(2, 4-dichlorophenyl)-2-oxoethyl)-pyridinium bromide or other pharmaceutically acceptable salts thereof,

(bv) 3-(tetrahydrobenzothiazol-2-yl) aminocarbonyl -1-(2-(2, 4-dichlorophenyl)-2-oxoethyl)-pyridinium bromide or other pharmaceutically acceptable salts thereof,
(bw) 1-(2-phenyl-2-oxoethyl)-3-((2-hydroxyethyl) aminocarbonyl) pyridinium bromide or other pharmaceutically acceptable salts thereof,
(bx) 3-carbonylamino-1- (2-thien-2'-yl-2-oxoethyl)-pyridinium bromide or other pharmaceutically acceptable salts thereof,
(by) 1-(2-phenyl-2-oxoethyl)-3-((p-sulfonamidophenylene) aminocarbonyl) pyridinium bromide or other pharmaceutically acceptable salts thereof,
(bz) 1-(2-ethoxy-2-oxoethyl)-3-((2-hydroxyethyl) aminocarbonyl) pyridinium bromide or other pharmaceutically acceptable salts thereof,
(ca) 1-(2-phenyl-2-oxoethyl)-3-(isopropyloxycarbonyl) pyridinium bromide or other pharmaceutically acceptable salts thereof,
(cb) 1-(2-oxopropyl)-3-((2-hydroxyethyl) aminocarbonyl) pyridinium chloride or other pharmaceutically acceptable salts thereof,
(cc) 1-(2-thien-2'-yl-2-oxoethyl)-3-((2-hydroxyethyl) aminocarbonyl) pyridinium bromide or other pharmaceutically acceptable salts thereof,
(cd) 1-(2-(2,4 - dichlorophenyl-2-oxoethyl) -3- (isopropyloxycarbonyl) pyridinium bromide or other pharmaceutically acceptable salts thereof,
(ce) 1- (2-phenyl - 2- oxoethyl) - 3- ((4-methylthiazol - 2 -yl) aminocarbonyl) pyridinium bromide or other pharmaceutically acceptable salts thereof,
(cf) 1-(2- phenylamino -2- oxoethyl ) -3- (n-butyloxycarbonyl) pyridinium chloride or other pharmaceutically acceptable salts thereof,
(cg) 1- (2- phenylamino - 2- oxoethyl)- 3- ((2-hydroxyethyl) aminocarbonyl) - pyridinium chloride or other pharmaceutically acceptable salts thereof,
(ch) 1- (2- (2,4 - dichlorophenyl ) - 2-oxoethyl) - 3- (n - butoxycarbonyl) pyridinium bromide or other pharmaceutically acceptable salts thereof,
(ci) 1-(2 - (2, 4- dichlorophenyl) - 2-oxoethyl) - 3- (n-butylamino-carbonyl) pyridinium bromide or other pharmaceutically acceptable salts thereof,
(cj) 1-(2-phenyl-2-oxoethyl)-3-(1-phenyl-1-oxomethyl) pyridinium bromide or other pharmaceutically acceptable salts thereof and
(ck) 1- (2 phenyl - 2-oxoethyl) - 3- (methoxycarbonyl) pyridinium bromide or other pharmaceutically acceptable salts thereof.

**57.** The use as claimed in claim 46, wherein the diseases for which the medicament is useful is selected from the group consisting of

a) Neurodegenerative disorders,
b) Diabetes and Diabetic Vascular Complications,
c) Intestinal Diseases,
d) Liver Diseases,
e) Cancers,
f) Cardiac Diseases,
g) Opthalmic Disorders,
h) HIV Disease,
i) Respiratory Disease and
j) Renal Diseases

**58.** A pharmaceutical composition for inhibiting AGE in a mammal, as defined in formula (I),

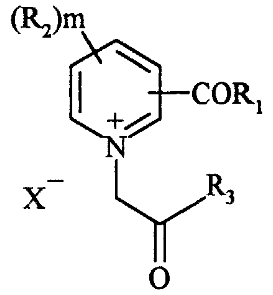

(I)

or its pharmaceutically acceptable salts in association with pharmaceutically acceptable carrier, diluent, excipient or solvate,
wherein
$R_1$ is $-R_4-R_5$ or $-N(R_7)$ N $(R_7)$ $R_9$;
$R_4$ is selected from the group consisting of $-N(R_7)R_6O-$,
$-N(R_7)R_6N(R_7)$,
$-OR_6O$, and $-OR_6N(R_7)-$,
where $R_6$ is alkyl with $C_2$-$C_8$ Carbon atoms;
$R_5$ is selected from the group consisting of alkyl, aryl including heteroaryl, $-COR_7$, $-SO_2R_7$, $-C(S)NHR_7$, $-C(NH)NHR_7$, $-COR_{10}$,

$-C(O)NHR_7$ and 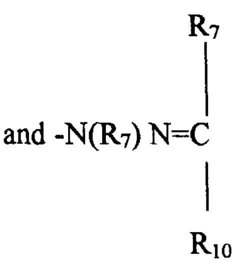

where $R_7$ is selected from the group consisting of H, alkyl and aryl including heteroaryl, provided $R_7$ may be the same or different for $R_1$ and $R_3$ in the same compound;
$R_2$ is selected from the group consisting of F, Cl, Br, I, $OR_7$, $NO_2$, alkyl, aryl including heteroaryl, formyl, acyl, C(O)$NR_7R_{10}$, $C(O)OR_7$, $NR_7R_{10}$, $N=C(R_7)(R_{10})$, $SR_7$, $SO_2NH_2$, $SO_2$ alkyl and $SO_2$aryl;
m is 0, 1 or 2;
$R_3$ is selected from the group consisting of $R_7$, $OR_7$, $N(R_7)$ $(R_{10})$, $N=C(R_7)$ $(R_{10})$, $N(R_7)$ $N(R_7)$ $(R_{10})$, $N(R_7)$ N=C $(R_7)$ $(R_{10})$ and $CH(R_7)C(O)R_8$
where $R_8$ is selected from the group consisting of $R_7$, $OR_7$ and $NR_7R_{10}$;
$R_9$ is selected from the group consisting of hydrogen, alkyl, aryl including heteroaryl, $C(O)R_{10}$, $-SO_2R_{10}$, $C(S)NHR_{10}$, C(NH) NH $(R_{10})$ and C(O) $NHR_{10}$;
$R_{10}$ is selected from the group consisting of H, alkyl and aryl, including heteroaryl and in each case may be the same or different from substituent $R_7$, provided $R_{10}$ may be the same or different for $R_1$ and $R_3$ in the same compound;
X is selected from group consisting of a halide ion, acetate ion, perchlorate ion, sulfonate ion, oxalate ion, citrate ion, tosylate ion, maleate ion, mesylate ion, carbonate ion, sulfite ion, phosphoric hydrogen ion, phosphonate ion, phosphate ion, $BF_4$- and $PF_6$-; with proviso that,

(i) when two alkyl groups are present on the same carbon or nitrogen, they may be linked together to form a cyclic structure;

(iii) the nitrogen of heteroaryl ring of $R_{10}$, when present, may be quaternized;
(iii) when $R_3$ is $OR_7$ and $R_1$ is $-NHNH_2$ then $R_7$ is not alkyl and
(iv) when $R_3$ is $OR_7$ , $R_1$ is $N(R_7)(NR_7)R_9$ and $R_9$ is $C(O)R_{10}$ where

$R_{10}$ is alkyl, then $R_7$ is not hydrogen.

**59.** The composition as claimed in claim 58, wherein the said compound is selected from the group comprising of

(a) N,N'-bis[3-carbonyl-1-(2-thien -2'- yl -2-oxoethyl) -3-pyridinium] hydrazine dibromide or other pharmaceutially acceptable salts thereof,
(b) 1-(2-ethoxy -2-oxoethyl) -3-(2-(2-pyridyl)hydrazinocarbonyl) pyridinium bromide or other pharmaceutially acceptable salts thereof,
(c) 1-(2-ethoxy -2-oxoethyl) -3-(2-(benzoyloxy) ethylamino carbonyl) pyridinium bromide or other pharmaceutially acceptable salts thereof,
(d)N,N'-bis[3-carbonyl-1-(2-phenyl-2-oxoethyl)pyridinium]hydrazine dibromide or other pharmaceutially acceptable salts thereof,
(e)1-(2-phenyl-2-oxoethyl)-3-(hydrazinocarbonyl)pyridinium bromide or other pharmaceutially acceptable salts thereof,
(f) 1-(2-thien -2'-yl -2-oxoethyl) -3-(methanesulfonyl hydrazinocarbonyl) pyridinium bromide or other pharmaceutially acceptable salts thereof,
(g) N,N'-bis[3-carbonyl-1-(2-(2',4'-dichlorophenyl)-2-oxoethyl) pyridinium] hydrazine dibromide or other pharmaceutially acceptable salts thereof,
(h) 1-(2-phenyl -2-oxoethyl) -3-(methanesulfonyl hydrazinocarbonyl) pyridinium bromide or other pharmaceutially acceptable salts thereof,
(i) 1-(2-ethoxy -2-oxoethyl) -3-(methanesulfonyl hydrazinocarbonyl) pyridinium bromide or other pharmaceutially acceptable salts thereof,
(j)1-(2-phenyl-2-oxoethyl)-3-(phenylsulfonylhydrazino carbonyl) pyridinium bromide or other pharmaceutially acceptable salts thereof,
(k)1-(2-phenyl-2-oxoethyl)-2-chloro-3-(phenylsulfonyl hydrazino carbonyl) pyridinium bromide or other pharmaceutically acceptable salts thereof,
(l) 1-(2-thien -2'-yl -2-oxoethyl)-4-(2-(benzoyloxy) ethyl aminocarbonyl) pyridinium bromide or other pharmaceutially acceptable salts thereof,
(m)1- (2- (2,'4'- dichlorophenyl) -2-oxoethyl) -3-(2- (benzoyloxy) ethylaminocarbonyl) pyridinium bromide or other pharmaceutially acceptable salts thereof,
(n) 1-(2-phenyl -2-oxoethyl) -3-(2-(acetoxy) ethyloxy) carbonyl) pyridinium bromide or other pharmaceutially acceptable salts thereof,
(o) 1-(2-ethoxy -2-oxoethyl) -3-(2-(benzoyloxy) ethyloxy carbonyl) pyridinium bromide or other pharmaceutially acceptable salts thereof,
(p) 1-(2-phenylamino-2-oxoethyl)-4-(phenylsulfonyl hydrazino carbonyl)pyridinium chloride or other pharmaceutially acceptable salts thereof,
(q) 1-(2-(2,'4'-dichlorophenyl)-2-oxoethyl)-3- (2(methoxy) ethyloxycarbonyl) pyridinium bromide or other pharmaceutially acceptable salts thereof,
(r) 1-(2-phenylamino-2-oxoethyl)-3-((benzoyloxy) ethylaminocarbonyl) pyridinium chloride or other pharmaceutially acceptable salts thereof,
(s) 1-(2-thien-2'-yl-2-oxoethyl)-3-(phenylaminocarbonyl hydrazinocarbonyl) pyridinium bromide or other pharmaceutially acceptable salts thereof,
(t) 1-(2-phenyl-2-oxoethyl)-3-(2-(acetoxy) ethylaminocarbonyl) pyridinium bromide or other pharmaceutially acceptable salts thereof,
(u) 1-(2-phenylamino-2-oxoethyl)-3-(phenyl sulfonyl hydrazino carbonyl) pyridinium chloride or other pharmaceutially acceptable salts thereof,
(v) 1-(2-phenylamino-2-oxoethyl)-3-((4-methylphenyl)sulfonyl hydrazinocarbonyl) pyridinium chloride or other pharmaceutially acceptable salts thereof,
(w) 1-(2-phenyl-2-oxoethyl)-3-(2-(benzoyloxy)ethyloxy carbonyl) pyridinium bromide or other pharmaceutially acceptable salts thereof,
(x) 1-(2-thien-2'-yl-2-oxoethyl)-3-(phenylcarbonyl hydrazino carbonyl) pyridinium bromide or other pharmaceutially acceptable salts thereof,
(y) 1-(2-ethoxy-2-oxoethyl)-3-((phenylmethyl)sulfonyl hydrazino carbonyl)pyridinium bromide or other pharmaceutially acceptable salts thereof,

(z)1-(2-phenyl-2-oxoethyl)-3-((phenylmethyl)sulfonyl hydrazino carbonyl)pyridinium bromide or other pharmaceutially acceptable salts thereof,

(aa) N, N' -- bis [3-carbonyl-1-(2-furan-2'-yl-2-oxoethyl) pyridinium] hydrazine dibromide or other pharmaceutially acceptable salts thereof,

(ab) N,N'-bis [3-carbonyl -1- (2-thien-2'-yl-2-oxoethyl) pyridinium] hydrazine dichloride or other pharmaceutially acceptable salts thereof,

(ac) N,N'-bis-[3-carbonyl-1-(2-cyclopropylamino-2-oxoethyl) pyridinium] hydrazine dichloride or other pharmaceutially acceptable salts thereof,

(ad) 1-(2',4'-dichlorophenyl-2-oxoethyl)-3-(2-methoxyethyl aminocarbonyl)-pyridinium bromide or other pharmaceutially acceptable salts thereof,

(ae) 1-(2-thien-2'-yl-2-oxoethyl)-3-((2-methoxy ethyl) amino carbonyl)-5-bromo pyridinium chloride or other pharmaceutially acceptable salts thereof,

(af) 1-(2-thien-2'-yl-2-oxoethyl)-3-(methanesulfonyl hydrazino carbonyl) pyridinium chloride or other pharmaceutially acceptable salts thereof,

(ag) 1-(2-thien-2'yl-2-oxoethyl)-3-(2-(2-chloro-3-pyridoylhydrazinocarbonyl)-pyridinium chloride or other pharmaceutially acceptable salts thereof,

(ah) 1-(2-cyclopropylamino-2-oxoethyl)-3-(2-methoxyethylaminocarbonyl) -pyridinium chloride or other pharmaceutially acceptable salts thereof,

(ai) 1-(2-isopropylamino-2-oxoethyl)-3-(2-methylsulfonylhydrazinocarbonyl)-pyridinium chloride or other pharmaceutially acceptable salts thereof,

(aj) 1-(2-phenylamino-2-oxo ethyl)-3-({2-(1-oxo-3-cyclohexyl)-propyl}-hydrazinocarbonyl)-pyridinium bromide or other pharmaceutially acceptable salts thereof,

(ak) 1-(2-thien-2'-yl-2-oxoethyl)-3-[2-(benzoyloxy)ethylamino carbonyl]-pyridinium bromide or other pharmaceutially acceptable salts thereof,

(al) 1-(4-ethoxy-2, 4-dioxobutyl)-3-(2-(benzoxyloxy)ethylamino carbonyl)-pyridinium chloride or other pharmaceutially acceptable salts thereof,

(am)1-(2-thien-2'-yl-2-oxoethyl)-3-[1-oxo-1-(2-methoxy carbonyl) pyridyl] hydrazino pyridinium chloride or other pharmaceutially acceptable salts thereof,

(an)1-[1-(2-thien-2'-yl-2-oxoethyl)-5-aminocarbonyl-3-carbonyl pyridinium]-2-[1-(2-thien-2'-yl-2-oxoethyl )-3-carbonyl pyridinium] hydrazine dichloride or other pharmaceutially acceptable salts thereof,

(ao)1-(2-thien-2'-yl-2-oxoethyl)-3-(trifluoromethanesulfonyl hydrazino carbonyl)-pyridinium bromide or other pharmaceutially acceptable salts thereof,

(ap) 1-[1-(2-thien-2'-yl-2-oxoethyl)-6-methyl-3-carbonyl pyridinium]-2-[1-(2-thien-2'-yl-2-oxoethyl )-3-carbonyl pyridinium ] hydrazine dichloride or other pharmaceutially acceptable salts thereof,

(aq) N,N'-bis[3-carbonyl-1-(2-(5-methyl-thien-2-yl)-2-oxoethyl) pyridinium] hydrazine dichloride or other pharmaceutially acceptable salts thereof,

(ar) N,N'-bis[3-carbonyl-1-(2-(5-chloro-thien-2-yl)-2-oxoethyl) pyridinium] hydrazine dichloride or other pharmaceutially acceptable salts thereof,

(as) 1-(2-thien-2'-yl-2-oxoethyl)-3-(methanesulfonyl hydrazino carbonyl )-6-methyl pyridinium bromide or other pharmaceutially acceptable salts thereof,

(at) N,N'-bis[3-carbonyl-1-(2-(4-nitro-thien-2-yl)-2-oxoethyl)pyridinium] hydrazine dichloride or other pharmaceutially acceptable salts thereof,

(au) 1-(2-phenylamino-2-oxoethyl)-3-(phenyl hydrazino carbonyl) pyridinium chloride or other pharmaceutially acceptable salts thereof,

(av) 1-(2-(4-nitro-thien-2-yl)-2-oxoethyl)-3-(methanesulfonyl hydrazino carbonyl ) pyridinium bromide or other pharmaceutially acceptable salts thereof,

(aw) 1-(2-(5-nitro-thien-2-yl)-2-oxoethyl)-3-(methanesulfonyl hydrazino carbonyl) pyridinium chloride or other pharmaceutially acceptable salts thereof,

(ax) 1-(2-(5-chloro-thien-2-yl)-2-oxoethyl)-3-(methanesulfonyl hydrazino carbonyl) pyridinium bromide or other pharmaceutially acceptable salts thereof,

(ay) 1-(2-thien-2'-yl-2-oxoethyl)-3-(ethoxycarbonyl hydrazino carbonyl) pyridinium bromide or other pharmaceutially acceptable salts thereof,

(az) 1-(2-thien-2'-yl-2-oxoethyl)-3-(isopropylsulfonyl hydrazino carbonyl) pyridinium bromide or other pharmaceutially acceptable salts thereof,

(ba) 1-(2-thien-2'-yl-2-oxoethyl)-3-(methanesulfonyl hydrazino carbonyl) -5-bromo pyridinium bromide or other pharmaceutially acceptable salts thereof,

(bb) 1-(2-(2-ethoxy carbonyl pyrrolidin-1-yl)-2-oxoethyl)-3-(methanesulfonyl hydrazino carbonyl) pyridinium chloride or other pharmaceutially acceptable salts thereof,

(bc) 1-(2-(5-methyl-thien-2-yl)-2-oxoethyl)-3-(methanesulfonyl hydrazino carbonyl) pyridinium chloride or other pharmaceutially acceptable salts thereof,
(bd) 1-(2-(4- carboethoxy -thiazolidin-3-yl)-2-oxoethyl) -3-(methanesulfonyl hydrazino carbonyl ) pyridinium chloride or other pharmaceutially acceptable salts thereof,
(be) 1-(2-(4-benzyl piperidin-1-yl)-2-oxoethyl)-3-(methanesulfonyl hydrazino carbonyl) pyridinium chloride or other pharmaceutically acceptable salts thereof,
(bf) N,N(-bis[3-carbonyl-1-(2-(2-ethoxycarbonyl pyrrolidin-1-yl)-2-oxoethyl) pyridinium] hydrazine dichloride or other pharmaceutically acceptable salts thereof,
(bg) 1-(2-phenylamino-2-oxoethyl)-4 -[ 2-(benzoyloxy) ethylamino carbonyl ] pyridinium chloride or other pharmaceutically acceptable salts thereof,
(bh) 1-(2-thien-2(-yl-2-oxoethyl)-3-(phenyl hydrazino carbonyl) pyridinium bromide or other pharmaceutically acceptable salts thereof,
(bi) 1-(2-thien-2(-yl-2-oxoethyl)-3-(p-methoxy phenyl sulfonyl hydrazino carbonyl) pyridinium bromide or other pharmaceutically acceptable salts thereof,
(bj) 1-(2-ethoxy-2-oxoethyl)-3-(phenyl aminocarbonyl hydrazino carbonyl ) pyridinium bromide or other pharmaceutically acceptable salts thereof,
(bk) 1-(2-ethoxy-2-oxoethyl)-3-(p-toluene sulfonyl hydrazino carbonyl) pyridinium bromide or other pharmaceutically acceptable salts thereof,
(bl) 1-(2-phenyl-2-oxoethyl)-3-(phenylamino carbonyl hydrazino carbonyl ) pyridinium bromide or other pharmaceutically acceptable salts thereof,
(bm) 1-(2-phenylamino-2-oxoethyl)-3-(benzyl sulfonyl hydrazino carbonyl) pyridiniumchloride or other pharmaceutically acceptable salts thereof,
(bn) 1-(2-phenyl-2-oxoethyl)-4-(methanesulfonyl hydrazino carbonyl ) pyridinium bromide or other pharmaceutically acceptable salts thereof,
(bo) 1-(2-phenyl-2-oxoethyl)-3-(phenyl hydrazino carbonyl ) pyridinium bromide or other pharmaceutically acceptable salts thereof,
(bp) 1 -(2-ethoxy-2-oxoethyl)-4-[2-(benzoyloxy) ethyl amino carbonyl ] pyridinium bromide or other pharmaceutically acceptable salts thereof,
(bq) 1-(2-ethoxy-2-oxoethyl)-3-(phenyl hydrazino carbonyl ) pyridinium bromide or other pharmaceutically acceptable salts thereof,
(br) 1-(2-phenyl-2-oxoethyl)-3-(p-methoxyphenyl sulfonyl hydrazino carbonyl ) pyridinium bromide or other pharmaceutically acceptable salts thereof,
(bs) 1-(2-phenyl-2-oxoethyl)- 4-[2-(benzoyloxy) ethyl amino carbonyl] pyridinium bromide or other pharmaceutically acceptable salts thereof,
(bt) 1-(2-ethoxy-2-oxoethyl)- 4-(p-methanesulfonyl hydrazino carbonyl ) pyridinium bromide or other pharmaceutically acceptable salts thereof,
(bu) 3-carbonylamino-1- (2-(2, 4-dichlorophenyl)-2-oxoethyl)-pyridinium bromide or other pharmaceutically acceptable salts thereof,
(bv) 3-(tetrahydrobenzothiazol-2-yl) aminocarbonyl -1-(2-(2, 4-dichlorophenyl)-2-oxoethyl)-pyridinium bromide or other pharmaceutically acceptable salts thereof,
(bw) 1-(2-phenyl-2-oxoethyl)-3-((2-hydroxyethyl) aminocarbonyl) pyridinium bromide or other pharmaceutically acceptable salts thereof,
(bx) 3-carbonylamino-1- (2-thien-2'-yl-2-oxoethyl)-pyridinium bromide or other pharmaceutically acceptable salts thereof,
(by) 1-(2-phenyl -2-oxoethyl)-3-((p-sulfonamidophenylene) aminocarbonyl) pyridinium bromide or other pharmaceutically acceptable salts thereof,
(bz) 1-(2-ethoxy-2-oxoethyl)-3-((2-hydroxyethyl) aminocarbonyl) pyridinium bromide or other pharmaceutically acceptable salts thereof,
(ca) 1-(2-phenyl-2-oxoethyl)-3-(isopropyloxycarbonyl) pyridinium bromide or other pharmaceutically acceptable salts thereof,
(cb) 1-(2-oxopropyl)-3-((2-hydroxyethyl) aminocarbonyl) pyridinium chloride or other pharmaceutically acceptable salts thereof,
(cc) 1-(2-thien-2'-yl-2-oxoethyl)-3-((2-hydroxyethyl) aminocarbonyl) pyridinium bromide or other pharmaceutically acceptable salts thereof,
(cd) 1-(2-(2,4 - dichlorophenyl-2-oxoethyl) -3- (isopropyloxycarbonyl) pyridinium bromide or other pharmaceutically acceptable salts thereof,
(ce) 1- (2-phenyl - 2- oxoethyl) - 3- ((4-methylthiazol - 2 -yl) aminocarbonyl) pyridinium bromide or other pharmaceutically acceptable salts thereof,

(cf) 1-(2- phenylamino -2- oxoethyl ) -3- (n-butyloxycarbonyl) pyridinium chloride or other pharmaceutically acceptable salts thereof,

(cg) 1- (2- phenylamino - 2- oxoethyl)- 3- ((2-hydroxyethyl) aminocarbonyl) - pyridinium chloride or other pharmaceutically acceptable salts thereof,

(ch) 1- (2- (2,4 - dichlorophenyl ) - 2-oxoethyl) - 3- (n - butoxycarbonyl) pyridinium bromide or other pharmaceutically acceptable salts thereof,

(ci) 1-(2 - (2, 4 - dichlorophenyl) - 2-oxoethyl) - 3- (n-butylamino-carbonyl) pyridinium bromide or other pharmaceutically acceptable salts thereof,

(cj) 1-(2-phenyl-2-oxoethyl)-3-(1-phenyl-1-oxomethyl) pyridinium bromide or other pharmaceutically acceptable salts thereof and

(ck) 1- (2 phenyl - 2-oxoethyl) - 3- (methoxycarbonyl) pyridinium bromide or other pharmaceutically acceptable salts thereof.

**60.** Use of a compound of Formula (I)

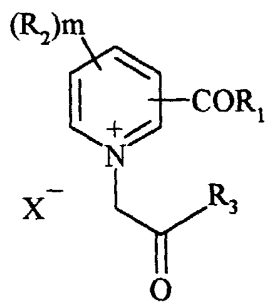

**(I)**

or its pharmaceutically acceptable salts in association with a pharmaceutically acceptable carrier, diluent, excipient or solvate,

wherein

$R_1$ is $-R_4-R_5$ or $-N(R_7)$ N $(R_7)$ $R_9$;

$R_4$ is selected from the group consisting of $-N(R_7)R_6O-$, $-N(R_7)R_6N(R_7)$,

$-OR_6O$, and $-OR_6N(R_7)-$, where $R_6$ is alkyl with $C_2$-$C_8$ Carbon atoms;

$R_5$ is selected from the group consisting of alkyl, aryl including heteroaryl, $-COR_7$, $-SO_2R_7$, -C(S) $NHR_7$, -C(NH) $NHR_7$, $-COR_{10}$,

$-C(O)NHR_7$ and 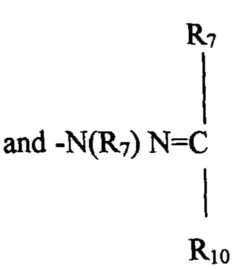

where $R_7$ is selected from the group consisting of H, alkyl and aryl including heteroaryl, provided $R_7$ may be the same or different for $R_1$ and $R_3$ in the same compound;
$R_2$ is selected from the group consisting of F, Cl, Br, I, $OR_7$, $NO_2$, alkyl, aryl including heteroaryl, formyl, acyl, C (O)$NR_7R_{10}$, C(O)$OR_7$, $NR_7R_{10}$, N=C($R_7$)($R_{10}$), $SR_7$, $SO_2NH_2$, $SO_2$ alkyl and $SO_2$aryl;
m is 0, 1 or 2;
$R_3$ is selected from the group consisting of $R_7$, $OR_7$, N($R_7$) ($R_{10}$), N=C($R_7$) ($R_{10}$), N($R_7$) N($R_7$) ($R_{10}$), N($R_7$) N=C($R_7$) ($R_{10}$) and CH($R_7$)C(O)$R_8$
where $R_8$ is selected from the group consisting of $R_7$, $OR_7$ and $NR_7R_{10}$;
$R_9$ is selected from the group consisting of hydrogen, alkyl, aryl including heteroaryl, C(O)$R_{10}$, -$SO_2R_{10}$, C(S) $NHR_{10}$, C(NH) NH ($R_{10}$) and C(O) $NHR_{10}$;
$R_{10}$ is selected from the group consisting of H, alkyl and aryl, including heteroaryl and in each case may be the same or different from substituent $R_7$, provided $R_{10}$ may be the same or different for $R_1$ and $R_3$ in the same compound;
X is selected from group consisting of a halide ion, acetate ion, perchlorate ion, sulfonate ion, oxalate ion, citrate ion, tosylate ion, maleate ion, mesylate ion, carbonate ion, sulfite ion, phosphoric hydrogen ion, phosphonate ion, phosphate ion, $BF_4$- and $PF_6$-;
with proviso that,

(i) when two alkyl groups are present on the same carbon or nitrogen, they may be linked together to form a cyclic structure;
(ii) the nitrogen of heteroaryl ring of $R_{10}$, when present, may be quaternized;
(iii) when $R_3$ is $OR_7$ and $R_1$ is -$NHNH_2$ then $R_7$ is not alkyl and
(iv) when $R_3$ is $OR_7$, $R_1$ is N($R_7$)($NR_7$)$R_9$ and $R_9$ is C(O)$R_{10}$ where $R_{10}$ is alkyl, then $R_7$ is not hydrogen, for the manufacture of a medicament useful for inhibiting onset of AGE (Advanced Glycation End products) in a mammal.

**61.** The use as claimed in claim 60, wherein the said compound is selected from the group consisting of:

(a) N,N'-bis[3-carbonyl-1-(2-thien -2'- yl -2-oxoethyl) -3-pyridinium] hydrazine dibromide or other pharmaceutially acceptable salts thereof,
(b) 1-(2-ethoxy -2-oxoethyl) -3-(2-(2-pyridyl)hydrazinocarbonyl) pyridinium bromide or other pharmaceutially acceptable salts thereof,
(c) 1-(2-ethoxy -2-oxoethyl) -3-(2-(benzoyloxy) ethylamino carbonyl) pyridinium bromide or other pharmaceutially acceptable salts thereof,
(d)N,N'-bis[3-carbonyl-1-(2-phenyl-2-oxoethyl)pyridinium]hydrazine dibromide or other pharmaceutially acceptable salts thereof,
(e)1-(2-phenyl-2-oxoethyl)-3-(hydrazinocarbonyl)pyridinium bromide or other pharmaceutially acceptable salts thereof,
(f) 1-(2-thien -2'-yl -2-oxoethyl) -3-(methanesulfonyl hydrazinocarbonyl) pyridinium bromide or other pharmaceutially acceptable salts thereof,
(g) N,N'-bis[3-carbonyl-1-(2-(2',4'-dichlorophenyl)-2-oxoethyl) pyridinium] hydrazine dibromide or other pharmaceutially acceptable salts thereof,
(h) 1-(2-phenyl -2-oxoethyl) -3-(methanesulfonyl hydrazinocarbonyl) pyridinium bromide or other pharmaceutially acceptable salts thereof,
(i) 1-(2-ethoxy -2-oxoethyl) -3-(methanesulfonyl hydrazinocarbonyl) pyridinium bromide or other pharmaceutially acceptable salts thereof,
(j)1-(2-phenyl-2-oxoethyl)-3-(phenylsulfonylhydrazino carbonyl) pyridinium bromide or other pharmaceutially acceptable salts thereof,
(k)1-(2-phenyl-2-oxoethyl)-2-chloro-3-(phenylsulfonyl hydrazino carbonyl) pyridinium bromide or other pharmaceutially acceptable salts thereof,
(l) 1-(2-thien -2'-yl -2-oxoethyl)-4-(2-(benzoyloxy) ethyl aminocarbonyl) pyridinium bromide or other pharmaceutially acceptable salts thereof,
(m)1- (2- (2,'4'- dichlorophenyl) -2-oxoethyl) -3-(2- (benzoyloxy) ethylaminocarbonyl) pyridinium bromide or other pharmaceutially acceptable salts thereof,
(n) 1-(2-phenyl -2-oxoethyl) -3-(2-(acetoxy) ethyloxy) carbonyl) pyridinium bromide or other pharmaceutially acceptable salts thereof,
(o) 1-(2-ethoxy -2-oxoethyl) -3-(2-(benzoyloxy) ethyloxy carbonyl) pyridinium bromide or other pharmaceutially acceptable salts thereof,

(p) 1-(2-phenylamino-2-oxoethyl)-4-(phenylsulfonyl hydrazino carbonyl)pyridinium chloride or other pharmaceutially acceptable salts thereof,
(q) 1-(2-(2,'4'-dichlorophenyl)-2-oxoethyl)-3- (2(methoxy) ethyloxycarbonyl) pyridinium bromide or other pharmaceutially acceptable salts thereof,
(r) 1-(2-phenylamino-2-oxoethyl)-3-((benzoyloxy) ethylaminocarbonyl) pyridinium chloride or other pharmaceutially acceptable salts thereof,
(s) 1-(2-thien-2'-yl-2-oxoethyl)-3-(phenylaminocarbonyl hydrazinocarbonyl) pyridinium bromide or other pharmaceutially acceptable salts thereof,
(t) 1-(2-phenyl-2-oxoethyl)-3-(2-(acetoxy) ethylaminocarbonyl) pyridinium bromide or other pharmaceutially acceptable salts thereof,
(u) 1-(2-phenylamino-2-oxoethyl)-3-(phenyl sulfonyl hydrazino carbonyl) pyridinium chloride or other pharmaceutially acceptable salts thereof,
(v) 1-(2-phenylamino-2-oxoethyl)-3-((4-methylphenyl)sulfonyl hydrazinocarbonyl) pyridinium chloride or other pharmaceutially acceptable salts thereof,
(w) 1-(2-phenyl-2-oxoethyl)-3-(2-(benzoyloxy)ethyloxy carbonyl) pyridinium bromide or other pharmaceutially acceptable salts thereof,
(x) 1-(2-thien-2'-yl-2-oxoethyl)-3-(phenylcarbonyl hydrazino carbonyl) pyridinium bromide or other pharmaceutially acceptable salts thereof,
(y) 1-(2-ethoxy-2-oxoethyl)-3-((phenylmethyl)sulfonyl hydrazino carbonyl)pyridinium bromide or other pharmaceutially acceptable salts thereof,
(z)1-(2-phenyl-2-oxoethyl)-3-((phenylmethyl)sulfonyl hydrazino carbonyl)pyridinium bromide or other pharmaceutially acceptable salts thereof,
(aa) N, N' - bis [3-carbonyl-1-(2-furan-2'-yl-2-oxoethyl) pyridinium] hydrazine dibromide or other pharmaceutially acceptable salts thereof,
(ab) N,N'-bis [3-carbonyl -1- (2-thien-2'-yl-2-oxoethyl) pyridinium] hydrazine dichloride or other pharmaceutially acceptable salts thereof,
(ac) N,N'-bis-[3-carbonyl-1-(2-cyclopropylamino-2-oxoethyl) pyridinium] hydrazine dichloride or other pharmaceutially acceptable salts thereof,
(ad) 1-(2',4'-dichlorophenyl-2-oxoethyl)-3-(2-methoxyethyl aminocarbonyl)-pyridinium bromide or other pharmaceutially acceptable salts thereof,
(ae) 1-(2-thien-2'-yl-2-oxoethyl)-3-((2-methoxy ethyl) amino carbonyl)-5-bromo pyridinium chloride or other pharmaceutially acceptable salts thereof,
(af) 1-(2-thien-2'-yl-2-oxoethyl)-3-(methanesulfonyl hydrazino carbonyl) pyridinium chloride or other pharmaceutially acceptable salts thereof,
(ag) 1-(2-thien-2'yl-2-oxoethyl)-3-(2-(2-chloro-3-pyridoylhydrazinocarbonyl)-pyridinium chloride or other pharmaceutially acceptable salts thereof,
(ah) 1-(2-cyclopropylamino-2-oxoethyl)-3-(2-methoxyethylaminocarbonyl) -pyridinium chloride or other pharmaceutially acceptable salts thereof,
(ai) 1-(2-isopropylamino-2-oxoethyl)-3-(2-methylsulfonylhydrazinocarbonyl)-pyridinium chloride or other pharmaceutially acceptable salts thereof,
(aj) 1-(2-phenylamino-2-oxo ethyl)-3-({2-(1-oxo-3-cyclohexyl)-propyl}-hydrazinocarbonyl)-pyridinium bromide or other pharmaceutially acceptable salts thereof,
(ak) 1-(2-thien-2'-yl-2-oxoethyl)-3-[2-(benzoyloxy)ethylamino carbonyl]-pyridinium bromide or other pharmaceutially acceptable salts thereof,
(al) 1-(4-ethoxy-2, 4-dioxobutyl)-3-(2-(benzoxyloxy)ethylamino carbonyl)-pyridinium chloride or other pharmaceutially acceptable salts thereof,
(am)1-(2-thien-2'-yl-2-oxoethyl)-3-[1-oxo-1-(2-methoxy carbonyl) pyridyl] hydrazino pyridinium chloride or other pharmaceutially acceptable salts thereof,
(an)1-[1-(2-thien-2'-yl-2-oxoethyl)-5-aminocarbonyl-3-carbonyl pyridinium]-2-[1-(2-thien-2'-yl-2-oxoethyl )-3-carbonyl pyridinium] hydrazine dichloride or other pharmaceutially acceptable salts thereof,
(ao)1-(2-thien-2'-yl-2-oxoethyl)-3-(trifluoromethanesulfonyl hydrazino carbonyl) - pyridinium bromide or other pharmaceutially acceptable salts thereof,
(ap)1-[1-(2-thien-2'-yl-2-oxoethyl)-6-methyl-3-carbonyl pyridinium]-2-[1-(2-thien-2'-yl-2-oxoethyl )-3-carbonyl pyridinium ] hydrazine dichloride or other pharmaceutially acceptable salts thereof,
(aq) N,N'-bis[3-carbonyl-1-(2-(5-methyl-thien-2-yl)-2-oxoethyl) pyridinium] hydrazine dichloride or other pharmaceutially acceptable salts thereof,
(ar) N,N'-bis[3-carbonyl-1-(2-(5-chloro-thien-2-yl)-2-oxoethyl) pyridinium] hydrazine dichloride or other pharmaceutially acceptable salts thereof,

(as) 1-(2-thien-2'-yl-2-oxoethyl)-3-(methanesulfonyl hydrazino carbonyl )-6-methyl pyridinium bromide or other pharmaceutially acceptable salts thereof,
(at) N,N'-bis[3-carbonyl-1-(2-(4-nitro-thien-2-yl)-2-oxoethyl)pyridinium] hydrazine dichloride or other pharmaceutially acceptable salts thereof,
(au) 1-(2-phenylamino-2-oxoethyl)-3-(phenyl hydrazino carbonyl) pyridinium chloride or other pharmaceutially acceptable salts thereof,
(av) 1-(2-(4-nitro-thien-2-yl)-2-oxoethyl)-3-(methanesulfonyl hydrazino carbonyl ) pyridinium bromide or other pharmaceutially acceptable salts thereof,
(aw) 1-(2-(5-nitro-thien-2-yl)-2-oxoethyl)-3-(methanesulfonyl hydrazino carbonyl) pyridinium chloride or other pharmaceutially acceptable salts thereof,
(ax) 1-(2-(5-chloro-thien-2-yl)-2-oxoethyl)-3-(methanesulfonyl hydrazino carbonyl) pyridinium bromide or other pharmaceutially acceptable salts thereof,
(ay) 1-(2-thien-2'-yl-2-oxoethyl)-3-(ethoxycarbonyl hydrazino carbonyl) pyridinium bromide or other pharmaceutially acceptable salts thereof,
(az) 1-(2-thien-2'-yl-2-oxoethyl)-3-(isopropylsulfonyl hydrazino carbonyl) pyridinium bromide or other pharmaceutially acceptable salts thereof,
(ba) 1-(2-thien-2'-yl-2-oxoethyl)-3-(methanesulfonyl hydrazino carbonyl) -5-bromo pyridinium bromide or other pharmaceutially acceptable salts thereof,
(bb) 1-(2-(2-ethoxy carbonyl pyrrolidin-1-yl)-2-oxoethyl)-3-(methanesulfonyl hydrazino carbonyl) pyridinium chloride or other pharmaceutially acceptable salts thereof,
(bc) 1-(2-(5-methyl-thien-2-yl)-2-oxoethyl)-3-(methanesulfonyl hydrazino carbonyl) pyridinium chloride or other pharmaceutially acceptable salts thereof,
(bd) 1-(2-(4- carboethoxy -thiazolidin-3-yl)-2-oxoethyl) -3-(methanesulfonyl hydrazino carbonyl ) pyridinium chloride or other pharmaceutially acceptable salts thereof,
(be) 1-(2-(4-benzyl piperidin-1-yl)-2-oxoethyl)-3-(methanesulfonyl hydrazino carbonyl) pyridinium chloride or other pharmaceutically acceptable salts thereof,
(bf) N,N(-bis[3-carbonyl-1-(2-(2-ethoxycarbonyl pyrrolidin-1-yl)-2-oxoethyl) pyridinium] hydrazine dichloride or other pharmaceutically acceptable salts thereof,
(bg) 1-(2-phenylamino-2-oxoethyl)-4 -[ 2-(benzoyloxy) ethylamino carbonyl ] pyridinium chloride or other pharmaceutically acceptable salts thereof,
(bh) 1-(2-thien-2(-yl-2-oxoethyl)-3-(phenyl hydrazino carbonyl) pyridinium bromide or other pharmaceutically acceptable salts thereof,
(bi) 1-(2-thien-2(-yl-2-oxoethyl)-3-(p-methoxy phenyl sulfonyl hydrazino carbonyl) pyridinium bromide or other pharmaceutically acceptable salts thereof,
(bj) 1-(2-ethoxy-2-oxoethyl)-3-(phenyl aminocarbonyl hydrazino carbonyl ) pyridinium bromide or other pharmaceutically acceptable salts thereof,
(bk) 1-(2-ethoxy-2-oxoethyl)-3-(p-toluene sulfonyl hydrazino carbonyl) pyridinium bromide or other pharmaceutically acceptable salts thereof,
(bl) 1-(2-phenyl-2-oxoethyl)-3-(phenylamino carbonyl hydrazino carbonyl ) pyridinium bromide or other pharmaceutically acceptable salts thereof,
(bm) 1-(2-phenylamino-2-oxoethyl)-3-(benzyl sulfonyl hydrazino carbonyl) pyridiniumchloride or other pharmaceutically acceptable salts thereof,
(bn) 1-(2-phenyl-2-oxoethyl)-4-(methanesulfonyl hydrazino carbonyl ) pyridinium bromide or other pharmaceutically acceptable salts thereof,
(bo) 1-(2-phenyl-2-oxoethyl)-3-(phenyl hydrazino carbonyl ) pyridinium bromide or other pharmaceutically acceptable salts thereof,
(bp) 1 -(2-ethoxy-2-oxoethyl)-4-[2-(benzoyloxy) ethyl amino carbonyl ] pyridinium bromide or other pharmaceutically acceptable salts thereof,
(bq) 1-(2-ethoxy-2-oxoethyl)-3-(phenyl hydrazino carbonyl ) pyridinium bromide or other pharmaceutically acceptable salts thereof,
(br) 1-(2-phenyl-2-oxoethyl)-3-(p-methoxyphenyl sulfonyl hydrazino carbonyl ) pyridinium bromide or other pharmaceutically acceptable salts thereof,
(bs) 1-(2-phenyl-2-oxoethyl)- 4-[2-(benzoyloxy) ethyl amino carbonyl] pyridinium bromide or other pharmaceutically acceptable salts thereof,
(bt) 1-(2-ethoxy-2-oxoethyl)- 4-(p-methanesulfonyl hydrazino carbonyl ) pyridinium bromide or other pharmaceutically acceptable salts thereof,
(bu) 3-carbonylamino-1- (2-(2, 4-dichlorophenyl)-2-oxoethyl)-pyridinium bromide or other pharmaceutically acceptable salts thereof,

(bv) 3-(tetrahydrobenzothiazol-2-yl) aminocarbonyl -1-(2-(2, 4-dichlorophenyl)-2-oxoethyl)-pyridinium bromide or other pharmaceutically acceptable salts thereof,
(bw) 1-(2-phenyl-2-oxoethyl)-3-((2-hydroxyethyl) aminocarbonyl) pyridinium bromide or other pharmaceutically acceptable salts thereof,
(bx) 3-carbonylamino-1- (2-thien-2'-yl-2-oxoethyl)-pyridinium bromide or other pharmaceutically acceptable salts thereof,
(by) 1-(2-phenyl -2-oxoethyl)-3-((p-sulfonamidophenylene) aminocarbonyl) pyridinium bromide or other pharmaceutically acceptable salts thereof,
(bz) 1-(2-ethoxy-2-oxoethyl)-3-((2-hydroxyethyl) aminocarbonyl) pyridinium bromide or other pharmaceutically acceptable salts thereof,
(ca) 1-(2-phenyl-2-oxoethyl)-3-(isopropyloxycarbonyl) pyridinium bromide or other pharmaceutically acceptable salts thereof,
(cb) 1-(2-oxopropyl)-3-((2-hydroxyethyl) aminocarbonyl) pyridinium chloride or other pharmaceutically acceptable salts thereof,
(cc) 1-(2-thien-2'-yl-2-oxoethyl)-3-((2-hydroxyethyl) aminocarbonyl) pyridinium bromide or other pharmaceutically acceptable salts thereof,
(cd) 1-(2-(2,4 - dichlorophenyl-2-oxoethyl) -3- (isopropyloxycarbonyl) pyridinium bromide or other pharmaceutically acceptable salts thereof,
(ce) 1- (2-phenyl - 2- oxoethyl) - 3- ((4-methylthiazol - 2 -yl) aminocarbonyl) pyridinium bromide or other pharmaceutically acceptable salts thereof,
(cf) 1-(2- phenylamino -2- oxoethyl ) -3- (n-butyloxycarbonyl) pyridinium chloride or other pharmaceutically acceptable salts thereof,
(cg) 1- (2- phenylamino - 2- oxoethyl)- 3- ((2-hydroxyethyl) aminocarbonyl) - pyridinium chloride or other pharmaceutically acceptable salts thereof,
(ch) 1- (2- (2,4 - dichlorophenyl ) - 2-oxoethyl) - 3- (n - butoxycarbonyl) pyridinium bromide or other pharmaceutically acceptable salts thereof,
(ci) 1-(2 - (2, 4 - dichlorophenyl) - 2-oxoethyl) - 3- (n-butylamino-carbonyl) pyridinium bromide or other pharmaceutically acceptable salts thereof,
(cj) 1-(2-phenyl-2-oxoethyl)-3-(1-phenyl-1-oxomethyl) pyridinium bromide or other pharmaceutically acceptable salts thereof and
(ck) 1- (2 phenyl - 2-oxoethyl) - 3- (methoxycarbonyl) pyridinium bromide or other pharmaceutically acceptable salts thereof.

**62.** The use as claimed in claim 60 wherein the diseases which are prevented by inhibiting onset of AGE in a mammal are selected from at least one of the following group:

a) vascular and neuro-vascular complications,
b) nephrological disorder,
c) neurological disorder,
d) atherosclerosis,
e) retinal disorder,
f) dermatological disorder,
g) non-enzymatic browning of oral cavity,
h) endothelial or other organ dysfunction,
i) growth impairment,
j) inflammatory disorder,
k) immunological discorder,
l) oxidative stress,
m) aging and diabetic complication,
n) alzheimer disease,
o) restenosis and
p) erectile dysfunction

**63.** Use of a compound of formula (I) or its pharmaceutically acceptable salts,

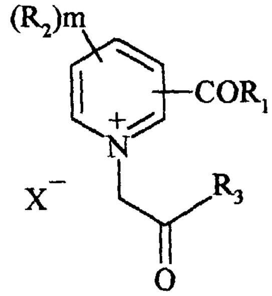

(I)

in association with a pharmaceutically acceptable carrier, diluent, excipient or solvate.

wherein

$R_1$ is $-R_4-R_5$ or $-N(R_7)\ N\ (R_7)\ R_9$;

$R_4$ is selected from the group consisting of $-N(R_7)R_6O-$, $-N(R_7)R_6N(R_7)$, $-OR_6O$, and $-OR_6N(R_7)-$, where $R_6$ is alkyl with $C_2$-$C_8$ Carbon atoms;

$R_5$ is selected from the group consisting of alkyl, aryl including heteroaryl, $-COR_7$, $-SO_2R_7$, $-C(S)\ NHR_7$, $-C(NH)NHR_7$, $-COR_{10}$,

$-C(O)NHR_7$ and 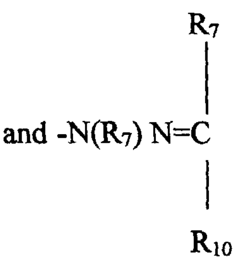

where $R_7$ is selected from the group consisting of H, alkyl and aryl including heteroaryl, provided $R_7$ may be the same or different for $R_1$ and $R_3$ in the same compound;

$R_2$ is selected from the group consisting of F, Cl, Br, I, $OR_7$, $NO_2$, alkyl, aryl including heteroaryl, formyl, acyl, $C(O)NR_7R_{10}$, $C(O)OR_7$, $NR_7R_{10}$, $N{=}C(R_7)(R_{10})$, $SR_7$, $SO_2NH_2$, $SO_2$ alkyl and $SO_2$aryl;

m is 0, 1 or 2;

$R_3$ is selected from the group consisting of $R_7$, $OR_7$, $N(R_7)\ (R_{10})$, $N{=}C(R_7)\ (R_{10})$, $N(R_7)\ N(R_7)\ (R_{10})$, $N(R_7)\ N{=}C(R_7)\ (R_{10})$ and $CH(R_7)C(O)R_8$

where $R_8$ is selected from the group consisting of $R_7$, $OR_7$ and $NR_7R_{10}$; $R_9$ is selected from the group consisting of hydrogen, alkyl, aryl including heteroaryl, $C(O)R_{10}$, $-SO_2R_{10}$, $C(S)NHR_{10}$, $C(NH)\ NH\ (R_{10})$ and $C(O)NHR_{10}$;

$R_{10}$ is selected from the group consisting of H, alkyl and aryl, including heteroaryl and in each case may be the same or different from substituent $R_7$, provided $R_{10}$ may be the same or different for $R_1$ and $R_3$ in the same compound;

X is selected from group consisting of a halide ion, acetate ion, perchlorate ion, sulfonate ion, oxalate ion, citrate ion, tosylate ion, maleate ion, mesylate ion, carbonate ion, sulfite ion, phosphoric hydrogen ion, phosphonate ion, phosphate ion, $BF_4$- and $PF_6$-; with proviso that,

(iii) when two alkyl groups are present on the same carbon or nitrogen, they may be linked together to form a cyclic structure;

(iv) the nitrogen of heteroaryl ring of $R_{10}$, when present, may be quaternized;

(iii) when $R_3$ is $OR_7$ and $R_1$ is $-NHNH_2$ then $R_7$ is not alkyl and

(iv) when $R_3$ is $OR_7$, $R_1$ is $N(R_7)(NR_7)R_9$ and $R_9$ is $C(O)R_{10}$ where $R_{10}$ is alkyl, then $R_7$ is not hydrogen, for

the manufacture of a medicament useful in treatment of conditions requiring simultaneous action of an AGE-breaker, AGE-inhibitor and a free radical scavenger.

**64.** The use of a compound as claimed in claim 63, wherein said compound is selected from the group consisting of

(a) N,N'-bis[3-carbonyl-1-(2-thien -2'- yl -2-oxoethyl) -3-pyridinium] hydrazine dibromide or other pharmaceutially acceptable salts thereof,
(b) 1-(2-ethoxy -2-oxoethyl) -3-(2-(2-pyridyl)hydrazinocarbonyl) pyridinium bromide or other pharmaceutially acceptable salts thereof,
(c) 1-(2-ethoxy -2-oxoethyl) -3-(2-(benzoyloxy) ethylamino carbonyl) pyridinium bromide or other pharmaceutially acceptable salts thereof,
(d)N,N'-bis[3-carbonyl-1-(2-phenyl-2-oxoethyl)pyridinium]hydrazine dibromide or other pharmaceutially acceptable salts thereof,
(e)1-(2-phenyl-2-oxoethyl)-3-(hydrazinocarbonyl)pyridinium bromide or other pharmaceutially acceptable salts thereof,
(f) 1-(2-thien -2'-yl -2-oxoethyl) -3-(methanesulfonyl hydrazinocarbonyl) pyridinium bromide or other pharmaceutially acceptable salts thereof,
(g) N,N'-bis[3-carbonyl-1-(2-(2',4'-dichlorophenyl)-2-oxoethyl) pyridinium] hydrazine dibromide or other pharmaceutially acceptable salts thereof,
(h) 1-(2-phenyl -2-oxoethyl) -3-(methanesulfonyl hydrazinocarbonyl) pyridinium bromide or other pharmaceutially acceptable salts thereof,
(i) 1-(2-ethoxy -2-oxoethyl) -3-(methanesulfonyl hydrazinocarbonyl) pyridinium bromide or other pharmaceutially acceptable salts thereof,
(j)1-(2-phenyl-2-oxoethyl)-3-(phenylsulfonylhydrazino carbonyl) pyridinium bromide or other pharmaceutially acceptable salts thereof,
(k)1-(2-phenyl-2-oxoethyl)-2-chloro-3-(phenylsulfonyl hydrazino carbonyl) pyridinium bromide or other pharmaceutially acceptable salts thereof,
(l) 1-(2-thien -2'-yl -2-oxoethyl)-4-(2-(benzoyloxy) ethyl aminocarbonyl) pyridinium bromide or other pharmaceutially acceptable salts thereof,
(m)1- (2- (2,'4'- dichlorophenyl) -2-oxoethyl) -3-(2- (benzoyloxy) ethylaminocarbonyl) pyridinium bromide or other pharmaceutially acceptable salts thereof,
(n) 1-(2-phenyl -2-oxoethyl) -3-(2-(acetoxy) ethyloxy) carbonyl) pyridinium bromide or other pharmaceutially acceptable salts thereof,
(o) 1-(2-ethoxy -2-oxoethyl) -3-(2-(benzoyloxy) ethyloxy carbonyl) pyridinium bromide or other pharmaceutially acceptable salts thereof,
(p) 1-(2-phenylamino-2-oxoethyl)-4-(phenylsulfonyl hydrazino carbonyl)pyridinium chloride or other pharmaceutially acceptable salts thereof,
(q) 1-(2-(2,'4'-dichlorophenyl)-2-oxoethyl)-3-(2(methoxy) ethyloxycarbonyl) pyridinium bromide or other pharmaceutially acceptable salts thereof,
(r) 1-(2-phenylamino-2-oxoethyl)-3-((benzoyloxy) ethylaminocarbonyl) pyridinium chloride or other pharmaceutially acceptable salts thereof,
(s) 1-(2-thien-2'-yl-2-oxoethyl)-3-(phenylaminocarbonyl hydrazinocarbonyl) pyridinium bromide or other pharmaceutially acceptable salts thereof,
(t) 1-(2-phenyl-2-oxoethyl)-3-(2-(acetoxy) ethylaminocarbonyl) pyridinium bromide or other pharmaceutially acceptable salts thereof,
(u) 1-(2-phenylamino-2-oxoethyl)-3-(phenyl sulfonyl hydrazino carbonyl) pyridinium chloride or other pharmaceutially acceptable salts thereof,
(v) 1-(2-phenylamino-2-oxoethyl)-3-((4-methylphenyl)sulfonyl hydrazinocarbonyl) pyridinium chloride or other pharmaceutially acceptable salts thereof,
(w) 1-(2-phenyl-2-oxoethyl)-3-(2-(benzoyloxy)ethyloxy carbonyl) pyridinium bromide or other pharmaceutially acceptable salts thereof,
(x) 1-(2-thien-2'-yl-2-oxoethyl)-3-(phenylcarbonyl hydrazino carbonyl) pyridinium bromide or other pharmaceutially acceptable salts thereof,
(y) 1-(2-ethoxy-2-oxoethyl)-3-((phenylmethyl)sulfonyl hydrazino carbonyl)pyridinium bromide or other pharmaceutially acceptable salts thereof,
(z)1-(2-phenyl-2-oxoethyl)-3-((phenylmethyl)sulfonyl hydrazino carbonyl)pyridinium bromide or other pharmaceutially acceptable salts thereof,
(aa) N, N' - bis [3-carbonyl-1-(2-furan-2'-yl-2-oxoethyl) pyridinium] hydrazine dibromide or other pharmaceutially acceptable salts thereof,

(ab) N,N'-bis [3-carbonyl -1- (2-thien-2'-yl-2-oxoethyl) pyridinium] hydrazine dichloride or other pharmaceutially acceptable salts thereof,

(ac) N,N'-bis-[3-carbonyl-1-(2-cyclopropylamino-2-oxoethyl) pyridinium] hydrazine dichloride or other pharmaceutially acceptable salts thereof,

(ad) 1-(2',4'-dichlorophenyl-2-oxoethyl)-3-(2-methoxyethyl aminocarbonyl)-pyridinium bromide or other pharmaceutially acceptable salts thereof,

(ae) 1-(2-thien-2'-yl-2-oxoethyl)-3-((2-methoxy ethyl) amino carbonyl)-5-bromo pyridinium chloride or other pharmaceutially acceptable salts thereof,

(af) 1-(2-thien-2'-yl-2-oxoethyl)-3-(methanesulfonyl hydrazino carbonyl) pyridinium chloride or other pharmaceutially acceptable salts thereof,

(ag) 1-(2-thien-2'yl-2-oxoethyl)-3-(2-(2-chloro-3-pyridoylhydrazinocarbonyl)-pyridinium chloride or other pharmaceutially acceptable salts thereof,

(ah) 1-(2-cyclopropylamino-2-oxoethyl)-3-(2-methoxyethylaminocarbonyl) -pyridinium chloride or other pharmaceutially acceptable salts thereof,

(ai) 1-(2-isopropylamino-2-oxoethyl)-3-(2-methylsulfonylhydrazinocarbonyl)-pyridinium chloride or other pharmaceutially acceptable salts thereof,

(aj) 1-(2-phenylamino-2-oxo ethyl)-3-({2-(1-oxo-3-cyclohexyl)-propyl}-hydrazinocarbonyl)-pyridinium bromide or other pharmaceutially acceptable salts thereof,

(ak) 1-(2-thien-2'-yl-2-oxoethyl)-3-[2-(benzoyloxy)ethylamino carbonyl]-pyridinium bromide or other pharmaceutially acceptable salts thereof,

(al) 1-(4-ethoxy-2, 4-dioxobutyl)-3-(2-(benzoxyloxy)ethylamino carbonyl)-pyridinium chloride or other pharmaceutially acceptable salts thereof,

(am)1-(2-thien-2'-yl-2-oxoethyl)-3-[1-oxo-1-(2-methoxy carbonyl) pyridyl] hydrazino pyridinium chloride or other pharmaceutially acceptable salts thereof,

(an)1-[1-(2-thien-2'-yl-2-oxoethyl)-5-aminocarbonyl-3-carbonyl pyridinium]-2-[1-(2-thien-2'-yl-2-oxoethyl )-3-carbonyl pyridinium] hydrazine dichloride or other pharmaceutially acceptable salts thereof,

(ao)1-(2-thien-2'-yl-2-oxoethyl)-3-(trifluoromethanesulfonyl hydrazino carbonyl) - pyridinium bromide or other pharmaceutially acceptable salts thereof,

(ap)1-[1-(2-thien-2'-yl-2-oxoethyl)-6-methyl-3-carbonyl pyridinium]-2-[1-(2-thien-2'-yl-2-oxoethyl )-3-carbonyl pyridinium ] hydrazine dichloride or other pharmaceutially acceptable salts thereof,

(aq) N,N'-bis[3-carbonyl-1-(2-(5-methyl-thien-2-yl)-2-oxoethyl) pyridinium] hydrazine dichloride or other pharmaceutially acceptable salts thereof,

(ar) N,N'-bis[3-carbonyl-1-(2-(5-chloro-thien-2-yl)-2-oxoethyl) pyridinium] hydrazine dichloride or other pharmaceutially acceptable salts thereof,

(as) 1-(2-thien-2'-yl-2-oxoethyl)-3-(methanesulfonyl hydrazino carbonyl )-6-methyl pyridinium bromide or other pharmaceutially acceptable salts thereof,

(at) N,N'-bis[3-carbonyl-1-(2-(4-nitro-thien-2-yl)-2-oxoethyl)pyridinium] hydrazine dichloride or other pharmaceutially acceptable salts thereof,

(au) 1-(2-phenylamino-2-oxoethyl)-3-(phenyl hydrazino carbonyl) pyridinium chloride or other pharmaceutially acceptable salts thereof,

(av) 1-(2-(4-nitro-thien-2-yl)-2-oxoethyl)-3-(methanesulfonyl hydrazino carbonyl ) pyridinium bromide or other pharmaceutially acceptable salts thereof,

(aw) 1-(2-(5-nitro-thien-2-yl)-2-oxoethyl)-3-(methanesulfonyl hydrazino carbonyl) pyridinium chloride or other pharmaceutially acceptable salts thereof,

(ax) 1-(2-(5-chloro-thien-2-yl)-2-oxoethyl)-3-(methanesulfonyl hydrazino carbonyl) pyridinium bromide or other pharmaceutially acceptable salts thereof,

(ay) 1-(2-thien-2'-yl-2-oxoethyl)-3-(ethoxycarbonyl hydrazino carbonyl) pyridinium bromide or other pharmaceutially acceptable salts thereof,

(az) 1-(2-thien-2'-yl-2-oxoethyl)-3-(isopropylsulfonyl hydrazino carbonyl) pyridinium bromide or other pharmaceutially acceptable salts thereof,

(ba) 1-(2-thien-2'-yl-2-oxoethyl)-3-(methanesulfonyl hydrazino carbonyl)
-5-bromo pyridinium bromide or other pharmaceutially acceptable salts thereof,

(bb) 1-(2-(2-ethoxy carbonyl pyrrolidin-1-yl)-2-oxoethyl)-3-(methanesulfonyl hydrazino carbonyl) pyridinium chloride or other pharmaceutially acceptable salts thereof,

(bc) 1-(2-(5-methyl-thien-2-yl)-2-oxoethyl)-3-(methanesulfonyl hydrazino carbonyl) pyridinium chloride or other pharmaceutially acceptable salts thereof,

(bd) 1-(2-(4- carboethoxy -thiazolidin-3-yl)-2-oxoethyl) -3-(methanesulfonyl hydrazino carbonyl ) pyridinium

chloride or other pharmaceutially acceptable salts thereof,
(be) 1-(2-(4-benzyl piperidin-1-yl)-2-oxoethyl)-3-(methanesulfonyl hydrazino carbonyl) pyridinium chloride or other pharmaceutically acceptable salts thereof,
(bf) N,N(-bis[3-carbonyl-1-(2-(2-ethoxycarbonyl pyrrolidin-1-yl)-2-oxoethyl) pyridinium] hydrazine dichloride or other pharmaceutically acceptable salts thereof,
(bg) 1-(2-phenylamino-2-oxoethyl)-4 -[ 2-(benzoyloxy) ethylamino carbonyl ] pyridinium chloride or other pharmaceutically acceptable salts thereof,
(bh) 1-(2-thien-2(-yl-2-oxoethyl)-3-(phenyl hydrazino carbonyl) pyridinium bromide or other pharmaceutically acceptable salts thereof,
(bi) 1-(2-thien-2(-yl-2-oxoethyl)-3-(p-methoxy phenyl sulfonyl hydrazino carbonyl) pyridinium bromide or other pharmaceutically acceptable salts thereof,
(bj) 1-(2-ethoxy-2-oxoethyl)-3-(phenyl aminocarbonyl hydrazino carbonyl ) pyridinium bromide or other pharmaceutically acceptable salts thereof,
(bk) 1-(2-ethoxy-2-oxoethyl)-3-(p-toluene sulfonyl hydrazino carbonyl) pyridinium bromide or other pharmaceutically acceptable salts thereof,
(bl) 1-(2-phenyl-2-oxoethyl)-3-(phenylamino carbonyl hydrazino carbonyl ) pyridinium bromide or other pharmaceutically acceptable salts thereof,
(bm) 1-(2-phenylamino-2-oxoethyl)-3-(benzyl sulfonyl hydrazino carbonyl) pyridiniumchloride or other pharmaceutically acceptable salts thereof,
(bn) 1-(2-phenyl-2-oxoethyl)-4-(methanesulfonyl hydrazino carbonyl ) pyridinium bromide or other pharmaceutically acceptable salts thereof,
(bo) 1-(2-phenyl-2-oxoethyl)-3-(phenyl hydrazino carbonyl ) pyridinium bromide or other pharmaceutically acceptable salts thereof,
(bp) 1 -(2-ethoxy-2-oxoethyl)-4-[2-(benzoyloxy) ethyl amino carbonyl ] pyridinium bromide or other pharmaceutically acceptable salts thereof,
(bq) 1-(2-ethoxy-2-oxoethyl)-3-(phenyl hydrazino carbonyl ) pyridinium bromide or other pharmaceutically acceptable salts thereof,
(br) 1-(2-phenyl-2-oxoethyl)-3-(p-methoxyphenyl sulfonyl hydrazino carbonyl ) pyridinium bromide or other pharmaceutically acceptable salts thereof,
(bs) 1-(2-phenyl-2-oxoethyl)- 4-[2-(benzoyloxy) ethyl amino carbonyl] pyridinium bromide or other pharmaceutically acceptable salts thereof,
(bt) 1-(2-ethoxy-2-oxoethyl)- 4-(p-methanesulfonyl hydrazino carbonyl ) pyridinium bromide or other pharmaceutically acceptable salts thereof,
(bu) 3-carbonylamino-1- (2-(2, 4-dichlorophenyl)-2-oxoethyl)-pyridinium bromide or other pharmaceutically acceptable salts thereof,
(bv) 3-(tetrahydrobenzothiazol-2-yl) aminocarbonyl-1-(2-(2, 4-dichlorophenyl)-2-oxoethyl)-pyridinium bromide or other pharmaceutically acceptable salts thereof,
(bw) 1-(2-phenyl-2-oxoethyl)-3-((2-hydroxyethyl) aminocarbonyl) pyridinium bromide or other pharmaceutically acceptable salts thereof,
(bx) 3-carbonylamino-1- (2-thien-2'-yl-2-oxoethyl)-pyridinium bromide or other pharmaceutically acceptable salts thereof,
(by) 1-(2-phenyl -2-oxoethyl)-3-((p-sulfonamidophenylene) aminocarbonyl) pyridinium bromide or other pharmaceutically acceptable salts thereof,
(bz) 1-(2-ethoxy-2-oxoethyl)-3-((2-hydroxyethyl) aminocarbonyl) pyridinium bromide or other pharmaceutically acceptable salts thereof,
(ca) 1-(2-phenyl-2-oxoethyl)-3-(isopropyloxycarbonyl) pyridinium bromide or other pharmaceutically acceptable salts thereof,
(cb) 1-(2-oxopropyl)-3-((2-hydroxyethyl) aminocarbonyl) pyridinium chloride or other pharmaceutically acceptable salts thereof,
(cc) 1-(2-thien-2'-yl-2-oxoethyl)-3-((2-hydroxyethyl) aminocarbonyl) pyridinium bromide or other pharmaceutically acceptable salts thereof,
(cd) 1-(2-(2,4 - dichlorophenyl-2-oxoethyl) -3- (isopropyloxycarbonyl) pyridinium bromide or other pharmaceutically acceptable salts thereof,
(ce) 1- (2-phenyl - 2- oxoethyl) - 3- ((4-methylthiazol - 2 -yl) aminocarbonyl) pyridinium bromide or other pharmaceutically acceptable salts thereof,
(cf) 1-(2- phenylamino -2- oxoethyl ) -3- (n-butyloxycarbonyl) pyridinium chloride or other pharmaceutically acceptable salts thereof,
(cg) 1- (2- phenylamino - 2- oxoethyl)- 3- ((2-hydroxyethyl) aminocarbonyl) - pyridinium chloride or other pharmaceutically acceptable salts thereof,

(ch) 1- (2- (2,4 - dichlorophenyl ) - 2-oxoethyl) - 3- (n - butoxycarbonyl) pyridinium bromide or other pharmaceutically acceptable salts thereof,

(ci) 1-(2 - (2, 4 - dichlorophenyl) - 2-oxoethyl) - 3- (n-butylamino-carbonyl) pyridinium bromide or other pharmaceutically acceptable salts thereof,

(cj) 1-(2-phenyl-2-oxoethyl)-3-(1-phenyl-1-oxomethyl) pyridinium bromide or other pharmaceutically acceptable salts thereof and

(ck) 1- (2 phenyl - 2-oxoethyl) - 3- (methoxycarbonyl) pyridinium bromide or other pharmaceutically acceptable salts thereof.

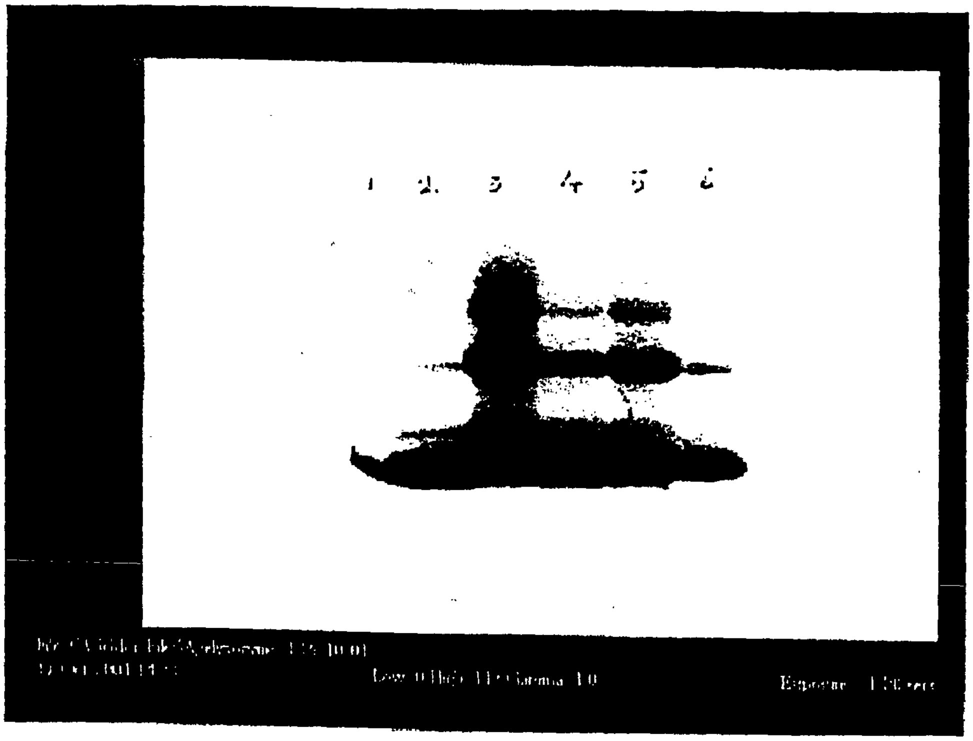

Fig. 1

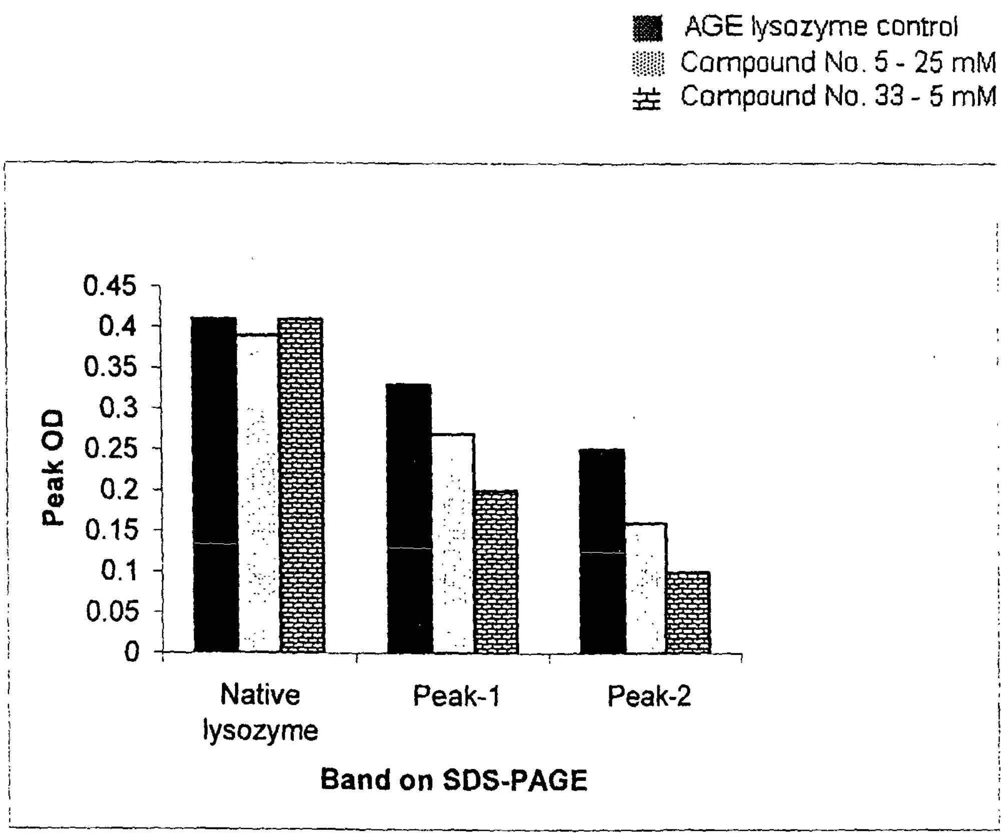
AGE lysozyme control
Compound No. 5 - 25 mM
Compound No. 33 - 5 mM
0.45
0.4
0.35
0.3
0.25
0.2
0.15
0.1
0.05
0
Peak OD
Native lysozyme
Peak-1
Peak-2
Band on SDS-PAGE

Fig. 2

European Patent Office

# EUROPEAN SEARCH REPORT

Application Number
EP 01 20 4295

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X,D<br><br>A | WO 01 25209 A (SANKARANARAYANAN ALANGUDI)<br>12 April 2001 (2001-04-12)<br>* page 26, line 18-22; claims 1-19 * | 1-14,<br>26-64<br>15-25 | A61K7/48<br>A61K31/44<br>A61P17/00 |
| X,D<br><br>A | WO 01 25208 A (SANKARANARAYANAN ALANGUDI)<br>12 April 2001 (2001-04-12)<br>* page 27, line 5 - page 29, line 24;<br>claims 1-19 * | 1-14,<br>26-64<br>15-25 | |
| A | TIWARI S S ET AL: "POSSIBLE ANTI-PARKINSONIAN COMPOUNDS-PART VII SYNTHESIS OF AMIDOMETHYLATION PRODUCTS WITH N-HYDROXYMETHYL NICOTINAMIDE AND THEIR CONVERSION INTO QUATERNARY AMMONIUM IODIDES"<br>JOURNAL OF THE INDIAN CHEMICAL SOCIETY, THE INDIAN CHEMICAL SOCIETY, CALCUTTA, IN,<br>vol. 52, no. 2, 1975, pages 166-167, XP000909760<br>ISSN: 0019-4522<br>* the whole document * | 1-64 | TECHNICAL FIELDS SEARCHED (Int.Cl.7) |
| A | JINDAL S ET AL: "SYNTHESIS OF POSSIBLE HYPOGLYCEMIC COMPOUNDS PART-V: SYNTHESIS OF N-(ACETOPHENONE)-3-(N-HYDROXY ALKYL/ARYL-METHYL-AMIDO)-PYRIDINIUM IODIDES"<br>INDIAN DRUGS, XX, XX,<br>vol. 32, no. 7, 1995, pages 317-319, XP000909803<br>* the whole document * | 1-64 | A61K |

The present search report has been drawn up for all claims

| Place of search | Date of completion of the search | Examiner |
|---|---|---|
| MUNICH | 12 July 2002 | Lindner, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 01 20 4295

This annex lists the patent family members relating to the patent documents cited in the above-mentioned European search report.
The members are as contained in the European Patent Office EDP file on
The European Patent Office is in no way liable for these particulars which are merely given for the purpose of information.

12-07-2002

| Patent document cited in search report | | Publication date | Patent family member(s) | | | Publication date |
|---|---|---|---|---|---|---|
| WO 0125209 | A | 12-04-2001 | AU | 5994499 | A | 10-05-2001 |
| | | | CN | 1329597 | T | 02-01-2002 |
| | | | CZ | 20011808 | A3 | 15-08-2001 |
| | | | EP | 1220843 | A1 | 10-07-2002 |
| | | | WO | 0125209 | A1 | 12-04-2001 |
| | | | PL | 348049 | A1 | 06-05-2002 |
| WO 0125208 | A | 12-04-2001 | AU | 5994299 | A | 10-05-2001 |
| | | | BR | 9913746 | A | 23-04-2002 |
| | | | CN | 1318056 | T | 17-10-2001 |
| | | | CZ | 20011033 | A3 | 15-08-2001 |
| | | | WO | 0125208 | A1 | 12-04-2001 |
| | | | PL | 348327 | A1 | 20-05-2002 |
| | | | US | 2001018524 | A1 | 30-08-2001 |

EPO FORM P0459

For more details about this annex : see Official Journal of the European Patent Office, No. 12/82